(19) Europäisches Patentamt
European Patent Office
Office européen des brevets

(11) Publication number: **0 611 759 A1**

# (12) EUROPEAN PATENT APPLICATION

(21) Application number: **94101542.2**

(22) Date of filing: **02.02.94**

(51) Int. Cl.5: **C07D 239/60**, C07D 401/12, A01N 43/54

(30) Priority: **15.02.93 JP 47110/93**
**30.08.93 JP 235908/93**

(43) Date of publication of application:
**24.08.94 Bulletin 94/34**

(84) Designated Contracting States:
**BE CH DE FR GB IT LI NL**

(71) Applicant: **NIHON BAYER AGROCHEM K.K.**
**10-8, Takanawa 4-chome**
**Minato-ku**
**Tokyo 108 (JP)**

(72) Inventor: **Goto, Toshio**
**214-18, Koganei,**
**Kokobunji-machi**
**Shimotsuga-gun, Tochigi (JP)**
Inventor: **Kitagawa, Yoshinori**
**1085, Ara-machi**
**Mohka-shi, Tochigi (JP)**
Inventor: **Ito, Seishi**
**1-39, Ekihigashidori,**
**Oyama-shi**
**Tochigi (JP)**
Inventor: **Shibuya, Katsuhiko**
**1425-2, Hitotonoya**
**Oh-aza, Oyama-shi, Tochigi (JP)**
Inventor: **Minegishi, Natsuko**
**1-9-31, Wakagi-cho**
**Oyama-shi, Tochigi (JP)**
Inventor: **Ukawa, Kazuhiro**
**1-23-13, Ekihigashidori**
**Oyama-shi, Tochigi (JP)**
Inventor: **Ueno, Chieko**
**934-7, Hitotonoya,**
**Oh-aza**
**Oyama-shi, Tochigi (JP)**
Inventor: **Kyo, Yoshiko**
**934-7, Hitotonoya,**
**Oh-aza**
**Oyama-shi, Tochigi (JP)**

(74) Representative: **Schumacher, Günter, Dr. et al**
**Bayer AG**
**Konzernverwaltung RP**
**Patentabteilung**
**D-51368 Leverkusen Bayerwerk (DE)**

## (54) Pyrimidinyloxy/triazinyloxy alkanol derivatives and their use as herbicides.

(57) The invention relates to novel pyrimidinyloxy/triazinyloxy alkanol derivatives of the formula (I)

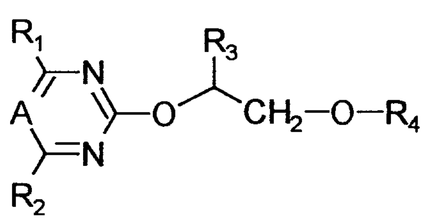

(I)

wherein $R_1$, $R_2$, $R_3$, $R_4$ and A have the meanings as described in the specification, and processes for their preparation and to their use as herbicides, as well as to novel intermediates for their preparation and to processes for their preparation.

EP 0 611 759 A1

The present invention relates to novel pyrimidinyloxy/triazinyloxy alkanol derivatives, to processes for their preparation, and to their use as herbicides, as well as to novel intermediates for their preparation and to processes for their preparation.

It has already been disclosed that a certain group of α-pyrimidinyl thiocarboxylic acid derivatives is useful as herbicides (see Japanese Laid-open Patent Application Nos. 301668/1989, 85262/1990, 20262/1990, 135963/1991 and 240777/1991). Further 2-(4,6-dimethoxypyrimidine-2-ylthio)-2-(methylthio)-ethanol is disclosed in WO92/10484, while WO92/16511 describes the haloacetic acid derivatives thereof.

There have now been found novel pyrimidinyloxy/triazinyloxy alkanol derivatives of the formula (II):

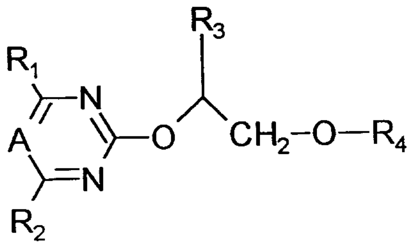

(I)

wherein

| | |
|---|---|
| $R_1$ | represents $C_{1-4}$ alkyl, $C_{1-4}$ alkoxy, halogen, halogeno-$C_{1-4}$ alkyl or halogeno-$C_{1-4}$ alkoxy, |
| $R_2$ | represents $C_{1-4}$ alkoxy, halogen, halogeno-$C_{1-4}$ alkyl or halogeno-$C_{1-4}$ alkoxy, |
| $R_3$ | represents $C_{2-5}$ alkyl, |
| A | represents -CH= or -N=, |
| $R_4$ | represents hydrogen or -CO-$R_5$ and |
| $R_5$ | represents hydrogen, carboxyl, $C_{1-4}$ alkoxycarbonyl or in each case optionally unsubstituted or substituted $C_{1-20}$ saturated hydrocarbon, $C_{2-20}$ unsaturated hydrocarbon, phenyl or $C_{3-8}$ cycloalkyl, |
| $R_5$ | further represents in each case an optionally five- or six-membered heterocyclic group, a condensed heterocyclic group or one of the group represented by the following formulae: |

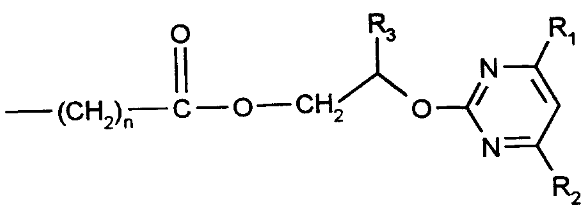

or

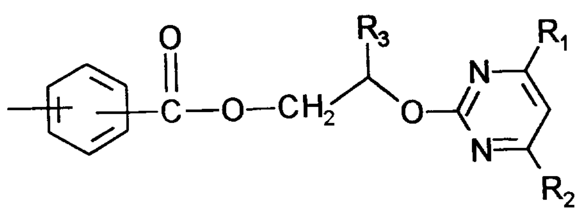

wherein

| | |
|---|---|
| $R_1$, $R_2$ and $R_3$ | have the meanings as mentioned above and |
| n | represents an integer from 1 to 6. |

Pyrimidinyloxy/triazinyloxy alkanol derivatives of the formula (I) wherein $R_1$, $R_2$, $R_3$, $R_4$ and A have the above-mentioned meanings are obtained, when

(a) in the case where $R_4$ represents a hydrogen atom: compounds of the formula (II)

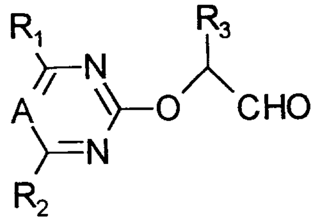 (II)

wherein

$R_1$, $R_2$, $R_3$ and A have the same meanings as mentioned above,

are reduced in the presence of inert solvents,

or

(b) in the case where the $R_4$ represents $-CO-R_5$:

compounds of the formula (IV)

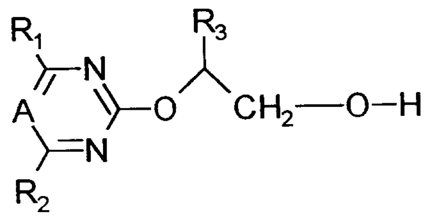 (IV)

wherein

$R_1$, $R_2$, $R_3$ and A have the same meanings as mentioned above,

are reacted with an acid halide of the formula (V),

$R_5-CO-R_7$ (V)

wherein

$R_5$ has the same meanings as mentioned above, and $R_7$ represents halogen,

in the presence of inert solvents and if appropriate, in the presence of an acid binder,

or

(c) in the case where $R_4$ represents $-CO-R_5$:

above-mentioned compounds of the formula (III) are reacted with acid anhydrides of the following formula (VI):

$R_5-CO-O-CO-R_5$ (VI)

wherein

$R_5$ has the same meanings as mentioned above, and the two $R_5$ may be the same or different from each other,

in the presence of inert solvents and if appropriate, in the presence of an acid binder.

The novel pyrimidinyloxy/triazinyloxy alkanol derivatives according to the invention exhibit powerful herbicidal properties.

Surprisingly, the pyrimidinyloxy/triazinyloxy alkanol derivatives according to the invention exhibit a substantially higher selective herbicidal action than those known from the prior art, for instance, the aforementioned Japanese Laid-open Patent Application Nos. 301668/1989, 85862/1990, 20262/1991, 135963/1991, 240777/1991, WO92/10484 and WO92/16511.

In the compounds of the formula (I) according to the invention and their intermediates, the halogen and the halogen atoms of the halogenoalkyl and halogenoalkoxy, represents fluoro, chloro, bromo and iodo, preferably chloro or fluoro.

The $C_{1-4}$ alkyl and the alkyl moiety of the alkoxy and halogeno alkyl represents a straight chain or branched chain alkyl containing 1 to 4 carbon atoms such as methyl, ethyl, propyl, isopropyl, n-(sec-, iso-, tert-)butyl.

The $C_{2-5}$ alkyl and the alkyl moiety of the alkoxy and halogeno alkyl represents a straight-chain or a branched alkyl containing 2 to 5 carbon atoms such as ethyl, propyl, isopropyl, n-(sec-, iso-, tert-)butyl, n-(sec, iso, neo-)pentyl, 1,1-dimethylpropyl, etc.

The $C_{1-20}$ saturated hydrocarbon group represents a straight chain or a branched alkyl containing 1 to 20 carbon atoms such as those exemplified by the above-mentioned $C_{1-4}$ alkyl and $C_{2-5}$ alkyl and, in addition hexyl, heptyl, nonyl, decyl, undecyl, dodecyl, tridecyl, tetradecyl, pentadecyl, 1-ethyl-1-methylpropyl, and so on.

The $C_{2-20}$ unsaturated hydrocarbon group represents a straight chain or a branched $C_{2-20}$ alkene, alkyne, or a hydrocarbon group comprising two or more double bonds and/or triple bonds such as vinyl, allyl, 1-propenyl, isopropenyl, 1-(2-, or 3-)butenyl, 1-(2-, 3-, 4-)pentenyl, 1-methyl-1-propenyl, 2-methyl-1-propenyl, 1-methyl-1-butenyl, 1,1-dimethyl-3-butenyl, 1,3-butanedienyl, ethynyl, propargyl and so on.

The five- or six-membered heterocyclic rings have one to four hetero atoms being selected from the group consisting of nitrogen, sulfur and oxygen, and represents heterocycles such as thiadiazolyl, furyl, thiazolyl, imidazolyl, pyridyl, pyrimidinyl, thienyl, pyrazinyl, pyridazinyl, isoxazolyl and pyrazolyl and the like.

The condensed heterocyclic ring represents a nine- or ten-membered bicyclic group that has been formed by the above-mentioned five- or six-membered ring being condensed with phenyl, such as quinolyl, benzoxazolyl, benzothiazolyl, benzisoxazolyl, benzimidazolyl, phthalozinyl and the like.

Among the pyrimidinyloxy/triazinyloxy alkanol derivatives according to the invention of the formula (I), the preferred compounds are those, wherein

$R_1$ represents methoxy, difluoromethoxy, or trifluoromethoxy,

$R_2$ represents methoxy, difluoromethoxy, or trifluoromethoxy,

$R_3$ represents $C_{2-5}$ alkyl,

A represents -CH=,

$R_4$ represents hydrogen or -CO-$R_5$,

$R_5$ represents hydrogen,

$R_5$ further represents an optionally unsubstituted or substituted **$C_{1-12}$ alkyl** (suitable substituent(s) are cyano, halogen, carboxyl or its salt, $C_{1-4}$ alkoxy, $C_{3-7}$ cycloalkyl [which may be itself substituted by halogen or $C_{1-4}$ alkyl], $C_{1-4}$ alkylthio [which may be itself substituted by halogen or $C_{1-4}$ alkyl], $C_{1-4}$ alkoxy-carbonyl, $C_{1-4}$ alkyl-carbonyl, an optionally substituted phenyl-sulfonyloxy, $C_{1-4}$ alkyl-sulfonyloxy, amino, $C_{1-4}$ alkyl-amino, di-$C_{1-4}$ alkyl amino, an optionally substituted phenyl [which may be itself substituted by nitro, cyano, halogen, $C_{1-4}$ alkyl $C_{1-4}$ alkoxy, halogeno-$C_{1-4}$ alkyl or halogeno-$C_{1-4}$ alkoxy], an optionally substituted phenoxy (which may be itself substituted by nitro, cyano, halogen, $C_{1-4}$ alkyl, $C_{1-4}$ alkoxy, halogeno-$C_{1-4}$ alkyl, halogeno-$C_{1-4}$ alkoxy, a substituted phenoxy, a substituted pyrimidinyloxy, and the below-mentioned group Q], an optionally substituted phenylthio [the substituent(s) may be selected from the group consisting of nitro, cyano, halogen, $C_{1-4}$ alkyl, $C_{1-4}$ alkoxy, halogeno-$C_{1-4}$ alkyl, halogeno-$C_{1-4}$ alkoxy, a substitutable phenoxy, a substitutable pyrimidinyloxy, and the below-mentioned group Q], an optionally substituted naphthyl [the substituent(s) may be selected from the group consisting of nitro, cyano, halogen, $C_{1-4}$ alkyl, $C_{1-4}$ alkoxy, halogeno-$C_{1-4}$ alkyl, halogeno-$C_{1-4}$ alkoxy, and a phenoxy substitutable with halogen or halogenoalkyl], an optionally substituted naphtoxy [the substituent(s) may be selected from the group consisting of nitro, cyano, halogen, $C_{1-4}$ alkyl, $C_{1-4}$ alkoxy, halogeno-$C_{1-4}$ alkyl, halogeno-$C_{1-4}$ alkoxy, and a phenoxy which may be itself substituted by halogen or halogenoalkyl),

$R_5$ further represents an optionally substituted **$C_{2-12}$ alkenyl** (the substituent(s) may be selected from halogen and phenyl which may be itself substituted by halogen or $C_{1-4}$ alkyl),

$R_5$ further represents **$C_{2-12}$ alkynyl**,

$R_5$ further represents an optionally substituted **phenyl** (the substituents may be selected from the group consisting of cyano, nitro, halogen, $C_{1-4}$ alkyl, $C_{1-4}$ alkoxy, halogeno-$C_{1-4}$ alkyl or halogeno-$C_{1-4}$ alkoxy),

$R_5$ further represents $C_{3-8}$ cycloalkyl being optionally substituted by $C_{1-4}$ alkyl,

$R_5$ further represents an optionally substituted **five- or six-membered heterocyclic group** (the hetero atom(s) of said heterocyclic group may be selected from the group consisting of nitrogen, oxygen or sulfur, and the substituents may be selected from the group consisting of cyano, nitro, halogen, $C_{1-4}$ alkyl, $C_{1-4}$ alkoxy, halogeno-$C_{1-4}$ alkyl, halogeno-$C_{1-4}$ alkoxy and an optionally substituted phenyl [which may be itself substituted by one of the group consisting of nitro, cyano, halogen, $C_{1-4}$ alkyl, $C_{1-4}$ alkoxy, halogeno-$C_{1-4}$ alkyl or halogeno-$C_{1-4}$ alkoxy]),

$R_5$ further represents an optionally substituted **condensed heterocyclic group** (the hetero atom(s) of said heterocyclic group may be selected from the group consisting of nitrogen, oxygen or

sulfur, and the substituents may be selected from the group consisting of cyano, nitro, halogen, $C_{1-4}$ alkyl, $C_{1-4}$ alkoxy, halogeno-$C_{1-4}$ alkyl or halogeno-$C_{1-4}$ alkoxy),

$R_5$ further represents **$C_{1-4}$ alkoxy-carbonyl, carboxyl or its salts**, or one of the following group:

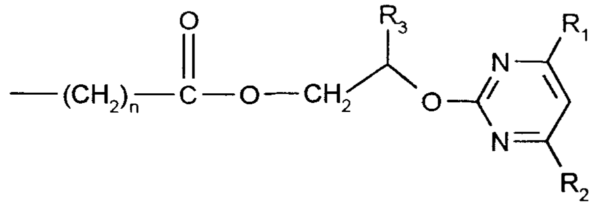

or

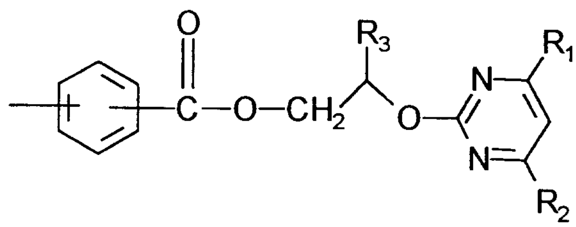

wherein

$R_1$, $R_2$ and $R_3$ have the same meanings as mentioned above, and n represents 0, 1, 2, 4, 5 or 6, and

Q represents an optionally halogen-substituted group selected from:

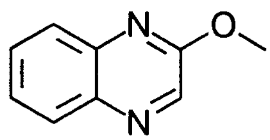

,

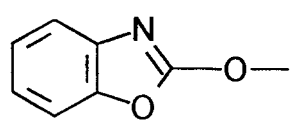

or

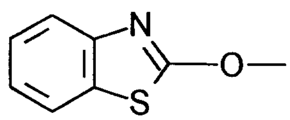

Very particularly preferred pyrimidinyloxy/triazinyloxy alkanol derivatives according to the invention of the formula (I) are those, wherein

$R_1$ represents methoxy,

$R_2$ represents methoxy,

$R_3$ represents $C_{2-4}$ alkyl,

A represents -CH=,

$R_4$ represents hydrogen or -CO-$R_5$,

$R_5$ represents hydrogen,

$R_5$ further represents an optionally substituted **$C_{1-12}$ alkyl** (suitable substituents are cyano, fluoro, chloro, carboxyl or its sodium salt, methoxy, ethoxy, butoxy, a methyl-substituted $C_{3-7}$ cycloalkyl, methylthio, ethylthio, butylthio, methoxy-carbonyl, methyl-carbonyl, a methyl-substituted phenyl-sulfonyloxy, methyl-sulfonyloxy, ethyl-sulfonyloxy, amino, methylamino, ethylamino, dimethylamino, an optionally substituted phenyl [the substituent(s) may be selected from the group consisting of nitro, cyano, fluoro, chloro, bromo, methyl or trifluoromethyl], an optionally substituted phenoxy [the substituent(s) may be selected from the group consisting of nitro, cyano, fluoro, chloro, bromo, methyl, trifluoromethyl, a substituted phenoxy (which may be itself substituted by fluro, chloro or trifluoromethyl), a substituted pyrimidinyloxy (which may be itself substituted by fluoro, chloro ortrifluoromethyl), and the below-mentioned group Q], an optionally substituted phenylthio [the substituent(s) may be selected from the group consisting of nitro, cyano, fluoro, chloro bromo, methyl, trifluoromethyl, halogeno-$C_{1-4}$ alkyl, halogeno-$C_{1-4}$ alkoxy, a

substituted phenyl (which may be itself substituted by fluoro, chloro or trifluoromethyl), a substituted pyrimidinyloxy (which may be itself substituted by fluoro, chloro or trifluoromethyl), and the below-mentioned groupQ], an optionally substituted naphtyl [the substituent(s) may be selected from the group consisting of nitro, cyano, fluoro, chloro, bromo, methyl, trifluoromethyl, halogeno-$C_{1-4}$ alkyl, halogeno-$C_{1-4}$ alkoxy, substituted phenyl (which may be itself substituted by fluoro, chloro, or trifluoromethyl), optionally substituted pyrimdinyloxy (the substituent(s) may be selected from the group consisting of fluoro, chloro or trifluoromethyl), and a phenoxy that may be optionally substituted with chloro or trifluoromethoxy], an optionally substituted naphtoxy [the substituent(s) may be selected from the group consisting of nitro, cyano, fluoro, chloro, bromo, methyl, trifluoromethyl, halogeno-$C_{1-4}$ alkyl,halogeno-$C_{1-4}$ alkoxy, optionally substituted phenyl (the substituent(s) may be selected from the group consisting of fluoro, chloro, or trifluoromethyl), optionally substituted pyrimidinyloxy (the substituent(s) may be selected from the group consisting of fluoro, chloro or trifluoromethyl), and a phenoxy that may be optionally substituted by chloro or trifluoromethoxy]),

$R_5$ further represents an optionally substituted **$C_{3-12}$ alkenyl** (the substituent(s) may be selected from chloro, bromo and phenyl which may be optionally substituted by fluoro, chloro, bromo, methyl or ethyl),

$R_5$ further represents **$C_{3-12}$ alkynyl**,

$R_5$ further represents an optionally substituted **phenyl** (the substituent(s) may be selected from the group consisting of cyano, nitro, fluoro, chloro, bromo, methyl, ethyl, methoxy, ethoxy, trifluoromethyl or trifluoromethoxy),

$R_5$ further represents in each case optionally methyl-substituted **cyclopropyl, cyclopentyl or cyclhexyl**,

$R_5$ further represents in each case an optionally substituted **thiadiazolyl, thiazolyl, imidazolyl, furyl, pyridyl, pyrimidinyl, pyrazinyl, pyridazinyl, isoxazolyl, pyrazolyl and thieyl** (the substituent(s) may be selected from the group consisting of cyano, nitro, fluoro, chloro, methyl, ethyl, methoxy, ethoxy, trifluoromethyl, trifluoromethoxy and an optionally substituted phenyl [the substituent(s) may be selected from the group consisting of nitro, fluoro, chloro, bromo, methyl, ethyl, methoxy or trifluoromethyl]),

$R_5$ further represents an optionally substituted **quinolyl, benzoxazolyl, benzothiazolyl, benzimidazolyl, and phthalazinyl** (the substituent(s) may be selected from the group consisting of cyano, nitro, fluoro, chloro, methyl, ethyl, methoxy, ethoxy, trifluoromethyl or trifluoromethoxy),

$R_5$ further represents **methoxycarbonyl, carboxyl or its sodium salt**, or one of the following group;

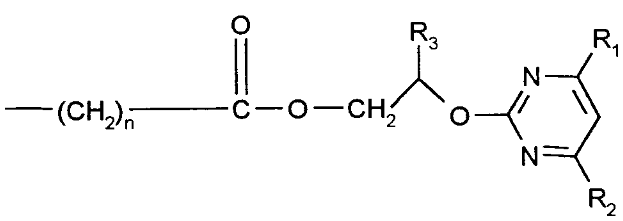

or

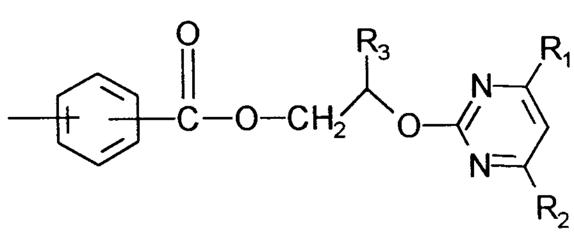

wherein

$R_1$, $R_2$ and $R_3$ have the same meanings as mentioned above, and n represents O, 1, 2, 4, 5 or 6, and

Q represents an optionally halogen-substituted group selected from:

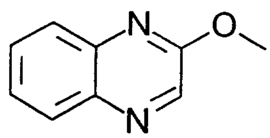 , 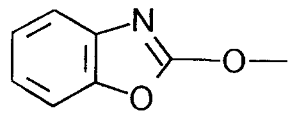 or

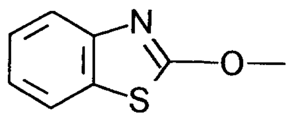

As examples of the compounds of the formula (I) according to the invention, there may be mentioned in the following table 1, 2 and 3 in addition to the compounds that will be enumerated in the Examples hereinafter.

Table 1

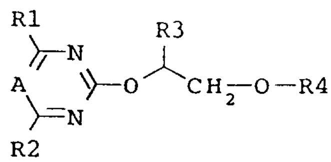

| R1 | R2 | R3 | R4 |
|---|---|---|---|
| $OCH_3$ | $OCH_3$ | $C_2H_5$ | H |
| $OCH_3$ | $OCH_3$ | $CH_2CH_2CH_3$ | H |
| $OCH_3$ | $OCH_3$ | $CH(CH_3)_2$ | H |
| $OCH_3$ | $OCH_3$ | $CH_2CH_2CH_2CH_3$ | H |
| $OCH_3$ | $OCH_3$ | $CH_2CH(CH_3)_2$ | H |
| $OCH_3$ | $OCH_3$ | $CH(CH_3)CH_2CH_3$ | H |
| $OCH_3$ | $OCH_3$ | $C(CH_3)_3$ | H |
| $OCH_3$ | $OCH_3$ | $(CH_2)_4CH_3$ | H |
| $OCH_3$ | $OCH_3$ | $CH(CH_3)(CH_3)_2CH_3$ | H |
| $OCH_3$ | $OCH_3$ | $CH(C_2H_5)_2$ | H |
| $OCH_3$ | $OCH_3$ | $C(CH_3)_2CH_2CH_3$ | H |
| $OCH_3$ | $OCH_3$ | $CH_2C(CH_3)_3$ | H |
| $OCH_3$ | $OCH_3$ | $(CH_2)_2CH(CH_3)_2$ | H |
| $OCH_3$ | $OCH_3$ | $C_2H_5$ | 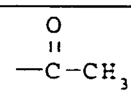 |
| $OCH_3$ | $OCH_3$ | $CH_2CH_2CH_3$ | 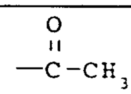 |
| $OCH_3$ | $OCH_3$ | $CH(CH_3)_2$ | 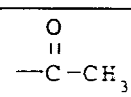 |

Table 1 (continued)

| R1 | R2 | R3 | R4 |
|---|---|---|---|
| $OCH_3$ | $OCH_3$ | $CH_2CH_2CH_2CH_3$ | 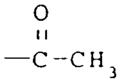 |
| $OCH_3$ | $OCH_3$ | $CH_2CH(CH_3)_2$ | 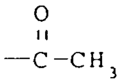 |
| $OCH_3$ | $OCH_3$ | $CH(CH_3)CH_2CH_3$ | 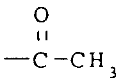 |
| $OCH_3$ | $OCH_3$ | $C(CH_3)_3$ | 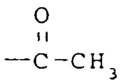 |
| $OCH_3$ | $OCH_3$ | $(CH_2)_4CH_3$ | 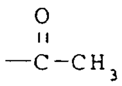 |
| $OCH_3$ | $OCH_3$ | $CH(CH_3)(CH_3)_2CH_3$ | 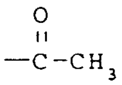 |
| $OCH_3$ | $OCH_3$ | $CH(C_2H_5)_2$ | 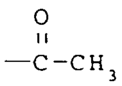 |
| $OCH_3$ | $OCH_3$ | $C(CH_3)_2CH_2CH_3$ | 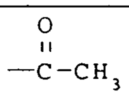 |
| $OCH_3$ | $OCH_3$ | $CH_2C(CH_3)_3$ | 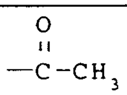 |
| $OCH_3$ | $OCH_3$ | $(CH_2)_2CH(CH_3)_2$ | 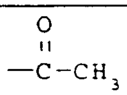 |
| $OCH_3$ | $OCH_3$ | $C_2H_5$ | 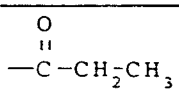 |
| $OCH_3$ | $OCH_3$ | $CH_2CH_2CH_3$ | 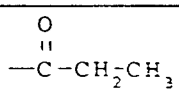 |
| $OCH_3$ | $OCH_3$ | $CH(CH_3)_2$ | 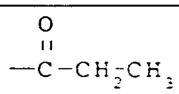 |
| $OCH_3$ | $OCH_3$ | $CH_2CH_2CH_2CH_3$ | 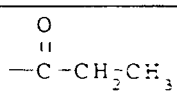 |
| $OCH_3$ | $OCH_3$ | $CH_2CH(CH_3)_2$ | 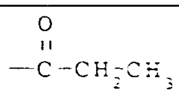 |

Table 1 (continued)

| R1 | R2 | R3 | R4 |
|---|---|---|---|
| $OCH_3$ | $OCH_3$ | $CH(CH_3)CH_2CH_3$ | $-\overset{O}{\overset{\|}{C}}-CH_2-CH_3$ |
| $OCH_3$ | $OCH_3$ | $C(CH_3)_3$ | $-\overset{O}{\overset{\|}{C}}-CH_2-CH_3$ |
| $OCH_3$ | $OCH_3$ | $(CH_2)_4CH_3$ | $-\overset{O}{\overset{\|}{C}}-CH_2-CH_3$ |
| $OCH_3$ | $OCH_3$ | $CH(CH_3)(CH_3)_2CH_3$ | $-\overset{O}{\overset{\|}{C}}-CH_2-CH_3$ |
| $OCH_3$ | $OCH_3$ | $CH(C_2H_5)_2$ | $-\overset{O}{\overset{\|}{C}}-CH_2-CH_3$ |
| $OCH_3$ | $OCH_3$ | $C(CH_3)_2CH_2CH_3$ | $-\overset{O}{\overset{\|}{C}}-CH_2-CH_3$ |
| $OCH_3$ | $OCH_3$ | $CH_2C(CH_3)_3$ | $-\overset{O}{\overset{\|}{C}}-CH_2-CH_3$ |
| $OCH_3$ | $OCH_3$ | $(CH_2)_2CH(CH_3)_2$ | $-\overset{O}{\overset{\|}{C}}-CH_2-CH_3$ |
| $OCH_3$ | $OCH_3$ | $C_2H_5$ | $-\overset{O}{\overset{\|}{C}}-CH_2-CH_2-CH_3$ |
| $OCH_3$ | $OCH_3$ | $CH_2CH_2CH_3$ | $-\overset{O}{\overset{\|}{C}}-CH_2-CH_2-CH_3$ |
| $OCH_3$ | $OCH_3$ | $CH(CH_3)_2$ | $-\overset{O}{\overset{\|}{C}}-CH_2-CH_2-CH_3$ |
| $OCH_3$ | $OCH_3$ | $CH_2CH_2CH_2CH_3$ | $-\overset{O}{\overset{\|}{C}}-CH_2-CH_2-CH_3$ |
| $OCH_3$ | $OCH_3$ | $CH_2CH(CH_3)_2$ | $-\overset{O}{\overset{\|}{C}}-CH_2-CH_2-CH_3$ |
| $OCH_3$ | $OCH_3$ | $CH(CH_3)CH_2CH_3$ | $-\overset{O}{\overset{\|}{C}}-CH_2-CH_2-CH_3$ |
| $OCH_3$ | $OCH_3$ | $C(CH_3)_3$ | $-\overset{O}{\overset{\|}{C}}-CH_2-CH_2-CH_3$ |

Table 1 (continued)

| R1 | R2 | R3 | R4 |
|---|---|---|---|
| $OCH_3$ | $OCH_3$ | $(CH_2)_4CH_3$ | 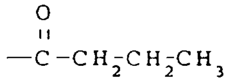 |
| $OCH_3$ | $OCH_3$ | $CH(CH_3)(CH_3)_2CH_3$ | 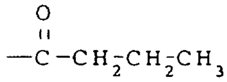 |
| $OCH_3$ | $OCH_3$ | $CH(C_2H_5)_2$ | 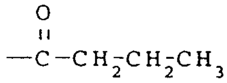 |
| $OCH_3$ | $OCH_3$ | $C(CH_3)_2CH_2CH_3$ | 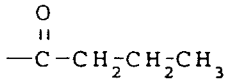 |
| $OCH_3$ | $OCH_3$ | $CH_2C(CH_3)_3$ | 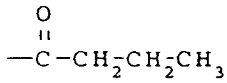 |
| $OCH_3$ | $OCH_3$ | $(CH_2)_2CH(CH_3)_2$ | 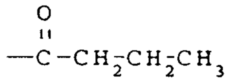 |
| $OCH_3$ | $OCH_3$ | $C_2H_5$ | 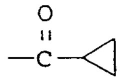 |
| $OCH_3$ | $OCH_3$ | $CH_2CH_2CH_3$ | 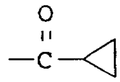 |
| $OCH_3$ | $OCH_3$ | $CH(CH_3)_2$ | 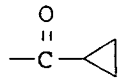 |
| $OCH_3$ | $OCH_3$ | $CH_2CH_2CH_2CH_3$ | 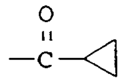 |
| $OCH_3$ | $OCH_3$ | $CH_2CH(CH_3)_2$ | 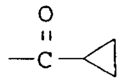 |
| $OCH_3$ | $OCH_3$ | $CH(CH_3)CH_2CH_3$ | 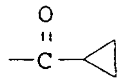 |
| $OCH_3$ | $OCH_3$ | $C(CH_3)_3$ | 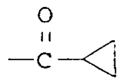 |
| $OCH_3$ | $OCH_3$ | $(CH_2)_4CH_3$ | 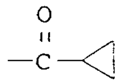 |

Table 1 (continued)

| R1 | R2 | R3 | R4 |
|---|---|---|---|
| $OCH_3$ | $OCH_3$ | $CH(CH_3)(CH_3)_2CH_3$ | 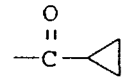 |
| $OCH_3$ | $OCH_3$ | $CH(C_2H_5)_2$ | 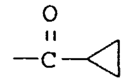 |
| $OCH_3$ | $OCH_3$ | $C(CH_3)_2CH_2CH_3$ | 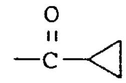 |
| $OCH_3$ | $OCH_3$ | $CH_2C(CH_3)_3$ | 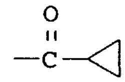 |
| $OCH_3$ | $OCH_3$ | $(CH_2)_2CH(CH_3)_2$ | 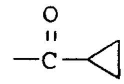 |
| $OCH_3$ | $OCH_3$ | $C_2H_5$ | 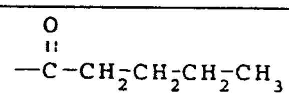 |
| $OCH_3$ | $OCH_3$ | $CH_2CH_2CH_3$ | 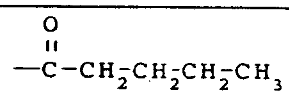 |
| $OCH_3$ | $OCH_3$ | $CH(CH_3)_2$ | 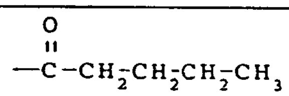 |
| $OCH_3$ | $OCH_3$ | $CH_2CH_2CH_2CH_3$ | 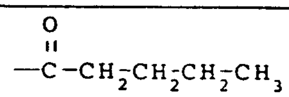 |
| $OCH_3$ | $OCH_3$ | $CH_2CH(CH_3)_2$ | 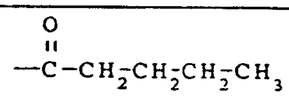 |
| $OCH_3$ | $OCH_3$ | $CH(CH_3)CH_2CH_3$ | 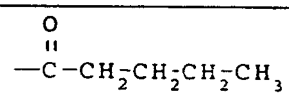 |
| $OCH_3$ | $OCH_3$ | $C(CH_3)_3$ | 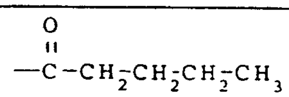 |
| $OCH_3$ | $OCH_3$ | $(CH_2)_4CH_3$ | 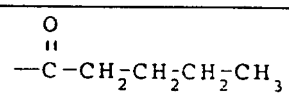 |
| $OCH_3$ | $OCH_3$ | $CH(CH_3)(CH_3)_2CH_3$ | 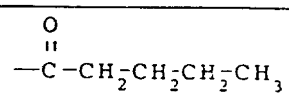 |

Table 1 (continued)

| R1 | R2 | R3 | R4 |
|---|---|---|---|
| $OCH_3$ | $OCH_3$ | $CH(C_2H_5)_2$ | $-\overset{O}{\overset{\|}{C}}-CH_2-CH_2-CH_2-CH_3$ |
| $OCH_3$ | $OCH_3$ | $C(CH_3)_2CH_2CH_3$ | $-\overset{O}{\overset{\|}{C}}-CH_2-CH_2-CH_2-CH_3$ |
| $OCH_3$ | $OCH_3$ | $CH_2C(CH_3)_3$ | $-\overset{O}{\overset{\|}{C}}-CH_2-CH_2-CH_2-CH_3$ |
| $OCH_3$ | $OCH_3$ | $(CH_2)_2CH(CH_3)_2$ | $-\overset{O}{\overset{\|}{C}}-CH_2-CH_2-CH_2-CH_3$ |
| $OCH_3$ | $OCH_3$ | $C_2H_5$ | $-\overset{O}{\overset{\|}{C}}-C(CH_3)_3$ |
| $OCH_3$ | $OCH_3$ | $CH_2CH_2CH_3$ | $-\overset{O}{\overset{\|}{C}}-C(CH_3)_3$ |
| $OCH_3$ | $OCH_3$ | $CH(CH_3)_2$ | $-\overset{O}{\overset{\|}{C}}-C(CH_3)_3$ |
| $OCH_3$ | $OCH_3$ | $CH_2CH_2CH_2CH_3$ | $-\overset{O}{\overset{\|}{C}}-C(CH_3)_3$ |
| $OCH_3$ | $OCH_3$ | $CH_2CH(CH_3)_2$ | $-\overset{O}{\overset{\|}{C}}-C(CH_3)_3$ |
| $OCH_3$ | $OCH_3$ | $CH(CH_3)CH_2CH_3$ | $-\overset{O}{\overset{\|}{C}}-C(CH_3)_3$ |
| $OCH_3$ | $OCH_3$ | $C(CH_3)_3$ | $-\overset{O}{\overset{\|}{C}}-C(CH_3)_3$ |
| $OCH_3$ | $OCH_3$ | $(CH_2)_4CH_3$ | $-\overset{O}{\overset{\|}{C}}-C(CH_3)_3$ |
| $OCH_3$ | $OCH_3$ | $CH(CH_3)(CH_3)_2CH_3$ | $-\overset{O}{\overset{\|}{C}}-C(CH_3)_3$ |
| $OCH_3$ | $OCH_3$ | $CH(C_2H_5)_2$ | $-\overset{O}{\overset{\|}{C}}-C(CH_3)_3$ |

Table 1 (continued)

| R1 | R2 | R3 | R4 |
|---|---|---|---|
| $OCH_3$ | $OCH_3$ | $C(CH_3)_2CH_2CH_3$ | $-\overset{O}{\overset{\|}{C}}-C(CH_3)_3$ |
| $OCH_3$ | $OCH_3$ | $CH_2C(CH_3)_3$ | $-\overset{O}{\overset{\|}{C}}-C(CH_3)_3$ |
| $OCH_3$ | $OCH_3$ | $(CH_2)_2CH(CH_3)_2$ | $-\overset{O}{\overset{\|}{C}}-C(CH_3)_3$ |
| $OCH_3$ | $OCH_3$ | $C_2H_5$ | $-\overset{O}{\overset{\|}{C}}-CH_2-CH(CH_3)_2$ |
| $OCH_3$ | $OCH_3$ | $CH_2CH_2CH_3$ | $-\overset{O}{\overset{\|}{C}}-CH_2-CH(CH_3)_2$ |
| $OCH_3$ | $OCH_3$ | $CH(CH_3)_2$ | $-\overset{O}{\overset{\|}{C}}-CH_2-CH(CH_3)_2$ |
| $OCH_3$ | $OCH_3$ | $CH_2CH_2CH_2CH_3$ | $-\overset{O}{\overset{\|}{C}}-CH_2-CH(CH_3)_2$ |
| $OCH_3$ | $OCH_3$ | $CH_2CH(CH_3)_2$ | $-\overset{O}{\overset{\|}{C}}-CH_2-CH(CH_3)_2$ |
| $OCH_3$ | $OCH_3$ | $CH(CH_3)CH_2CH_3$ | $-\overset{O}{\overset{\|}{C}}-CH_2-CH(CH_3)_2$ |
| $OCH_3$ | $OCH_3$ | $C(CH_3)_3$ | $-\overset{O}{\overset{\|}{C}}-CH_2-CH(CH_3)_2$ |
| $OCH_3$ | $OCH_3$ | $(CH_2)_4CH_3$ | $-\overset{O}{\overset{\|}{C}}-CH_2-CH(CH_3)_2$ |
| $OCH_3$ | $OCH_3$ | $CH(CH_3)(CH_3)_2CH_3$ | $-\overset{O}{\overset{\|}{C}}-CH_2-CH(CH_3)_2$ |
| $OCH_3$ | $OCH_3$ | $CH(C_2H_5)_2$ | $-\overset{O}{\overset{\|}{C}}-CH_2-CH(CH_3)_2$ |
| $OCH_3$ | $OCH_3$ | $C(CH_3)_2CH_2CH_3$ | $-\overset{O}{\overset{\|}{C}}-CH_2-CH(CH_3)_2$ |

Table 1 (continued)

| R1 | R2 | R3 | R4 |
|---|---|---|---|
| $OCH_3$ | $OCH_3$ | $CH_2C(CH_3)_3$ | 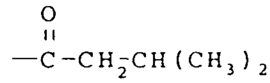 |
| $OCH_3$ | $OCH_3$ | $(CH_2)_2CH(CH_3)_2$ | 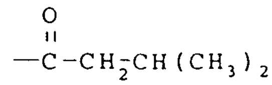 |
| $OCH_3$ | $OCH_3$ | $C_2H_5$ | 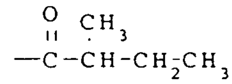 |
| $OCH_3$ | $OCH_3$ | $CH_2CH_2CH_3$ | 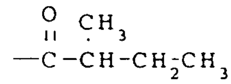 |
| $OCH_3$ | $OCH_3$ | $CH(CH_3)_2$ | 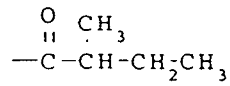 |
| $OCH_3$ | $OCH_3$ | $CH_2CH_2CH_2CH_3$ | 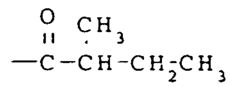 |
| $OCH_3$ | $OCH_3$ | $CH_2CH(CH_3)_2$ | 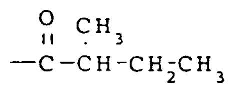 |
| $OCH_3$ | $OCH_3$ | $CH(CH_3)CH_2CH_3$ | 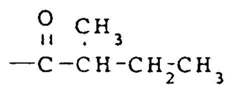 |
| $OCH_3$ | $OCH_3$ | $C(CH_3)_3$ | 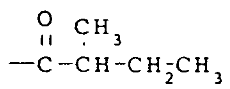 |
| $OCH_3$ | $OCH_3$ | $(CH_2)_4CH_3$ | 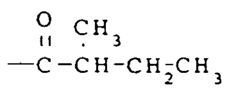 |
| $OCH_3$ | $OCH_3$ | $CH(CH_3)(CH_3)_2CH_3$ | 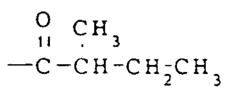 |
| $OCH_3$ | $OCH_3$ | $CH(C_2H_5)_2$ | 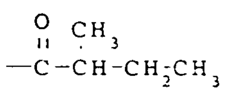 |
| $OCH_3$ | $OCH_3$ | $C(CH_3)_2CH_2CH_3$ | 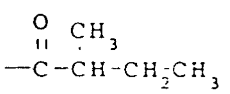 |
| $OCH_3$ | $OCH_3$ | $CH_2C(CH_3)_3$ | 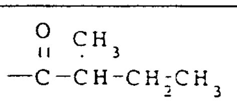 |

Table 1 (continued)

| R1 | R2 | R3 | R4 |
|---|---|---|---|
| $OCH_3$ | $OCH_3$ | $(CH_2)_2CH(CH_3)_2$ | 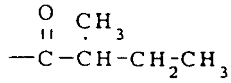 |
| $OCH_3$ | $OCH_3$ | $C_2H_5$ | 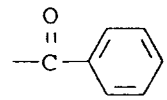 |
| $OCH_3$ | $OCH_3$ | $CH_2CH_2CH_3$ | 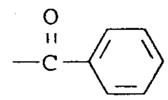 |
| $OCH_3$ | $OCH_3$ | $CH(CH_3)_2$ | 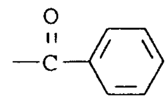 |
| $OCH_3$ | $OCH_3$ | $CH_2CH_2CH_2CH_3$ | 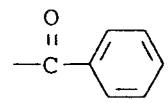 |
| $OCH_3$ | $OCH_3$ | $CH_2CH(CH_3)_2$ | 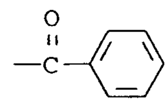 |
| $OCH_3$ | $OCH_3$ | $CH(CH_3)CH_2CH_3$ | 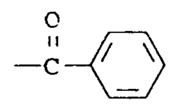 |
| $OCH_3$ | $OCH_3$ | $C(CH_3)_3$ | 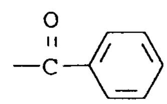 |
| $OCH_3$ | $OCH_3$ | $(CH_2)_4CH_3$ | 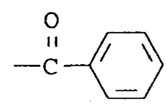 |
| $OCH_3$ | $OCH_3$ | $CH(CH_3)(CH_3)_2CH_3$ | 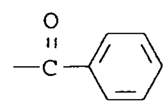 |
| $OCH_3$ | $OCH_3$ | $CH(C_2H_5)_2$ | 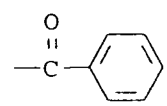 |
| $OCH_3$ | $OCH_3$ | $C(CH_3)_2CH_2CH_3$ | 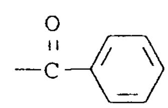 |

Table 1 (continued)

| R1 | R2 | R3 | R4 |
|---|---|---|---|
| $OCH_3$ | $OCH_3$ | $CH_2C(CH_3)_3$ | 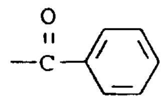 |
| $OCH_3$ | $OCH_3$ | $(CH_2)_2CH(CH_3)_2$ | 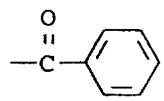 |
| $OCH_3$ | $OCH_3$ | $C_2H_5$ | 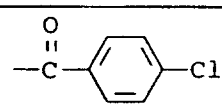 |
| $OCH_3$ | $OCH_3$ | $CH_2CH_2CH_3$ | 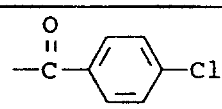 |
| $OCH_3$ | $OCH_3$ | $CH(CH_3)_2$ | 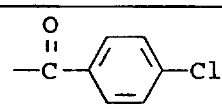 |
| $OCH_3$ | $OCH_3$ | $CH_2CH_2CH_2CH_3$ | 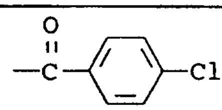 |
| $OCH_3$ | $OCH_3$ | $CH_2CH(CH_3)_2$ | 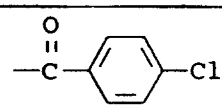 |
| $OCH_3$ | $OCH_3$ | $CH(CH_3)CH_2CH_3$ | 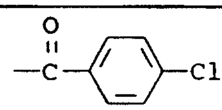 |
| $OCH_3$ | $OCH_3$ | $C(CH_3)_3$ | 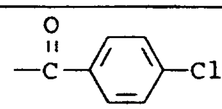 |
| $OCH_3$ | $OCH_3$ | $(CH_2)_4CH_3$ | 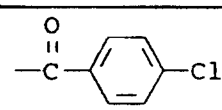 |
| $OCH_3$ | $OCH_3$ | $CH(CH_3)(CH_3)_2CH_3$ | 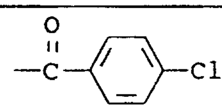 |
| $OCH_3$ | $OCH_3$ | $CH(C_2H_5)_2$ | 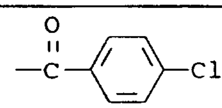 |

Table 1 (continued)

| R1 | R2 | R3 | R4 |
|---|---|---|---|
| $OCH_3$ | $OCH_3$ | $C(CH_3)_2CH_2CH_3$ | 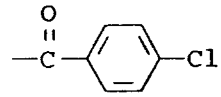 |
| $OCH_3$ | $OCH_3$ | $CH_2C(CH_3)_3$ | 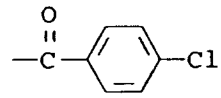 |
| $OCH_3$ | $OCH_3$ | $(CH_2)_2CH(CH_3)_2$ | 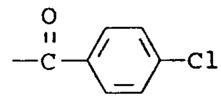 |
| $OCH_3$ | $OCH_3$ | $CH_2CH_2CH_3$ | O<br>‖<br>$—C-(CH_2)_4-CH_3$ |
| $OCH_3$ | $OCH_3$ | $CH(CH_3)_2$ | O<br>‖<br>$—C-(CH_2)_4-CH_3$ |
| $OCH_3$ | $OCH_3$ | $C(CH_3)_3$ | O<br>‖<br>$—C-(CH_2)_4-CH_3$ |
| $OCH_3$ | $OCH_3$ | $CH_2CH_2CH_3$ | O<br>‖<br>$—C-(CH_2)_5-CH_3$ |
| $OCH_3$ | $OCH_3$ | $CH(CH_3)_2$ | O<br>‖<br>$—C-(CH_2)_5-CH_3$ |
| $OCH_3$ | $OCH_3$ | $C(CH_3)_3$ | O<br>‖<br>$—C-(CH_2)_5-CH_3$ |
| $OCH_3$ | $OCH_3$ | $CH_2CH_2CH_3$ | O<br>‖<br>$—C-(CH_2)_6-CH_3$ |
| $OCH_3$ | $OCH_3$ | $CH(CH_3)_2$ | O<br>‖<br>$—C-(CH_2)_6-CH_3$ |
| $OCH_3$ | $OCH_3$ | $C(CH_3)_3$ | O<br>‖<br>$—C-(CH_2)_6-CH_3$ |
| $OCH_3$ | $OCH_3$ | $CH_2CH_2CH_3$ | O<br>‖<br>$—C-(CH_2)_7-CH_3$ |
| $OCH_3$ | $OCH_3$ | $CH(CH_3)_2$ | O<br>‖<br>$—C-(CH_2)_7-CH_3$ |

Table 1 (continued)

| R1 | R2 | R3 | R4 |
|---|---|---|---|
| $OCH_3$ | $OCH_3$ | $C(CH_3)_3$ | $-\overset{O}{\overset{\|}{C}}-(CH_2)_7-CH_3$ |
| $OCH_3$ | $OCH_3$ | $CH_2CH_2CH_3$ | $-\overset{O}{\overset{\|}{C}}-(CH_2)_8-CH_3$ |
| $OCH_3$ | $OCH_3$ | $CH(CH_3)_2$ | $-\overset{O}{\overset{\|}{C}}-(CH_2)_8-CH_3$ |
| $OCH_3$ | $OCH_3$ | $C(CH_3)_3$ | $-\overset{O}{\overset{\|}{C}}-(CH_2)_8-CH_3$ |
| $OCH_3$ | $OCH_3$ | $CH_2CH_2CH_3$ | $-\overset{O}{\overset{\|}{C}}-(CH_2)_9-CH_3$ |
| $OCH_3$ | $OCH_3$ | $CH(CH_3)_2$ | $-\overset{O}{\overset{\|}{C}}-(CH_2)_9-CH_3$ |
| $OCH_3$ | $OCH_3$ | $C(CH_3)_3$ | $-\overset{O}{\overset{\|}{C}}-(CH_2)_9-CH_3$ |
| $OCH_3$ | $OCH_3$ | $CH_2CH_2CH_3$ | $-\overset{O}{\overset{\|}{C}}-(CH_2)_{10}-CH_3$ |
| $OCH_3$ | $OCH_3$ | $CH(CH_3)_2$ | $-\overset{O}{\overset{\|}{C}}-(CH_2)_{10}-CH_3$ |
| $OCH_3$ | $OCH_3$ | $C(CH_3)_3$ | $-\overset{O}{\overset{\|}{C}}-(CH_2)_{10}-CH_3$ |
| $OCH_3$ | $OCH_3$ | $CH_2CH_2CH_3$ | $-\overset{O}{\overset{\|}{C}}-(CH_2)_{11}-CH_3$ |
| $OCH_3$ | $OCH_3$ | $CH(CH_3)_2$ | $-\overset{O}{\overset{\|}{C}}-(CH_2)_{11}-CH_3$ |
| $OCH_3$ | $OCH_3$ | $C(CH_3)_3$ | $-\overset{O}{\overset{\|}{C}}-(CH_2)_{11}-CH_3$ |
| $OCH_3$ | $OCH_3$ | $CH_2CH_2CH_3$ | $-\overset{O}{\overset{\|}{C}}-(CH_2)_{12}-CH_3$ |

Table 1 (continued)

| R1 | R2 | R3 | R4 |
|---|---|---|---|
| $OCH_3$ | $OCH_3$ | $CH(CH_3)_2$ | 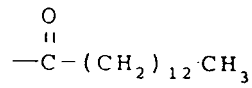 |
| $OCH_3$ | $OCH_3$ | $C(CH_3)_3$ | 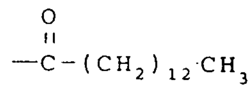 |
| $OCH_3$ | $OCH_3$ | $CH_2CH_2CH_3$ | 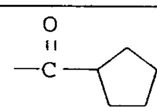 |
| $OCH_3$ | $OCH_3$ | $CH(CH_3)_2$ | 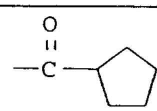 |
| $OCH_3$ | $OCH_3$ | $C(CH_3)_3$ | 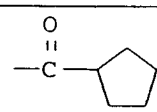 |
| $OCH_3$ | $OCH_3$ | $CH_2CH_2CH_3$ | 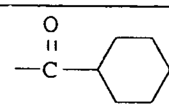 |
| $OCH_3$ | $OCH_3$ | $CH(CH_3)_2$ | 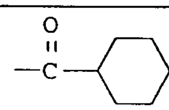 |
| $OCH_3$ | $OCH_3$ | $C(CH_3)_3$ | 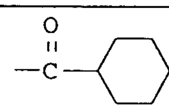 |
| $OCH_3$ | $OCH_3$ | $CH_2CH_2CH_3$ | 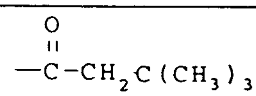 |
| $OCH_3$ | $OCH_3$ | $CH(CH_3)_2$ | 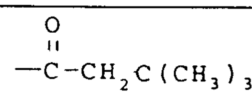 |
| $OCH_3$ | $OCH_3$ | $C(CH_3)_3$ | 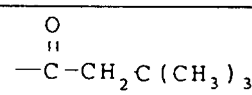 |
| $OCH_3$ | $OCH_3$ | $CH_2CH_2CH_3$ | 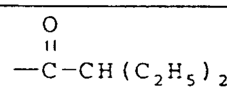 |
| $OCH_3$ | $OCH_3$ | $CH(CH_3)_2$ | 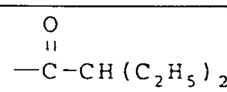 |

Table 1 (continued)

| R1 | R2 | R3 | R4 |
|---|---|---|---|
| $OCH_3$ | $OCH_3$ | $C(CH_3)_3$ | $\overset{O}{\overset{\|}{-C}}-CH(C_2H_5)_2$ |
| $OCH_3$ | $OCH_3$ | $CH_2CH_2CH_3$ | $\overset{O}{\overset{\|}{-C}}-(CH_2)_2CH(CH_3)_2$ |
| $OCH_3$ | $OCH_3$ | $CH(CH_3)_2$ | $\overset{O}{\overset{\|}{-C}}-(CH_2)_2CH(CH_3)_2$ |
| $OCH_3$ | $OCH_3$ | $C(CH_3)_3$ | $\overset{O}{\overset{\|}{-C}}-(CH_2)_2CH(CH_3)_2$ |
| $OCH_3$ | $OCH_3$ | $CH_2CH_2CH_3$ | $\overset{O}{\overset{\|}{-C}}-CH(CH_3)(CH_2)_2CH_3$ |
| $OCH_3$ | $OCH_3$ | $CH(CH_3)_2$ | $\overset{O}{\overset{\|}{-C}}-CH(CH_3)(CH_2)_2CH_3$ |
| $OCH_3$ | $OCH_3$ | $C(CH_3)_3$ | $\overset{O}{\overset{\|}{-C}}-CH(CH_3)(CH_2)_2CH_3$ |
| $OCH_3$ | $OCH_3$ | $CH_2CH_2CH_3$ | $\overset{O}{\overset{\|}{-C}}-CH(CH_3)(CH_2)_3CH_3$ |
| $OCH_3$ | $OCH_3$ | $CH(CH_3)_2$ | $\overset{O}{\overset{\|}{-C}}-CH(CH_3)(CH_2)_3CH_3$ |
| $OCH_3$ | $OCH_3$ | $C(CH_3)_3$ | $\overset{O}{\overset{\|}{-C}}-CH(CH_3)(CH_2)_3CH_3$ |
| $OCH_3$ | $OCH_3$ | $CH_2CH_2CH_3$ | $\overset{O}{\overset{\|}{-C}}-CH(C_2H_5)(CH_2)_2CH_3$ |
| $OCH_3$ | $OCH_3$ | $CH(CH_3)_2$ | $\overset{O}{\overset{\|}{-C}}-CH(C_2H_5)(CH_2)_2CH_3$ |
| $OCH_3$ | $OCH_3$ | $C(CH_3)_3$ | $\overset{O}{\overset{\|}{-C}}-CH(C_2H_5)(CH_2)_2CH_3$ |
| $OCH_3$ | $OCH_3$ | $CH_2CH_2CH_3$ | $\overset{O}{\overset{\|}{-C}}-C(C_2H_5)(CH_3)_2$ |

Table 1 (continued)

| R1 | R2 | R3 | R4 |
|---|---|---|---|
| $OCH_3$ | $OCH_3$ | $CH(CH_3)_2$ | $-\overset{\overset{O}{\|}}{C}-C(C_2H_5)(CH_3)_2$ |
| $OCH_3$ | $OCH_3$ | $C(CH_3)_3$ | $-\overset{\overset{O}{\|}}{C}-C(C_2H_5)(CH_3)_2$ |
| $OCH_3$ | $OCH_3$ | $CH_2CH_2CH_3$ | $-\overset{\overset{O}{\|}}{C}-CH(C_2H_5)(CH_2)_3CH_3$ |
| $OCH_3$ | $OCH_3$ | $CH(CH_3)_2$ | $-\overset{\overset{O}{\|}}{C}-CH(C_2H_5)(CH_2)_3CH_3$ |
| $OCH_3$ | $OCH_3$ | $C(CH_3)_3$ | $-\overset{\overset{O}{\|}}{C}-CH(C_2H_5)(CH_2)_3CH_3$ |
| $OCH_3$ | $OCH_3$ | $CH_2CH_2CH_3$ | $-\overset{\overset{O}{\|}}{C}-CH_2CH(CH_3)C_2H_5$ |
| $OCH_3$ | $OCH_3$ | $CH(CH_3)_2$ | $-\overset{\overset{O}{\|}}{C}-CH_2CH(CH_3)C_2H_5$ |
| $OCH_3$ | $OCH_3$ | $C(CH_3)_3$ | $-\overset{\overset{O}{\|}}{C}-CH_2CH(CH_3)C_2H_5$ |
| $OCH_3$ | $OCH_3$ | $CH_2CH_2CH_3$ | $-\overset{\overset{O}{\|}}{C}-H$ |
| $OCH_3$ | $OCH_3$ | $CH(CH_3)_2$ | $-\overset{\overset{O}{\|}}{C}-H$ |
| $OCH_3$ | $OCH_3$ | $C(CH_3)_3$ | $-\overset{\overset{O}{\|}}{C}-H$ |
| $OCH_3$ | $OCH_3$ | $CH_2CH_2CH_3$ | $-\overset{\overset{O}{\|}}{C}-(CH_2)_{13}CH_3$ |
| $OCH_3$ | $OCH_3$ | $CH(CH_3)_2$ | $-\overset{\overset{O}{\|}}{C}-(CH_2)_{13}CH_3$ |
| $OCH_3$ | $OCH_3$ | $C(CH_3)_3$ | $-\overset{\overset{O}{\|}}{C}-(CH_2)_{13}CH_3$ |

Table 1 (continued)

| R1 | R2 | R3 | R4 |
|---|---|---|---|
| $OCH_3$ | $OCH_3$ | $CH_2CH_2CH_3$ | $-\overset{O}{\overset{\|}{C}}-(CH_2)_{14}CH_3$ |
| $OCH_3$ | $OCH_3$ | $CH(CH_3)_2$ | $-\overset{O}{\overset{\|}{C}}-(CH_2)_{14}CH_3$ |
| $OCH_3$ | $OCH_3$ | $C(CH_3)_3$ | $-\overset{O}{\overset{\|}{C}}-(CH_2)_{14}CH_3$ |
| $OCH_3$ | $OCH_3$ | $CH_2CH_2CH_3$ | $-\overset{O}{\overset{\|}{C}}-(CH_2)_{15}CH_3$ |
| $OCH_3$ | $OCH_3$ | $CH(CH_3)_2$ | $-\overset{O}{\overset{\|}{C}}-(CH_2)_{15}CH_3$ |
| $OCH_3$ | $OCH_3$ | $C(CH_3)_3$ | $-\overset{O}{\overset{\|}{C}}-(CH_2)_{15}CH_3$ |
| $OCH_3$ | $OCH_3$ | $CH_2CH_2CH_3$ | $-\overset{O}{\overset{\|}{C}}-(CH_2)_{16}CH_3$ |
| $OCH_3$ | $OCH_3$ | $CH(CH_3)_2$ | $-\overset{O}{\overset{\|}{C}}-(CH_2)_{16}CH_3$ |
| $OCH_3$ | $OCH_3$ | $C(CH_3)_3$ | $-\overset{O}{\overset{\|}{C}}-(CH_2)_{16}CH_3$ |
| $OCH_3$ | $OCH_3$ | $CH_2CH_2CH_3$ | $-\overset{O}{\overset{\|}{C}}-(CH_2)_{17}CH_3$ |
| $OCH_3$ | $OCH_3$ | $CH(CH_3)_2$ | $-\overset{O}{\overset{\|}{C}}-(CH_2)_{17}CH_3$ |
| $OCH_3$ | $OCH_3$ | $C(CH_3)_3$ | $-\overset{O}{\overset{\|}{C}}-(CH_2)_{17}CH_3$ |
| $OCH_3$ | $OCH_3$ | $CH_2CH_2CH_3$ | $-\overset{O}{\overset{\|}{C}}-CH(CH_2CH_2CH_3)_2$ |
| $OCH_3$ | $OCH_3$ | $CH(CH_3)_2$ | $-\overset{O}{\overset{\|}{C}}-CH(CH_2CH_2CH_3)_2$ |

Table 1 (continued)

| R1 | R2 | R3 | R4 |
|---|---|---|---|
| $OCH_3$ | $OCH_3$ | $C(CH_3)_3$ | 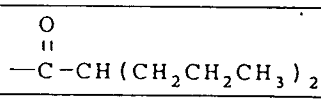 |
| $OCH_3$ | $OCH_3$ | $CH_2CH_2CH_3$ | 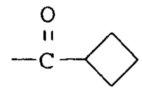 |
| $OCH_3$ | $OCH_3$ | $CH(CH_3)_2$ | 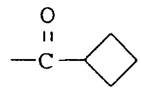 |
| $OCH_3$ | $OCH_3$ | $C(CH_3)_3$ | 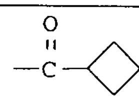 |
| $OCH_3$ | $OCH_3$ | $CH_2CH_2CH_3$ | 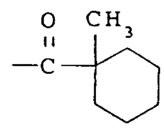 |
| $OCH_3$ | $OCH_3$ | $CH(CH_3)_2$ | 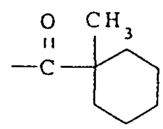 |
| $OCH_3$ | $OCH_3$ | $C(CH_3)_3$ | 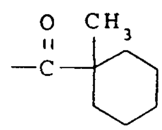 |
| $OCH_3$ | $OCH_3$ | $CH_2CH_2CH_3$ | 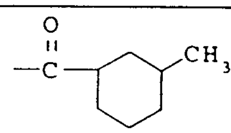 |
| $OCH_3$ | $OCH_3$ | $CH(CH_3)_2$ | 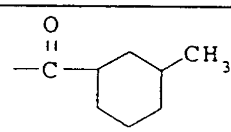 |
| $OCH_3$ | $OCH_3$ | $C(CH_3)_3$ | 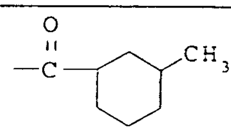 |
| $OCH_3$ | $OCH_3$ | $CH_2CH_2CH_3$ | 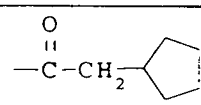 |

Table 1 (continued)

| R1 | R2 | R3 | R4 |
|---|---|---|---|
| $OCH_3$ | $OCH_3$ | $CH(CH_3)_2$ | 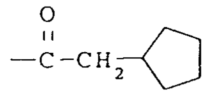 |
| $OCH_3$ | $OCH_3$ | $C(CH_3)_3$ | 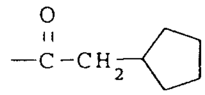 |
| $OCH_3$ | $OCH_3$ | $CH_2CH_2CH_3$ | 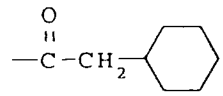 |
| $OCH_3$ | $OCH_3$ | $CH(CH_3)_2$ | 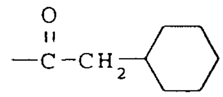 |
| $OCH_3$ | $OCH_3$ | $C(CH_3)_3$ | 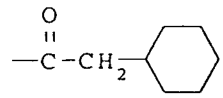 |
| $OCH_3$ | $OCH_3$ | $CH_2CH_2CH_3$ | 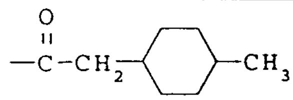 |
| $OCH_3$ | $OCH_3$ | $CH(CH_3)_2$ | 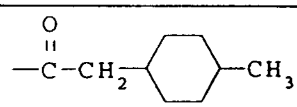 |
| $OCH_3$ | $OCH_3$ | $C(CH_3)_3$ | 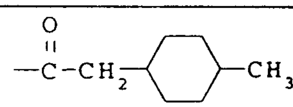 |
| $OCH_3$ | $OCH_3$ | $CH_2CH_2CH_3$ | 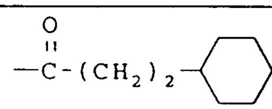 |
| $OCH_3$ | $OCH_3$ | $CH(CH_3)_2$ | 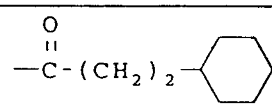 |
| $OCH_3$ | $OCH_3$ | $C(CH_3)_3$ | 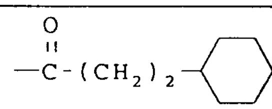 |

Table 1 (continued)

| R1 | R2 | R3 | R4 |
|---|---|---|---|
| $OCH_3$ | $OCH_3$ | $CH_2CH_2CH_3$ | 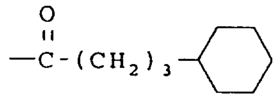 |
| $OCH_3$ | $OCH_3$ | $CH(CH_3)_2$ | 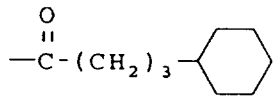 |
| $OCH_3$ | $OCH_3$ | $C(CH_3)_3$ | 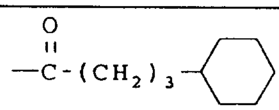 |
| $OCH_3$ | $OCH_3$ | $CH_2CH_2CH_3$ | 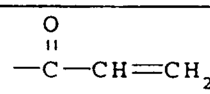 |
| $OCH_3$ | $OCH_3$ | $CH(CH_3)_2$ | 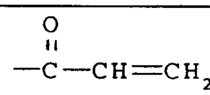 |
| $OCH_3$ | $OCH_3$ | $C(CH_3)_3$ | 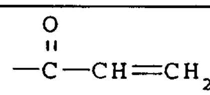 |
| $OCH_3$ | $OCH_3$ | $CH_2CH_2CH_3$ | 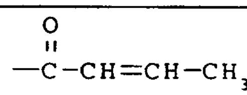 |
| $OCH_3$ | $OCH_3$ | $CH(CH_3)_2$ | 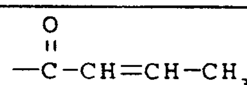 |
| $OCH_3$ | $OCH_3$ | $C(CH_3)_3$ | 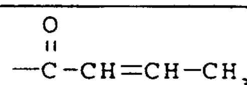 |
| $OCH_3$ | $OCH_3$ | $CH_2CH_2CH_3$ | 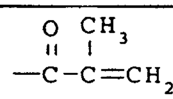 |
| $OCH_3$ | $OCH_3$ | $CH(CH_3)_2$ | 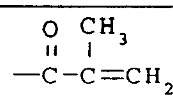 |
| $OCH_3$ | $OCH_3$ | $C(CH_3)_3$ | 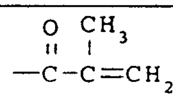 |
| $OCH_3$ | $OCH_3$ | $CH_2CH_2CH_3$ | 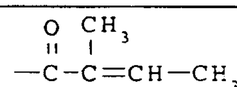 |
| $OCH_3$ | $OCH_3$ | $CH(CH_3)_2$ | 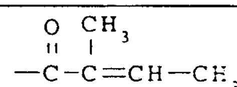 |

Table 1 (continued)

| R1 | R2 | R3 | R4 |
|---|---|---|---|
| $OCH_3$ | $OCH_3$ | $C(CH_3)_3$ | 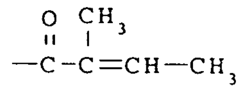 |
| $OCH_3$ | $OCH_3$ | $CH_2CH_2CH_3$ | 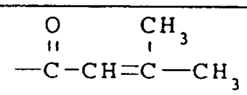 |
| $OCH_3$ | $OCH_3$ | $CH(CH_3)_2$ | 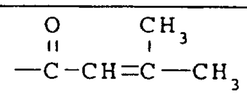 |
| $OCH_3$ | $OCH_3$ | $C(CH_3)_3$ | 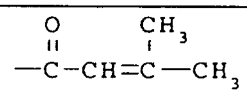 |
| $OCH_3$ | $OCH_3$ | $CH_2CH_2CH_3$ | 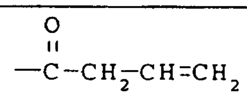 |
| $OCH_3$ | $OCH_3$ | $CH(CH_3)_2$ | 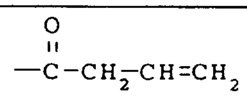 |
| $OCH_3$ | $OCH_3$ | $C(CH_3)_3$ | 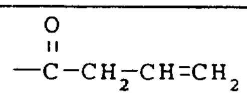 |
| $OCH_3$ | $OCH_3$ | $CH_2CH_2CH_3$ | 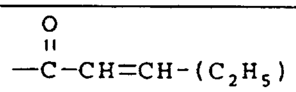 |
| $OCH_3$ | $OCH_3$ | $CH(CH_3)_2$ | 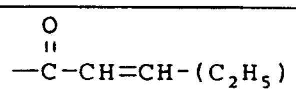 |
| $OCH_3$ | $OCH_3$ | $C(CH_3)_3$ | 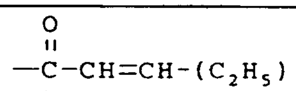 |
| $OCH_3$ | $OCH_3$ | $CH_2CH_2CH_3$ | 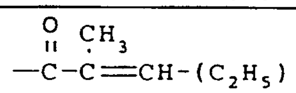 |
| $OCH_3$ | $OCH_3$ | $CH(CH_3)_2$ | 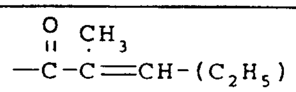 |
| $OCH_3$ | $OCH_3$ | $C(CH_3)_3$ | 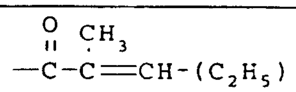 |
| $OCH_3$ | $OCH_3$ | $CH_2CH_2CH_3$ | 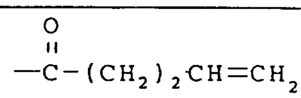 |

Table 1 (continued)

| R1 | R2 | R3 | R4 |
|---|---|---|---|
| $OCH_3$ | $OCH_3$ | $CH(CH_3)_2$ | $-\overset{O}{\overset{\|}{C}}-(CH_2)_2CH{=}CH_2$ |
| $OCH_3$ | $OCH_3$ | $C(CH_3)_3$ | $-\overset{O}{\overset{\|}{C}}-(CH_2)_2CH{=}CH_2$ |
| $OCH_3$ | $OCH_3$ | $CH_2CH_2CH_3$ | $-\overset{O}{\overset{\|}{C}}-CH_2-CH{=}CH-C_2H_5$ |
| $OCH_3$ | $OCH_3$ | $CH(CH_3)_2$ | $-\overset{O}{\overset{\|}{C}}-CH_2-CH{=}CH-C_2H_5$ |
| $OCH_3$ | $OCH_3$ | $C(CH_3)_3$ | $-\overset{O}{\overset{\|}{C}}-CH_2-CH{=}CH-C_2H_5$ |
| $OCH_3$ | $OCH_3$ | $CH_2CH_2CH_3$ | $-\overset{O}{\overset{\|}{C}}-CH{=}CH-(CH_2)_2CH_3$ |
| $OCH_3$ | $OCH_3$ | $CH(CH_3)_2$ | $-\overset{O}{\overset{\|}{C}}-CH{=}CH-(CH_2)_2CH_3$ |
| $OCH_3$ | $OCH_3$ | $C(CH_3)_3$ | $-\overset{O}{\overset{\|}{C}}-CH{=}CH-(CH_2)_2CH_3$ |
| $OCH_3$ | $OCH_3$ | $CH_2CH_2CH_3$ | $-\overset{O}{\overset{\|}{C}}-CH{=}CH-(CH_2)_4CH_3$ |
| $OCH_3$ | $OCH_3$ | $CH(CH_3)_2$ | $-\overset{O}{\overset{\|}{C}}-CH{=}CH-(CH_2)_4CH_3$ |
| $OCH_3$ | $OCH_3$ | $C(CH_3)_3$ | $-\overset{O}{\overset{\|}{C}}-CH{=}CH-(CH_2)_4CH_3$ |
| $OCH_3$ | $OCH_3$ | $CH_2CH_2CH_3$ | $-\overset{O}{\overset{\|}{C}}-(CH_2)_4CH{=}CH_2$ |
| $OCH_3$ | $OCH_3$ | $CH(CH_3)_2$ | $-\overset{O}{\overset{\|}{C}}-(CH_2)_4CH{=}CH_2$ |
| $OCH_3$ | $OCH_3$ | $C(CH_3)_3$ | $-\overset{O}{\overset{\|}{C}}-(CH_2)_4CH{=}CH_2$ |

Table 1 (continued)

| R1 | R2 | R3 | R4 |
|---|---|---|---|
| $OCH_3$ | $OCH_3$ | $CH_2CH_2CH_3$ | 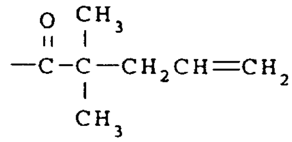 |
| $OCH_3$ | $OCH_3$ | $CH(CH_3)_2$ | 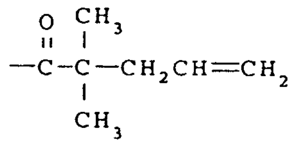 |
| $OCH_3$ | $OCH_3$ | $C(CH_3)_3$ | 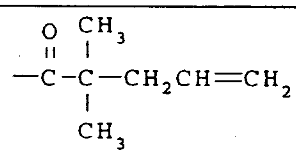 |
| $OCH_3$ | $OCH_3$ | $CH_2CH_2CH_3$ | 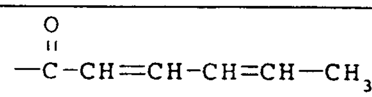 |
| $OCH_3$ | $OCH_3$ | $CH(CH_3)_2$ | 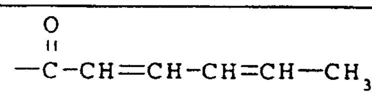 |
| $OCH_3$ | $OCH_3$ | $C(CH_3)_3$ | 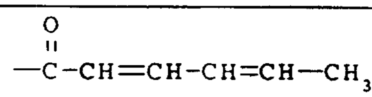 |
| $OCH_3$ | $OCH_3$ | $CH_2CH_2CH_3$ | 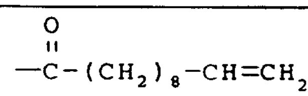 |
| $OCH_3$ | $OCH_3$ | $CH(CH_3)_2$ | 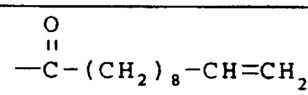 |
| $OCH_3$ | $OCH_3$ | $C(CH_3)_3$ | 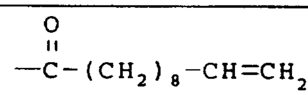 |
| $OCH_3$ | $OCH_3$ | $CH_2CH_2CH_3$ | 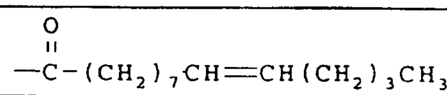 |
| $OCH_3$ | $OCH_3$ | $CH(CH_3)_2$ | 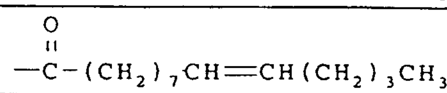 |
| $OCH_3$ | $OCH_3$ | $C(CH_3)_3$ | 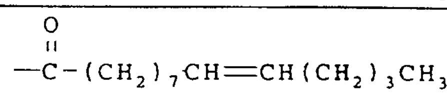 |
| $OCH_3$ | $OCH_3$ | $CH_2CH_2CH_3$ | 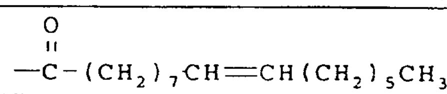 |
| $OCH_3$ | $OCH_3$ | $CH(CH_3)_2$ | 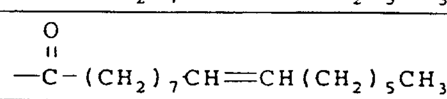 |

Table 1 (continued)

| R1 | R2 | R3 | R4 |
|---|---|---|---|
| $OCH_3$ | $OCH_3$ | $C(CH_3)_3$ | $-\overset{O}{\overset{\|}{C}}-(CH_2)_7CH=CH(CH_2)_5CH_3$ |
| $OCH_3$ | $OCH_3$ | $CH_2CH_2CH_3$ | $-\overset{O}{\overset{\|}{C}}-(CH_2)_7CH=CH(CH_2)_7CH_3$ |
| $OCH_3$ | $OCH_3$ | $CH(CH_3)_2$ | $-\overset{O}{\overset{\|}{C}}-(CH_2)_7CH=CH(CH_2)_7CH_3$ |
| $OCH_3$ | $OCH_3$ | $C(CH_3)_3$ | $-\overset{O}{\overset{\|}{C}}-(CH_2)_7CH=CH(CH_2)_7CH_3$ |
| $OCH_3$ | $OCH_3$ | $CH_2CH_2CH_3$ | $-\overset{O}{\overset{\|}{C}}-(CH_2)_9CH=CH(CH_2)_7CH_3$ |
| $OCH_3$ | $OCH_3$ | $CH(CH_3)_2$ | $-\overset{O}{\overset{\|}{C}}-(CH_2)_9CH=CH(CH_2)_7CH_3$ |
| $OCH_3$ | $OCH_3$ | $C(CH_3)_3$ | $-\overset{O}{\overset{\|}{C}}-(CH_2)_9CH=CH(CH_2)_7CH_3$ |
| $OCH_3$ | $OCH_3$ | $CH_2CH_2CH_3$ | $-\overset{O}{\overset{\|}{C}}-(CH_2)_{19}CH_3$ |
| $OCH_3$ | $OCH_3$ | $CH(CH_3)_2$ | $-\overset{O}{\overset{\|}{C}}-(CH_2)_{19}CH_3$ |
| $OCH_3$ | $OCH_3$ | $C(CH_3)_3$ | $-\overset{O}{\overset{\|}{C}}-(CH_2)_{19}CH_3$ |
| $OCH_3$ | $OCH_3$ | $CH_2CH_2CH_3$ | $-\overset{O}{\overset{\|}{C}}-C\equiv CH$ |
| $OCH_3$ | $OCH_3$ | $CH(CH_3)_2$ | $-\overset{O}{\overset{\|}{C}}-C\equiv CH$ |
| $OCH_3$ | $OCH_3$ | $C(CH_3)_3$ | $-\overset{O}{\overset{\|}{C}}-C\equiv CH$ |
| $OCH_3$ | $OCH_3$ | $CH_2CH_2CH_3$ | $-\overset{O}{\overset{\|}{C}}-C\equiv C-CH_3$ |
| $OCH_3$ | $OCH_3$ | $CH(CH_3)_2$ | $-\overset{O}{\overset{\|}{C}}-C\equiv C-CH_3$ |

Table 1 (continued)

| R1 | R2 | R3 | R4 |
|---|---|---|---|
| $OCH_3$ | $OCH_3$ | $C(CH_3)_3$ | 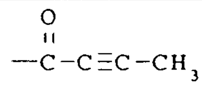 |
| $OCH_3$ | $OCH_3$ | $CH_2CH_2CH_3$ | 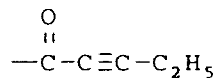 |
| $OCH_3$ | $OCH_3$ | $CH(CH_3)_2$ | 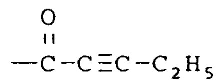 |
| $OCH_3$ | $OCH_3$ | $C(CH_3)_3$ | 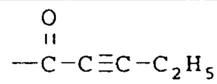 |
| $OCH_3$ | $OCH_3$ | $CH_2CH_2CH_3$ | 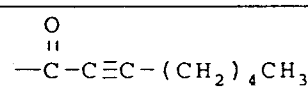 |
| $OCH_3$ | $OCH_3$ | $CH(CH_3)_2$ | 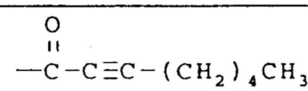 |
| $OCH_3$ | $OCH_3$ | $C(CH_3)_3$ | 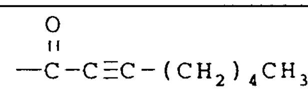 |
| $OCH_3$ | $OCH_3$ | $CH_2CH_2CH_3$ | 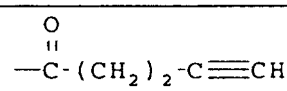 |
| $OCH_3$ | $OCH_3$ | $CH(CH_3)_2$ | 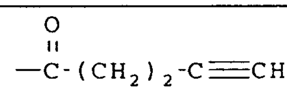 |
| $OCH_3$ | $OCH_3$ | $C(CH_3)_3$ | 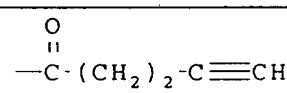 |
| $OCH_3$ | $OCH_3$ | $CH_2CH_2CH_3$ | 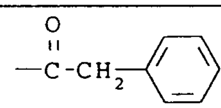 |
| $OCH_3$ | $OCH_3$ | $CH(CH_3)_2$ | 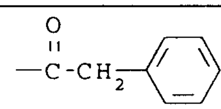 |
| $OCH_3$ | $OCH_3$ | $C(CH_3)_3$ | 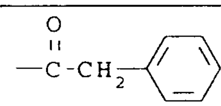 |
| $OCH_3$ | $OCH_3$ | $CH_2CH_2CH_3$ | 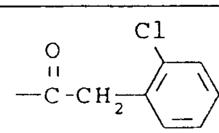 |

Table 1 (continued)

| R1 | R2 | R3 | R4 |
|---|---|---|---|
| $OCH_3$ | $OCH_3$ | $CH(CH_3)_2$ | 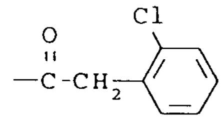 |
| $OCH_3$ | $OCH_3$ | $C(CH_3)_3$ | 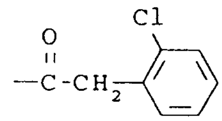 |
| $OCH_3$ | $OCH_3$ | $CH_2CH_2CH_3$ | 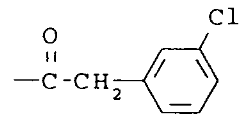 |
| $OCH_3$ | $OCH_3$ | $CH(CH_3)_2$ | 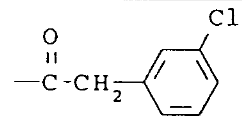 |
| $OCH_3$ | $OCH_3$ | $C(CH_3)_3$ | 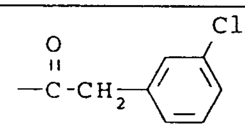 |
| $OCH_3$ | $OCH_3$ | $CH_2CH_2CH_3$ | 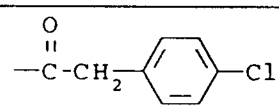 |
| $OCH_3$ | $OCH_3$ | $CH(CH_3)_2$ | 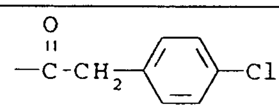 |
| $OCH_3$ | $OCH_3$ | $C(CH_3)_3$ | 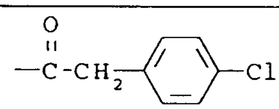 |
| $OCH_3$ | $OCH_3$ | $CH_2CH_2CH_3$ | 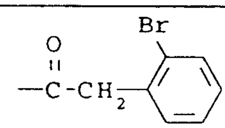 |
| $OCH_3$ | $OCH_3$ | $CH(CH_3)_2$ | 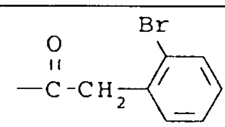 |
| $OCH_3$ | $OCH_3$ | $C(CH_3)_3$ | 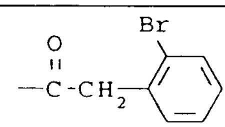 |

Table 1 (continued)

| R1 | R2 | R3 | R4 |
|---|---|---|---|
| $OCH_3$ | $OCH_3$ | $CH_2CH_2CH_3$ | 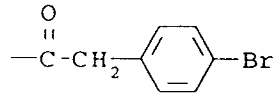 |
| $OCH_3$ | $OCH_3$ | $CH(CH_3)_2$ | 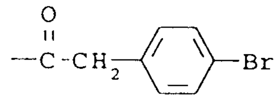 |
| $OCH_3$ | $OCH_3$ | $C(CH_3)_3$ | 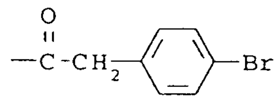 |
| $OCH_3$ | $OCH_3$ | $CH_2CH_2CH_3$ | 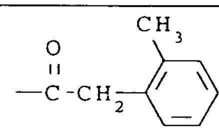 |
| $OCH_3$ | $OCH_3$ | $CH(CH_3)_2$ | 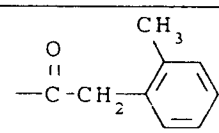 |
| $OCH_3$ | $OCH_3$ | $C(CH_3)_3$ | 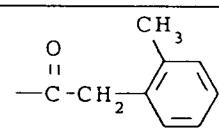 |
| $OCH_3$ | $OCH_3$ | $CH_2CH_2CH_3$ | 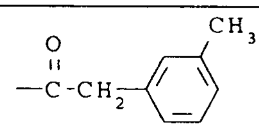 |
| $OCH_3$ | $OCH_3$ | $CH(CH_3)_2$ | 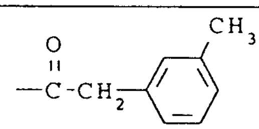 |
| $OCH_3$ | $OCH_3$ | $C(CH_3)_3$ | 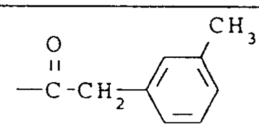 |
| $OCH_3$ | $OCH_3$ | $CH_2CH_2CH_3$ | 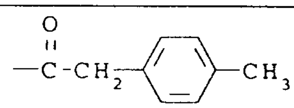 |
| $OCH_3$ | $OCH_3$ | $CH(CH_3)_2$ | 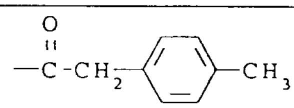 |

Table 1 (continued)

| R1 | R2 | R3 | R4 |
|---|---|---|---|
| $OCH_3$ | $OCH_3$ | $C(CH_3)_3$ | 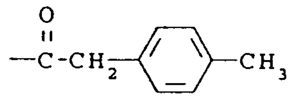 |
| $OCH_3$ | $OCH_3$ | $CH_2CH_2CH_3$ | 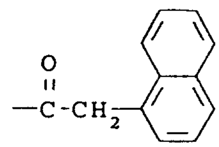 |
| $OCH_3$ | $OCH_3$ | $CH(CH_3)_2$ | 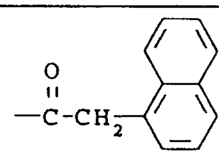 |
| $OCH_3$ | $OCH_3$ | $C(CH_3)_3$ | 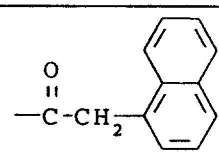 |
| $OCH_3$ | $OCH_3$ | $CH_2CH_2CH_3$ | 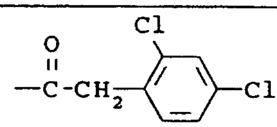 |
| $OCH_3$ | $OCH_3$ | $CH(CH_3)_2$ | 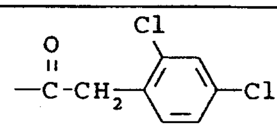 |
| $OCH_3$ | $OCH_3$ | $C(CH_3)_3$ | 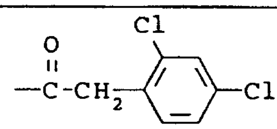 |
| $OCH_3$ | $OCH_3$ | $CH_2CH_2CH_3$ | 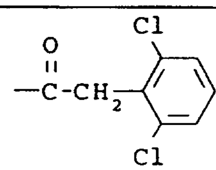 |
| $OCH_3$ | $OCH_3$ | $CH(CH_3)_2$ | 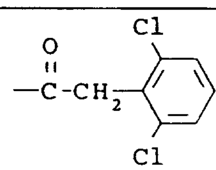 |

Table 1 (continued)

| R1 | R2 | R3 | R4 |
|---|---|---|---|
| $OCH_3$ | $OCH_3$ | $C(CH_3)_3$ | 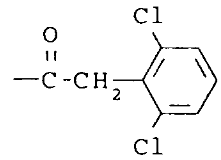 |
| $OCH_3$ | $OCH_3$ | $CH_2CH_2CH_3$ | 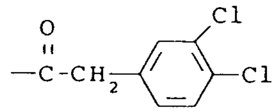 |
| $OCH_3$ | $OCH_3$ | $CH(CH_3)_2$ | 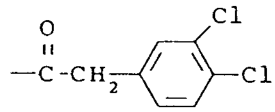 |
| $OCH_3$ | $OCH_3$ | $C(CH_3)_3$ | 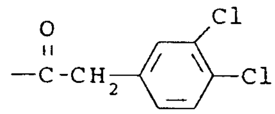 |
| $OCH_3$ | $OCH_3$ | $CH_2CH_2CH_3$ | 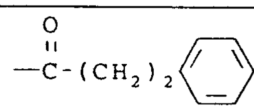 |
| $OCH_3$ | $OCH_3$ | $CH(CH_3)_2$ | 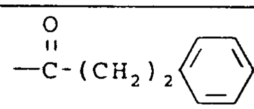 |
| $OCH_3$ | $OCH_3$ | $C(CH_3)_3$ | 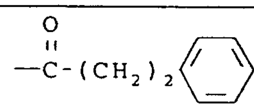 |
| $OCH_3$ | $OCH_3$ | $CH_2CH_2CH_3$ | 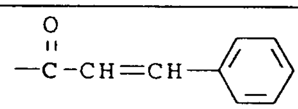 |
| $OCH_3$ | $OCH_3$ | $CH(CH_3)_2$ | 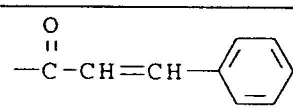 |
| $OCH_3$ | $OCH_3$ | $C(CH_3)_3$ | 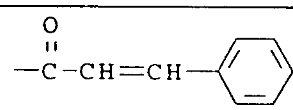 |
| $OCH_3$ | $OCH_3$ | $CH_2CH_2CH_3$ | 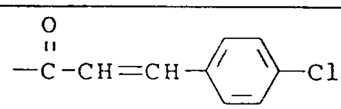 |

Table 1 (continued)

| R1 | R2 | R3 | R4 |
|---|---|---|---|
| $OCH_3$ | $OCH_3$ | $CH(CH_3)_2$ | 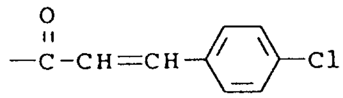 |
| $OCH_3$ | $OCH_3$ | $C(CH_3)_3$ | 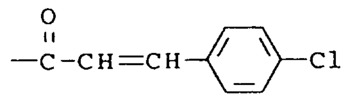 |
| $OCH_3$ | $OCH_3$ | $CH_2CH_2CH_3$ | 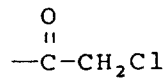 |
| $OCH_3$ | $OCH_3$ | $CH(CH_3)_2$ | 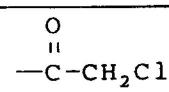 |
| $OCH_3$ | $OCH_3$ | $C(CH_3)_3$ | 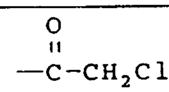 |
| $OCH_3$ | $OCH_3$ | $CH_2CH_2CH_3$ | 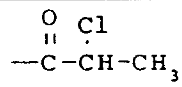 |
| $OCH_3$ | $OCH_3$ | $CH(CH_3)_2$ | 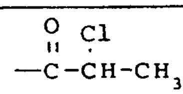 |
| $OCH_3$ | $OCH_3$ | $C(CH_3)_3$ | 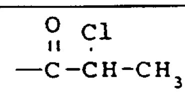 |
| $OCH_3$ | $OCH_3$ | $CH_2CH_2CH_3$ | 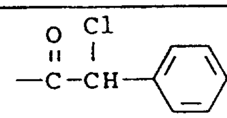 |
| $OCH_3$ | $OCH_3$ | $CH(CH_3)_2$ | 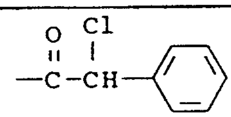 |
| $OCH_3$ | $OCH_3$ | $C(CH_3)_3$ | 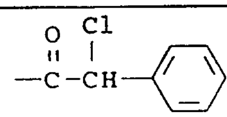 |
| $OCH_3$ | $OCH_3$ | $CH_2CH_2CH_3$ | 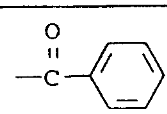 |
| $OCH_3$ | $OCH_3$ | $CH(CH_3)_2$ | 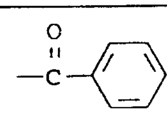 |

Table 1 (continued)

| R1 | R2 | R3 | R4 |
|---|---|---|---|
| $OCH_3$ | $OCH_3$ | $C(CH_3)_3$ | 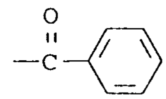 |
| $OCH_3$ | $OCH_3$ | $CH_2CH_2CH_3$ | 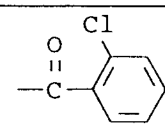 |
| $OCH_3$ | $OCH_3$ | $CH(CH_3)_2$ | 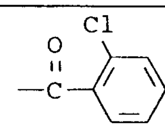 |
| $OCH_3$ | $OCH_3$ | $C(CH_3)_3$ | 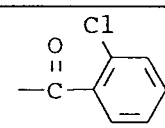 |
| $OCH_3$ | $OCH_3$ | $CH_2CH_2CH_3$ | 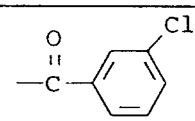 |
| $OCH_3$ | $OCH_3$ | $CH(CH_3)_2$ | 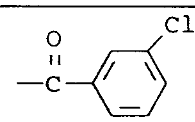 |
| $OCH_3$ | $OCH_3$ | $C(CH_3)_3$ | 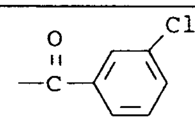 |
| $OCH_3$ | $OCH_3$ | $CH_2CH_2CH_3$ | 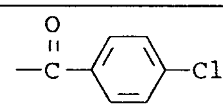 |
| $OCH_3$ | $OCH_3$ | $CH(CH_3)_2$ | 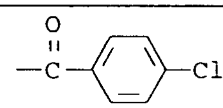 |
| $OCH_3$ | $OCH_3$ | $C(CH_3)_3$ | 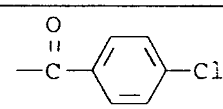 |
| $OCH_3$ | $OCH_3$ | $CH_2CH_2CH_3$ | 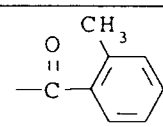 |

Table 1 (continued)

| R1 | R2 | R3 | R4 |
|---|---|---|---|
| $OCH_3$ | $OCH_3$ | $CH(CH_3)_2$ | 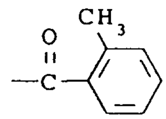 |
| $OCH_3$ | $OCH_3$ | $C(CH_3)_3$ | 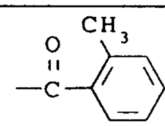 |
| $OCH_3$ | $OCH_3$ | $CH_2CH_2CH_3$ | 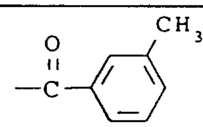 |
| $OCH_3$ | $OCH_3$ | $CH(CH_3)_2$ | 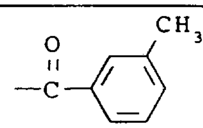 |
| $OCH_3$ | $OCH_3$ | $C(CH_3)_3$ | 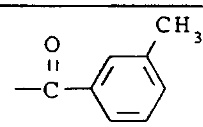 |
| $OCH_3$ | $OCH_3$ | $CH_2CH_2CH_3$ | 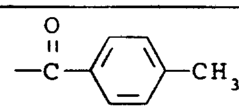 |
| $OCH_3$ | $OCH_3$ | $CH(CH_3)_2$ | 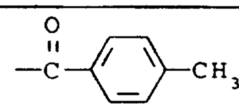 |
| $OCH_3$ | $OCH_3$ | $C(CH_3)_3$ | 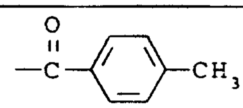 |
| $OCH_3$ | $OCH_3$ | $CH_2CH_2CH_3$ | 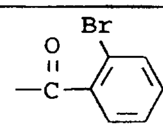 |
| $OCH_3$ | $OCH_3$ | $CH(CH_3)_2$ | 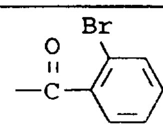 |

Table 1 (continued)

| R1 | R2 | R3 | R4 |
|---|---|---|---|
| $OCH_3$ | $OCH_3$ | $C(CH_3)_3$ | 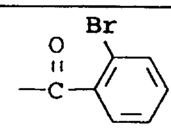 |
| $OCH_3$ | $OCH_3$ | $CH_2CH_2CH_3$ | 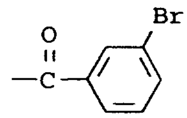 |
| $OCH_3$ | $OCH_3$ | $CH(CH_3)_2$ | 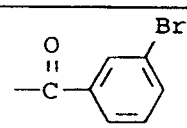 |
| $OCH_3$ | $OCH_3$ | $C(CH_3)_3$ | 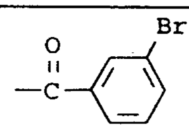 |
| $OCH_3$ | $OCH_3$ | $CH_2CH_2CH_3$ | 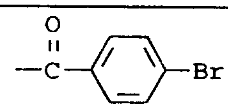 |
| $OCH_3$ | $OCH_3$ | $CH(CH_3)_2$ | 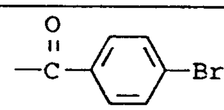 |
| $OCH_3$ | $OCH_3$ | $C(CH_3)_3$ | 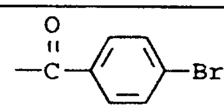 |
| $OCH_3$ | $OCH_3$ | $CH_2CH_2CH_3$ | 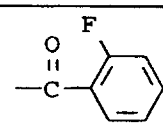 |
| $OCH_3$ | $OCH_3$ | $CH(CH_3)_2$ | 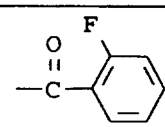 |
| $OCH_3$ | $OCH_3$ | $C(CH_3)_3$ | 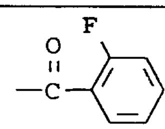 |

Table 1 (continued)

| R1 | R2 | R3 | R4 |
|---|---|---|---|
| $OCH_3$ | $OCH_3$ | $CH_2CH_2CH_3$ | 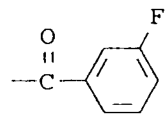 |
| $OCH_3$ | $OCH_3$ | $CH(CH_3)_2$ | 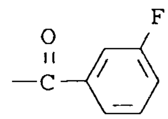 |
| $OCH_3$ | $OCH_3$ | $C(CH_3)_3$ | 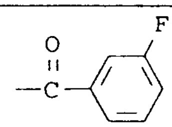 |
| $OCH_3$ | $OCH_3$ | $CH_2CH_2CH_3$ | 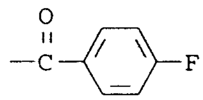 |
| $OCH_3$ | $OCH_3$ | $CH(CH_3)_2$ | 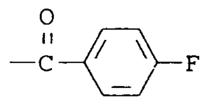 |
| $OCH_3$ | $OCH_3$ | $C(CH_3)_3$ | 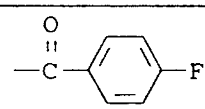 |
| $OCH_3$ | $OCH_3$ | $CH_2CH_2CH_3$ | 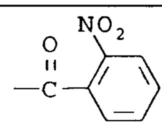 |
| $OCH_3$ | $OCH_3$ | $CH(CH_3)_2$ | 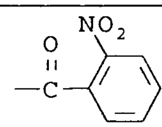 |
| $OCH_3$ | $OCH_3$ | $C(CH_3)_3$ | 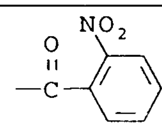 |
| $OCH_3$ | $OCH_3$ | $CH_2CH_2CH_3$ | 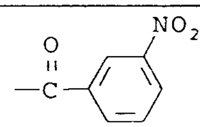 |

Table 1 (continued)

| R1 | R2 | R3 | R4 |
|---|---|---|---|
| $OCH_3$ | $OCH_3$ | $CH(CH_3)_2$ | 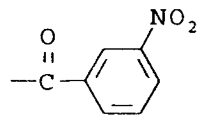 |
| $OCH_3$ | $OCH_3$ | $C(CH_3)_3$ | 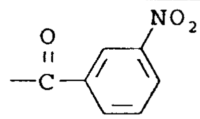 |
| $OCH_3$ | $OCH_3$ | $CH_2CH_2CH_3$ | 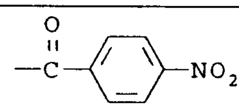 |
| $OCH_3$ | $OCH_3$ | $CH(CH_3)_2$ | 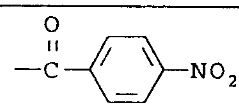 |
| $OCH_3$ | $OCH_3$ | $C(CH_3)_3$ | 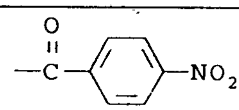 |
| $OCH_3$ | $OCH_3$ | $CH_2CH_2CH_3$ | 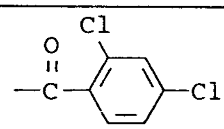 |
| $OCH_3$ | $OCH_3$ | $CH(CH_3)_2$ | 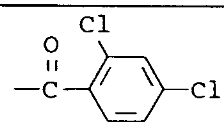 |
| $OCH_3$ | $OCH_3$ | $C(CH_3)_3$ | 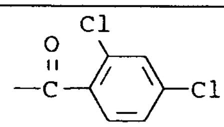 |
| $OCH_3$ | $OCH_3$ | $CH_2CH_2CH_3$ | 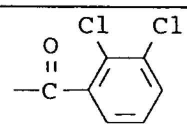 |
| $OCH_3$ | $OCH_3$ | $CH(CH_3)_2$ | 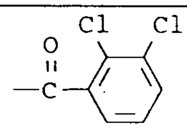 |

Table 1 (continued)

| R1 | R2 | R3 | R4 |
|---|---|---|---|
| $OCH_3$ | $OCH_3$ | $C(CH_3)_3$ | 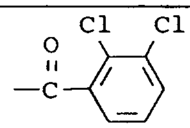 |
| $OCH_3$ | $OCH_3$ | $CH_2CH_2CH_3$ | 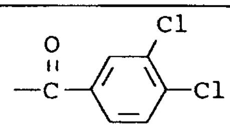 |
| $OCH_3$ | $OCH_3$ | $CH(CH_3)_2$ | 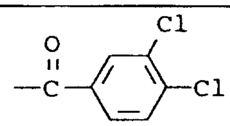 |
| $OCH_3$ | $OCH_3$ | $C(CH_3)_3$ | 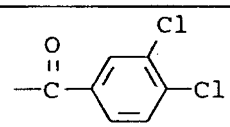 |
| $OCH_3$ | $OCH_3$ | $CH_2CH_2CH_3$ | 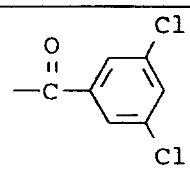 |
| $OCH_3$ | $OCH_3$ | $CH(CH_3)_2$ | 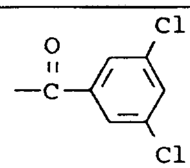 |
| $OCH_3$ | $OCH_3$ | $C(CH_3)_3$ | 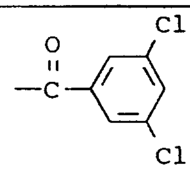 |
| $OCH_3$ | $OCH_3$ | $CH_2CH_2CH_3$ | 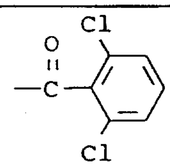 |

Table 1 (continued)

| R1 | R2 | R3 | R4 |
|---|---|---|---|
| $OCH_3$ | $OCH_3$ | $CH(CH_3)_2$ | 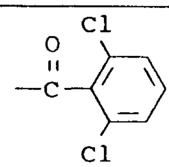 |
| $OCH_3$ | $OCH_3$ | $C(CH_3)_3$ | 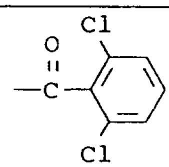 |
| $OCH_3$ | $OCH_3$ | $CH_2CH_2CH_3$ | 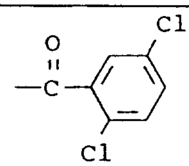 |
| $OCH_3$ | $OCH_3$ | $CH(CH_3)_2$ | 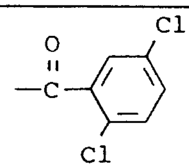 |
| $OCH_3$ | $OCH_3$ | $C(CH_3)_3$ | 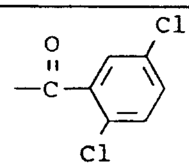 |
| $OCH_3$ | $OCH_3$ | $CH_2CH_2CH_3$ | 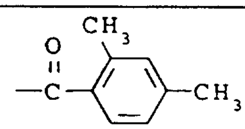 |
| $OCH_3$ | $OCH_3$ | $CH(CH_3)_2$ | 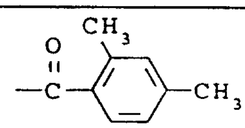 |
| $OCH_3$ | $OCH_3$ | $C(CH_3)_3$ | 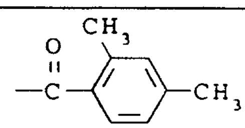 |

Table 1 (continued)

| R1 | R2 | R3 | R4 |
|---|---|---|---|
| $OCH_3$ | $OCH_3$ | $CH_2CH_2CH_3$ | 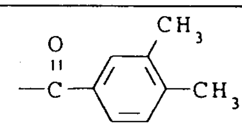 |
| $OCH_3$ | $OCH_3$ | $CH(CH_3)_2$ | 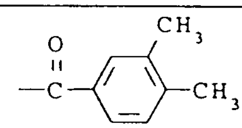 |
| $OCH_3$ | $OCH_3$ | $C(CH_3)_3$ | 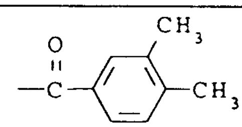 |
| $OCH_3$ | $OCH_3$ | $CH_2CH_2CH_3$ | 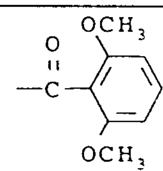 |
| $OCH_3$ | $OCH_3$ | $CH(CH_3)_2$ | 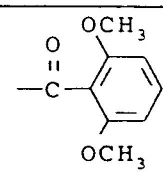 |
| $OCH_3$ | $OCH_3$ | $C(CH_3)_3$ | 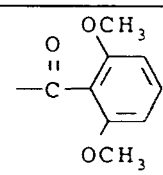 |
| $OCH_3$ | $OCH_3$ | $CH_2CH_2CH_3$ | 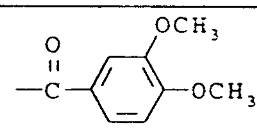 |
| $OCH_3$ | $OCH_3$ | $CH(CH_3)_2$ | 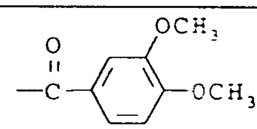 |
| $OCH_3$ | $OCH_3$ | $C(CH_3)_3$ | 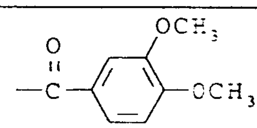 |

Table 1 (continued)

| R1 | R2 | R3 | R4 |
|---|---|---|---|
| $OCH_3$ | $OCH_3$ | $CH_2CH_2CH_3$ | 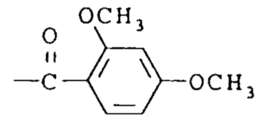 |
| $OCH_3$ | $OCH_3$ | $CH(CH_3)_2$ | 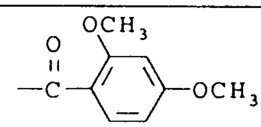 |
| $OCH_3$ | $OCH_3$ | $C(CH_3)_3$ | 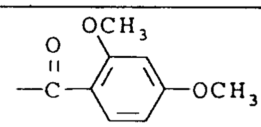 |
| $OCH_3$ | $OCH_3$ | $CH_2CH_2CH_3$ | 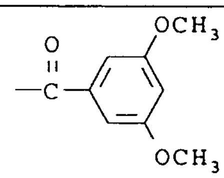 |
| $OCH_3$ | $OCH_3$ | $CH(CH_3)_2$ | 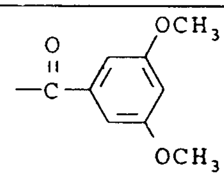 |
| $OCH_3$ | $OCH_3$ | $C(CH_3)_3$ | 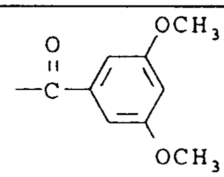 |
| $OCH_3$ | $OCH_3$ | $CH_2CH_2CH_3$ | 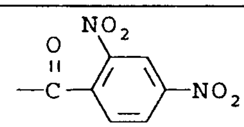 |
| $OCH_3$ | $OCH_3$ | $CH(CH_3)_2$ | 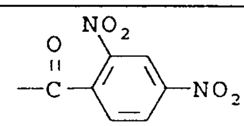 |

Table 1 (continued)

| R1 | R2 | R3 | R4 |
|---|---|---|---|
| $OCH_3$ | $OCH_3$ | $C(CH_3)_3$ | 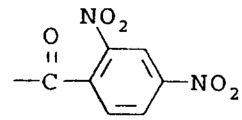 |
| $OCH_3$ | $OCH_3$ | $CH_2CH_2CH_3$ | 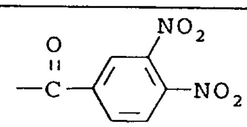 |
| $OCH_3$ | $OCH_3$ | $CH(CH_3)_2$ | 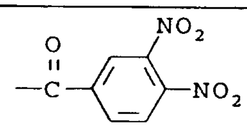 |
| $OCH_3$ | $OCH_3$ | $C(CH_3)_3$ | 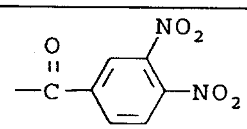 |
| $OCH_3$ | $OCH_3$ | $CH_2CH_2CH_3$ | 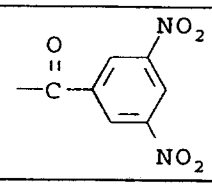 |
| $OCH_3$ | $OCH_3$ | $CH(CH_3)_2$ | 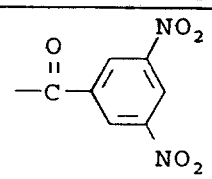 |
| $OCH_3$ | $OCH_3$ | $C(CH_3)_3$ | 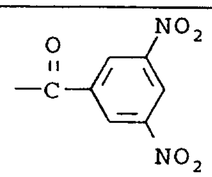 |
| $OCH_3$ | $OCH_3$ | $CH_2CH_2CH_3$ | 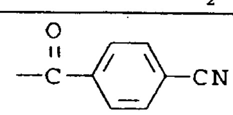 |
| $OCH_3$ | $OCH_3$ | $CH(CH_3)_2$ | 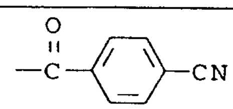 |

Table 1 (continued)

| R1 | R2 | R3 | R4 |
|---|---|---|---|
| $OCH_3$ | $OCH_3$ | $C(CH_3)_3$ | 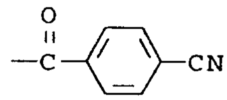 |
| $OCH_3$ | $OCH_3$ | $CH_2CH_2CH_3$ | 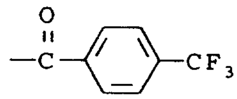 |
| $OCH_3$ | $OCH_3$ | $CH(CH_3)_2$ | 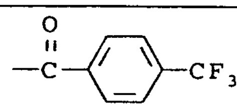 |
| $OCH_3$ | $OCH_3$ | $C(CH_3)_3$ | 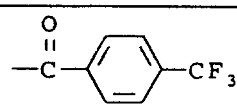 |
| $OCH_3$ | $OCH_3$ | $CH_2CH_2CH_3$ | 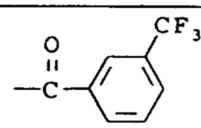 |
| $OCH_3$ | $OCH_3$ | $CH(CH_3)_2$ | 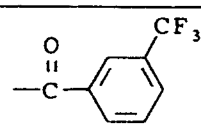 |
| $OCH_3$ | $OCH_3$ | $C(CH_3)_3$ | 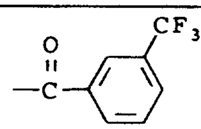 |
| $OCH_3$ | $OCH_3$ | $CH_2CH_2CH_3$ | 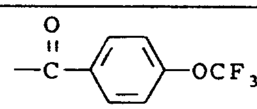 |
| $OCH_3$ | $OCH_3$ | $CH(CH_3)_2$ | 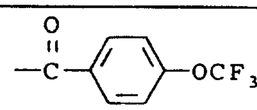 |
| $OCH_3$ | $OCH_3$ | $C(CH_3)_3$ | 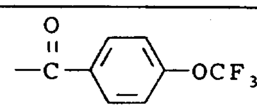 |
| $OCH_3$ | $OCH_3$ | $CH_2CH_2CH_3$ | 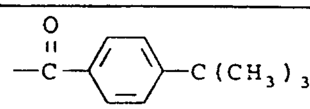 |

Table 1 (continued)

| R1 | R2 | R3 | R4 |
|---|---|---|---|
| $OCH_3$ | $OCH_3$ | $CH(CH_3)_2$ | 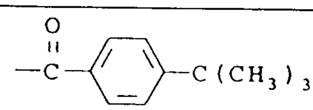 |
| $OCH_3$ | $OCH_3$ | $C(CH_3)_3$ | 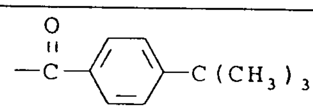 |
| $OCH_3$ | $OCH_3$ | $CH_2CH_2CH_3$ | 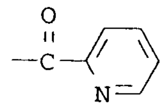 |
| $OCH_3$ | $OCH_3$ | $CH(CH_3)_2$ | 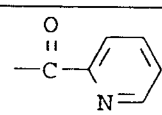 |
| $OCH_3$ | $OCH_3$ | $C(CH_3)_3$ | 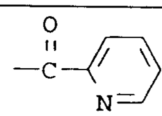 |
| $OCH_3$ | $OCH_3$ | $CH_2CH_2CH_3$ | 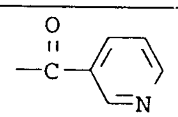 |
| $OCH_3$ | $OCH_3$ | $CH(CH_3)_2$ | 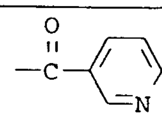 |
| $OCH_3$ | $OCH_3$ | $C(CH_3)_3$ | 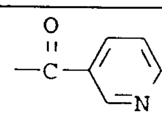 |
| $OCH_3$ | $OCH_3$ | $CH_2CH_2CH_3$ | 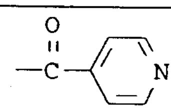 |
| $OCH_3$ | $OCH_3$ | $CH(CH_3)_2$ | 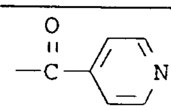 |
| $OCH_3$ | $OCH_3$ | $C(CH_3)_3$ | 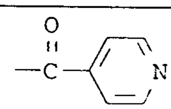 |

Table 1 (continued)

| R1 | R2 | R3 | R4 |
|---|---|---|---|
| $OCH_3$ | $OCH_3$ | $CH_2CH_2CH_3$ | 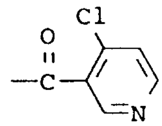 |
| $OCH_3$ | $OCH_3$ | $CH(CH_3)_2$ | 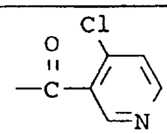 |
| $OCH_3$ | $OCH_3$ | $C(CH_3)_3$ | 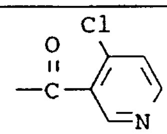 |
| $OCH_3$ | $OCH_3$ | $CH_2CH_2CH_3$ | 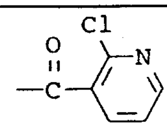 |
| $OCH_3$ | $OCH_3$ | $CH(CH_3)_2$ | 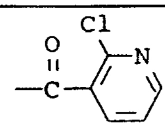 |
| $OCH_3$ | $OCH_3$ | $C(CH_3)_3$ | 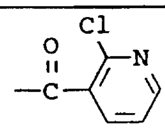 |
| $OCH_3$ | $OCH_3$ | $CH_2CH_2CH_3$ | 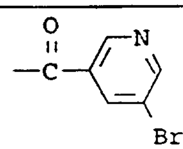 |
| $OCH_3$ | $OCH_3$ | $CH(CH_3)_2$ | 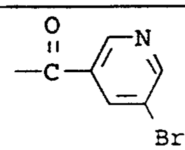 |
| $OCH_3$ | $OCH_3$ | $C(CH_3)_3$ | 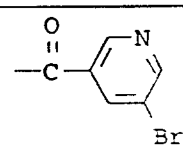 |

Table 1 (continued)

| R1 | R2 | R3 | R4 |
|---|---|---|---|
| $OCH_3$ | $OCH_3$ | $CH_2CH_2CH_3$ | 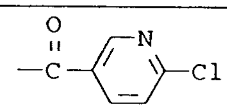 |
| $OCH_3$ | $OCH_3$ | $CH(CH_3)_2$ | 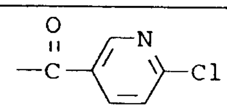 |
| $OCH_3$ | $OCH_3$ | $C(CH_3)_3$ | 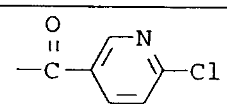 |
| $OCH_3$ | $OCH_3$ | $CH_2CH_2CH_3$ | 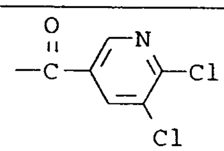 |
| $OCH_3$ | $OCH_3$ | $CH(CH_3)_2$ | 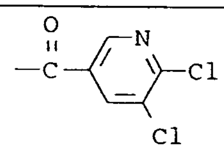 |
| $OCH_3$ | $OCH_3$ | $C(CH_3)_3$ | 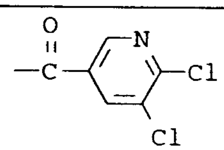 |
| $OCH_3$ | $OCH_3$ | $CH_2CH_2CH_3$ | 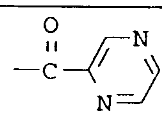 |
| $OCH_3$ | $OCH_3$ | $CH(CH_3)_2$ | 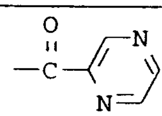 |
| $OCH_3$ | $OCH_3$ | $C(CH_3)_3$ | 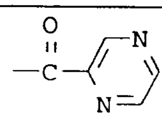 |
| $OCH_3$ | $OCH_3$ | $CH_2CH_2CH_3$ | 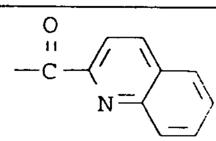 |

Table 1 (continued)

| R1 | R2 | R3 | R4 |
|---|---|---|---|
| $OCH_3$ | $OCH_3$ | $CH(CH_3)_2$ | 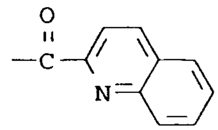 |
| $OCH_3$ | $OCH_3$ | $C(CH_3)_3$ | 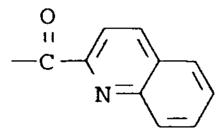 |
| $OCH_3$ | $OCH_3$ | $CH_2CH_2CH_3$ | 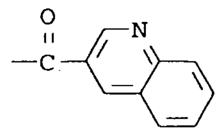 |
| $OCH_3$ | $OCH_3$ | $CH(CH_3)_2$ | 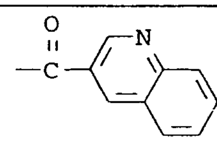 |
| $OCH_3$ | $OCH_3$ | $C(CH_3)_3$ | 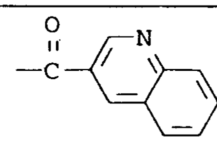 |
| $OCH_3$ | $OCH_3$ | $CH_2CH_2CH_3$ | 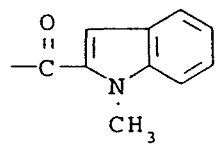 |
| $OCH_3$ | $OCH_3$ | $CH(CH_3)_2$ | 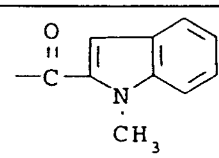 |
| $OCH_3$ | $OCH_3$ | $C(CH_3)_3$ | 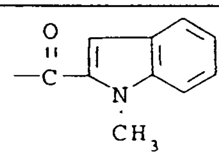 |
| $OCH_3$ | $OCH_3$ | $CH_2CH_2CH_3$ | 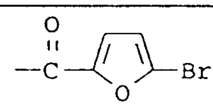 |
| $OCH_3$ | $OCH_3$ | $CH(CH_3)_2$ | 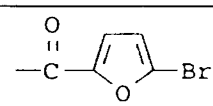 |

Table 1 (continued)

| R1 | R2 | R3 | R4 |
|---|---|---|---|
| $OCH_3$ | $OCH_3$ | $C(CH_3)_3$ | 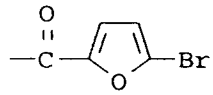 |
| $OCH_3$ | $OCH_3$ | $CH_2CH_2CH_3$ | 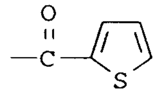 |
| $OCH_3$ | $OCH_3$ | $CH(CH_3)_2$ | 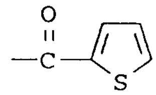 |
| $OCH_3$ | $OCH_3$ | $C(CH_3)_3$ | 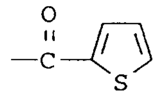 |
| $OCH_3$ | $OCH_3$ | $CH_2CH_2CH_3$ | 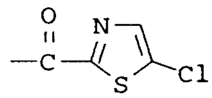 |
| $OCH_3$ | $OCH_3$ | $CH(CH_3)_2$ | 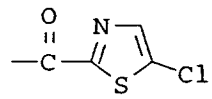 |
| $OCH_3$ | $OCH_3$ | $C(CH_3)_3$ | 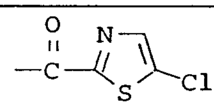 |
| $OCH_3$ | $OCH_3$ | $CH_2CH_2CH_3$ | 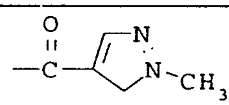 |
| $OCH_3$ | $OCH_3$ | $CH(CH_3)_2$ | 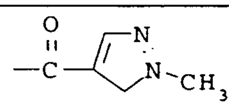 |
| $OCH_3$ | $OCH_3$ | $C(CH_3)_3$ | 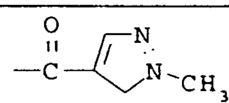 |

Table 1 (continued)

| R1 | R2 | R3 | R4 |
|---|---|---|---|
| $OCHF_2$ | $OCHF_2$ | $CH(CH_3)_2$ | H |
| $OCF_3$ | $OCF_3$ | $CH(CH_3)_2$ | H |
| $OCHF_2$ | $OCHF_2$ | $C(CH_3)_3$ | H |
| $OCF_3$ | $OCF_3$ | $C(CH_3)_3$ | H |
| $OCHF_2$ | $OCHF_2$ | $CH(CH_3)_2$ | 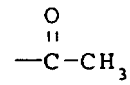 |
| $OCF_3$ | $OCF_3$ | $C(CH_3)_3$ | 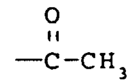 |
| $OCHF_2$ | $OCHF_2$ | $C(CH_3)_3$ | 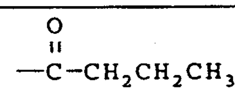 |
| $OCF_3$ | $OCF_3$ | $CH_2CH_2CH_3$ | 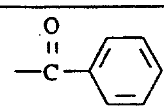 |
| $OCHF_2$ | $OCHF_2$ | $CH(CH_3)_2$ | 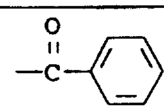 |
| $OCF_3$ | $OCF_3$ | $C(CH_3)_3$ | 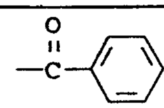 |
| $OCF_3$ | $OCF_3$ | $CH(CH_3)_2$ | 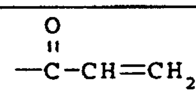 |
| $OCHF_2$ | $OCHF_2$ | $C(CH_3)_3$ | 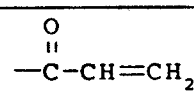 |
| $OCF_3$ | $OCF_3$ | $C(CH_3)_3$ | 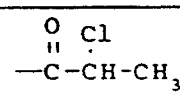 |
| $OCHF_2$ | $OCHF_2$ | $C(CH_3)_3$ | 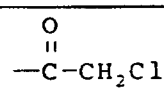 |

Table 1 (continued)

| R1 | R2 | R3 | R4 |
|---|---|---|---|
| $OCH_3$ | $OCH_3$ | $CH_2CH(CH_3)_2$ | $-\overset{O}{\overset{\|}{C}}-CH_2-O-C_2H_5$ |
| $OCH_3$ | $OCH_3$ | $C(CH_3)_3$ | $-\overset{O}{\overset{\|}{C}}-CH_2-O-C_2H_5$ |
| $OCH_3$ | $OCH_3$ | $C_2H_5$ | $-\overset{O}{\overset{\|}{C}}-CH_2-O-(CH_2)_3CH_3$ |
| $OCH_3$ | $OCH_3$ | $CH_2CH_2CH_3$ | $-\overset{O}{\overset{\|}{C}}-CH_2-O-(CH_2)_3CH_3$ |
| $OCH_3$ | $OCH_3$ | $CH(CH_3)_2$ | $-\overset{O}{\overset{\|}{C}}-CH_2-O-(CH_2)_3CH_3$ |
| $OCH_3$ | $OCH_3$ | $CH_2CH_2CH_2CH_3$ | $-\overset{O}{\overset{\|}{C}}-CH_2-O-(CH_2)_3CH_3$ |
| $OCH_3$ | $OCH_3$ | $CH(CH_3)C_2H_5$ | $-\overset{O}{\overset{\|}{C}}-CH_2-O-(CH_2)_3CH_3$ |
| $OCH_3$ | $OCH_3$ | $CH_2CH(CH_3)_2$ | $-\overset{O}{\overset{\|}{C}}-CH_2-O-(CH_2)_3CH_3$ |
| $OCH_3$ | $OCH_3$ | $C(CH_3)_3$ | $-\overset{O}{\overset{\|}{C}}-CH_2-O-(CH_2)_3CH_3$ |
| $OCH_3$ | $OCH_3$ | $C_2H_5$ | $-\overset{O}{\overset{\|}{C}}-CH_2CH_2OCH_3$ |
| $OCH_3$ | $OCH_3$ | $CH_2CH_2CH_3$ | $-\overset{O}{\overset{\|}{C}}-CH_2CH_2OCH_3$ |
| $OCH_3$ | $OCH_3$ | $CH(CH_3)_2$ | $-\overset{O}{\overset{\|}{C}}-CH_2CH_2OCH_3$ |
| $OCH_3$ | $OCH_3$ | $CH_2CH_2CH_2CH_3$ | $-\overset{O}{\overset{\|}{C}}-CH_2CH_2OCH_3$ |
| $OCH_3$ | $OCH_3$ | $CH(CH_3)C_2H_5$ | $-\overset{O}{\overset{\|}{C}}-CH_2CH_2OCH_3$ |
| $OCH_3$ | $OCH_3$ | $CH_2CH(CH_3)_2$ | $-\overset{O}{\overset{\|}{C}}-CH_2CH_2OCH_3$ |

Table 1 (continued)

| R1 | R2 | R3 | R4 |
|---|---|---|---|
| $OCH_3$ | $OCH_3$ | $C(CH_3)_3$ | 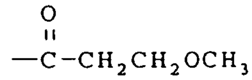 |
| $OCH_3$ | $OCH_3$ | $C_2H_5$ | 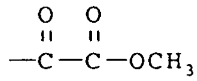 |
| $OCH_3$ | $OCH_3$ | $CH_2CH_2CH_3$ | 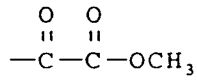 |
| $OCH_3$ | $OCH_3$ | $CH(CH_3)_2$ | 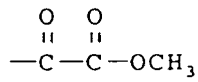 |
| $OCH_3$ | $OCH_3$ | $CH_2CH_2CH_2CH_3$ | 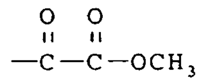 |
| $OCH_3$ | $OCH_3$ | $CH(CH_3)C_2H_5$ | 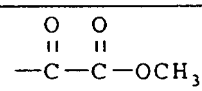 |
| $OCH_3$ | $OCH_3$ | $CH_2CH(CH_3)_2$ | 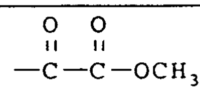 |
| $OCH_3$ | $OCH_3$ | $C(CH_3)_3$ | 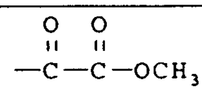 |
| $OCH_3$ | $OCH_3$ | $C_2H_5$ | 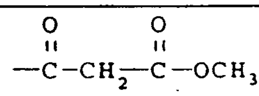 |
| $OCH_3$ | $OCH_3$ | $CH_2CH_2CH_3$ | 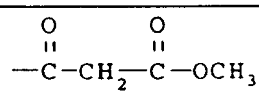 |
| $OCH_3$ | $OCH_3$ | $CH(CH_3)_2$ | 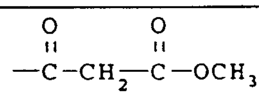 |
| $OCH_3$ | $OCH_3$ | $CH_2CH_2CH_2CH_3$ | 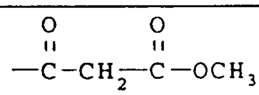 |
| $OCH_3$ | $OCH_3$ | $CH(CH_3)C_2H_5$ | 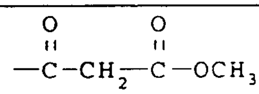 |
| $OCH_3$ | $OCH_3$ | $CH_2CH(CH_3)_2$ | 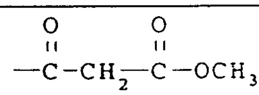 |

Table 1 (continued)

| R1 | R2 | R3 | R4 |
|---|---|---|---|
| $OCH_3$ | $OCH_3$ | $C(CH_3)_3$ | 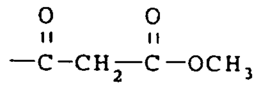 |
| $OCH_3$ | $OCH_3$ | $C_2H_5$ | 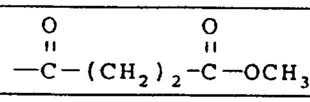 |
| $OCH_3$ | $OCH_3$ | $CH_2CH_2CH_3$ | 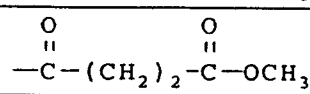 |
| $OCH_3$ | $OCH_3$ | $CH(CH_3)_2$ | 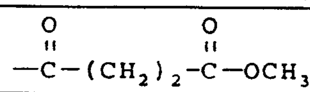 |
| $OCH_3$ | $OCH_3$ | $CH_2CH_2CH_2CH_3$ | 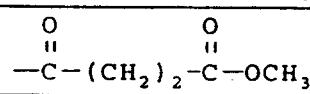 |
| $OCH_3$ | $OCH_3$ | $CH(CH_3)C_2H_5$ | 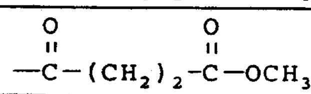 |
| $OCH_3$ | $OCH_3$ | $CH_2CH(CH_3)_2$ | 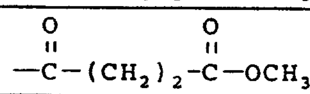 |
| $OCH_3$ | $OCH_3$ | $C(CH_3)_3$ | 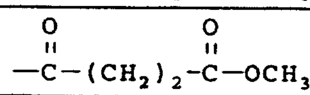 |
| $OCH_3$ | $OCH_3$ | $C_2H_5$ | 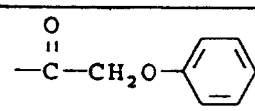 |
| $OCH_3$ | $OCH_3$ | $CH_2CH_2CH_3$ | 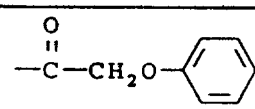 |
| $OCH_3$ | $OCH_3$ | $CH(CH_3)_2$ | 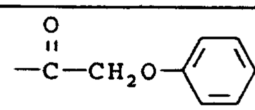 |
| $OCH_3$ | $OCH_3$ | $CH_2CH_2CH_2CH_3$ | 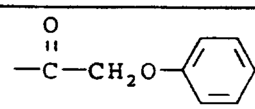 |
| $OCH_3$ | $OCH_3$ | $CH(CH_3)C_2H_5$ | 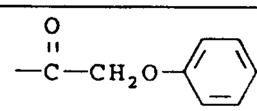 |

Table 1 (continued)

| R1 | R2 | R3 | R4 |
|---|---|---|---|
| $OCH_3$ | $OCH_3$ | $CH_2CH(CH_3)_2$ | 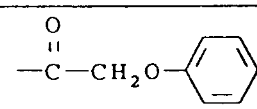 |
| $OCH_3$ | $OCH_3$ | $C(CH_3)_3$ | 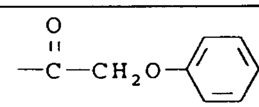 |
| $OCH_3$ | $OCH_3$ | $C_2H_5$ | 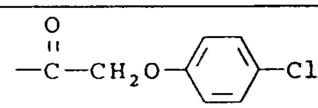 |
| $OCH_3$ | $OCH_3$ | $CH_2CH_2CH_3$ | 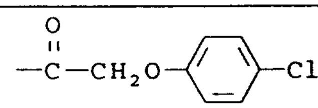 |
| $OCH_3$ | $OCH_3$ | $CH(CH_3)_2$ | 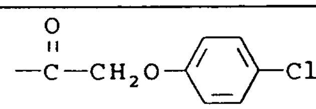 |
| $OCH_3$ | $OCH_3$ | $CH_2CH_2CH_2CH_3$ | 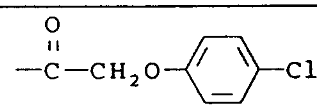 |
| $OCH_3$ | $OCH_3$ | $CH(CH_3)C_2H_5$ | 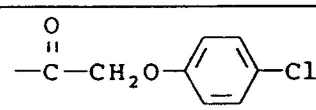 |
| $OCH_3$ | $OCH_3$ | $CH_2CH(CH_3)_2$ | 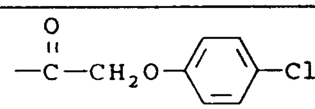 |
| $OCH_3$ | $OCH_3$ | $C(CH_3)_3$ | 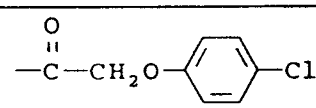 |
| $OCH_3$ | $OCH_3$ | $C_2H_5$ | 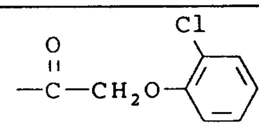 |
| $OCH_3$ | $OCH_3$ | $CH_2CH_2CH_3$ | 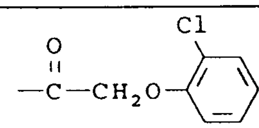 |

Table 1 (continued)

| R1 | R2 | R3 | R4 |
|---|---|---|---|
| $OCH_3$ | $OCH_3$ | $CH(CH_3)_2$ | 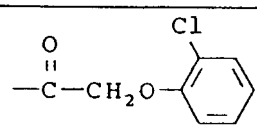 |
| $OCH_3$ | $OCH_3$ | $CH_2CH_2CH_2CH_3$ | 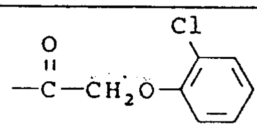 |
| $OCH_3$ | $OCH_3$ | $CH(CH_3)C_2H_5$ | 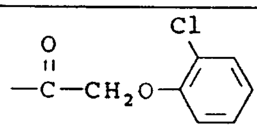 |
| $OCH_3$ | $OCH_3$ | $CH_2CH(CH_3)_2$ | 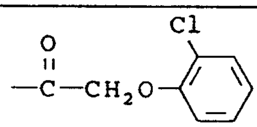 |
| $OCH_3$ | $OCH_3$ | $C(CH_3)_3$ | 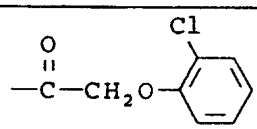 |
| $OCH_3$ | $OCH_3$ | $C_2H_5$ | 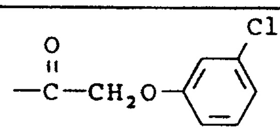 |
| $OCH_3$ | $OCH_3$ | $CH_2CH_2CH_3$ | 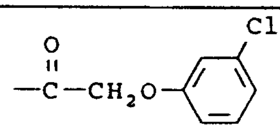 |
| $OCH_3$ | $OCH_3$ | $CH(CH_3)_2$ | 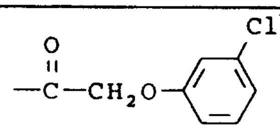 |
| $OCH_3$ | $OCH_3$ | $CH_2CH_2CH_2CH_3$ | 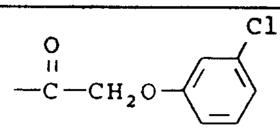 |
| $OCH_3$ | $OCH_3$ | $CH(CH_3)C_2H_5$ | 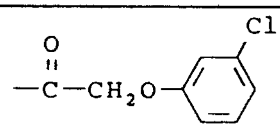 |

Table 1 (continued)

| R1 | R2 | R3 | R4 |
|---|---|---|---|
| $OCH_3$ | $OCH_3$ | $CH_2CH(CH_3)_2$ | 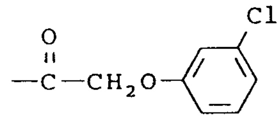 |
| $OCH_3$ | $OCH_3$ | $C(CH_3)_3$ | 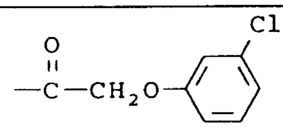 |
| $OCH_3$ | $OCH_3$ | $C_2H_5$ | 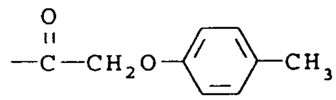 |
| $OCH_3$ | $OCH_3$ | $CH_2CH_2CH_3$ | 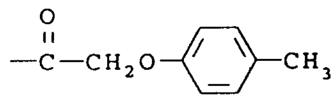 |
| $OCH_3$ | $OCH_3$ | $CH(CH_3)_2$ | 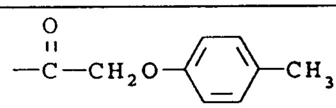 |
| $OCH_3$ | $OCH_3$ | $CH_2CH_2CH_2CH_3$ | 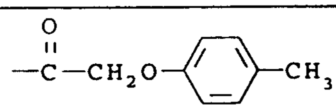 |
| $OCH_3$ | $OCH_3$ | $CH(CH_3)C_2H_5$ | 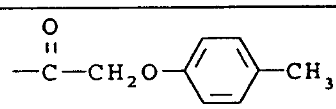 |
| $OCH_3$ | $OCH_3$ | $CH_2CH(CH_3)_2$ | 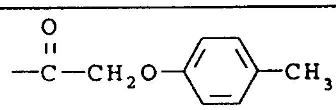 |
| $OCH_3$ | $OCH_3$ | $C(CH_3)_3$ | 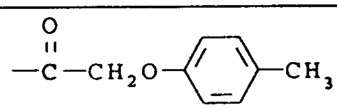 |
| $OCH_3$ | $OCH_3$ | $C_2H_5$ | 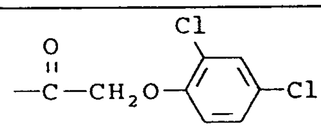 |
| $OCH_3$ | $OCH_3$ | $CH_2CH_2CH_3$ | 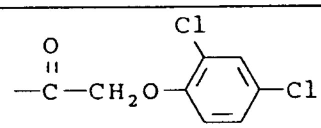 |

Table 1 (continued)

| R1 | R2 | R3 | R4 |
|---|---|---|---|
| $OCH_3$ | $OCH_3$ | $CH(CH_3)_2$ | 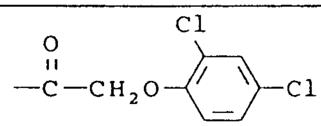 |
| $OCH_3$ | $OCH_3$ | $CH_2CH_2CH_2CH_3$ | 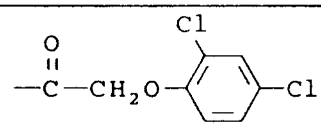 |
| $OCH_3$ | $OCH_3$ | $CH(CH_3)C_2H_5$ | 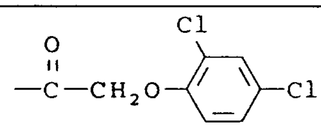 |
| $OCH_3$ | $OCH_3$ | $CH_2CH(CH_3)_2$ | 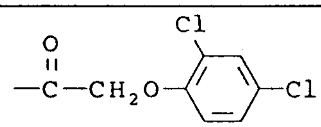 |
| $OCH_3$ | $OCH_3$ | $C(CH_3)_3$ | 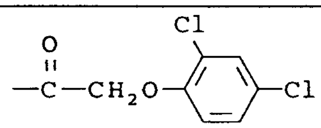 |
| $OCH_3$ | $OCH_3$ | $C_2H_5$ | 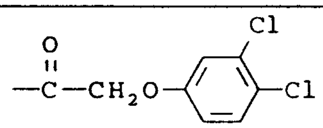 |
| $OCH_3$ | $OCH_3$ | $CH_2CH_2CH_3$ | 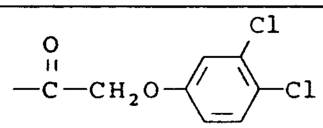 |
| $OCH_3$ | $OCH_3$ | $CH(CH_3)_2$ | 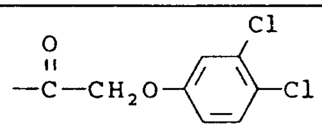 |
| $OCH_3$ | $OCH_3$ | $CH_2CH_2CH_2CH_3$ | 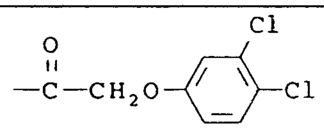 |
| $OCH_3$ | $OCH_3$ | $CH(CH_3)C_2H_5$ | 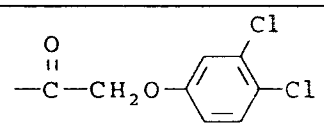 |

Table 1 (continued)

| R1 | R2 | R3 | R4 |
|---|---|---|---|
| $OCH_3$ | $OCH_3$ | $CH_2CH(CH_3)_2$ | 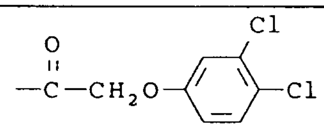 |
| $OCH_3$ | $OCH_3$ | $C(CH_3)_3$ | 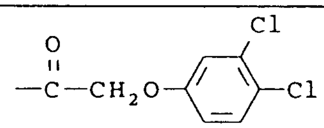 |
| $OCH_3$ | $OCH_3$ | $C_2H_5$ | 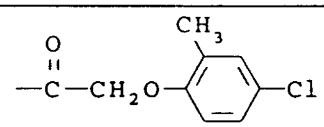 |
| $OCH_3$ | $OCH_3$ | $CH_2CH_2CH_3$ | 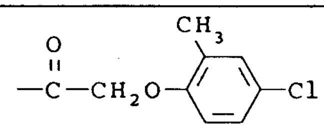 |
| $OCH_3$ | $OCH_3$ | $CH(CH_3)_2$ | 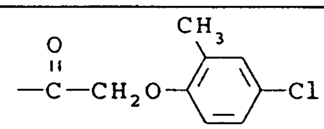 |
| $OCH_3$ | $OCH_3$ | $CH_2CH_2CH_2CH_3$ | 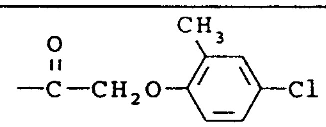 |
| $OCH_3$ | $OCH_3$ | $CH(CH_3)C_2H_5$ | 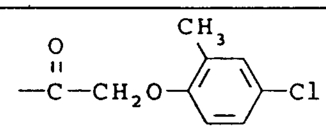 |
| $OCH_3$ | $OCH_3$ | $CH_2CH(CH_3)_2$ | 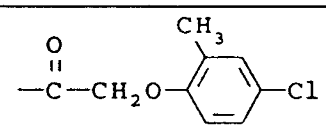 |
| $OCH_3$ | $OCH_3$ | $C(CH_3)_3$ | 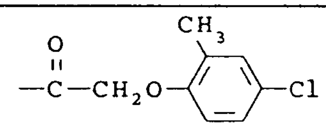 |
| $OCH_3$ | $OCH_3$ | $C_2H_5$ | 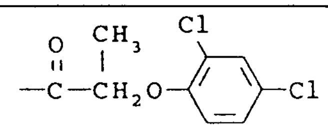 |

Table 1 (continued)

| R1 | R2 | R3 | R4 |
|---|---|---|---|
| $OCH_3$ | $OCH_3$ | $CH_2CH_2CH_3$ | 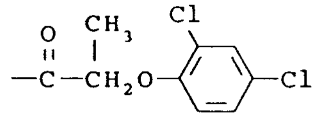 |
| $OCH_3$ | $OCH_3$ | $CH(CH_3)_2$ | 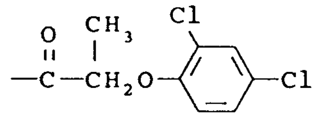 |
| $OCH_3$ | $OCH_3$ | $CH_2CH_2CH_2CH_3$ | 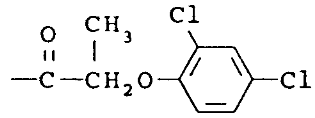 |
| $OCH_3$ | $OCH_3$ | $CH(CH_3)C_2H_5$ | 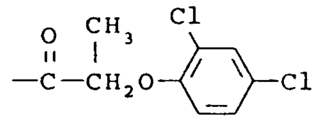 |
| $OCH_3$ | $OCH_3$ | $CH_2CH(CH_3)_2$ | 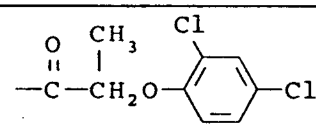 |
| $OCH_3$ | $OCH_3$ | $C(CH_3)_3$ | 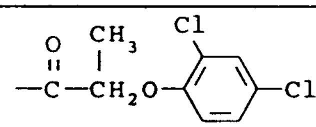 |
| $OCH_3$ | $OCH_3$ | $C_2H_5$ | 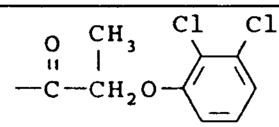 |
| $OCH_3$ | $OCH_3$ | $CH_2CH_2CH_3$ | 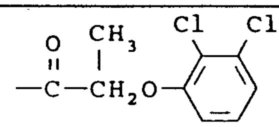 |
| $OCH_3$ | $OCH_3$ | $CH(CH_3)_2$ | 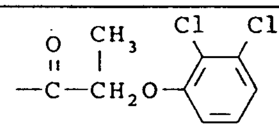 |
| $OCH_3$ | $OCH_3$ | $CH_2CH_2CH_2CH_3$ | 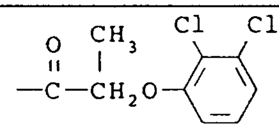 |

Table 1 (continued)

| R1 | R2 | R3 | R4 |
|---|---|---|---|
| $OCH_3$ | $OCH_3$ | $CH(CH_3)C_2H_5$ | 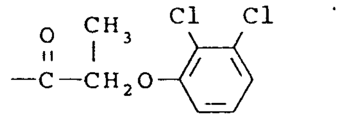 |
| $OCH_3$ | $OCH_3$ | $CH_2CH(CH_3)_2$ | 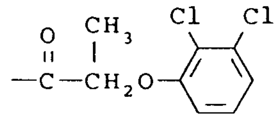 |
| $OCH_3$ | $OCH_3$ | $C(CH_3)_3$ | 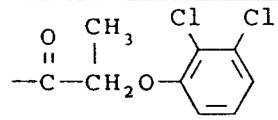 |
| $OCH_3$ | $OCH_3$ | $C_2H_5$ | 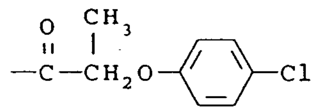 |
| $OCH_3$ | $OCH_3$ | $CH_2CH_2CH_3$ | 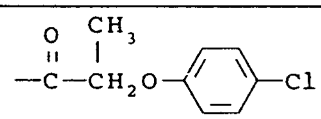 |
| $OCH_3$ | $OCH_3$ | $CH(CH_3)_2$ | 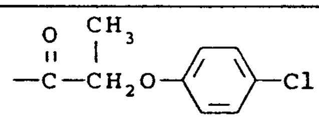 |
| $OCH_3$ | $OCH_3$ | $CH_2CH_2CH_2CH_3$ | 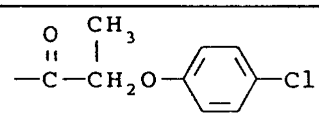 |
| $OCH_3$ | $OCH_3$ | $CH(CH_3)C_2H_5$ | 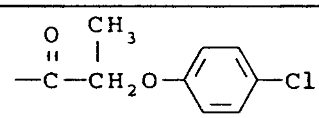 |
| $OCH_3$ | $OCH_3$ | $CH_2CH(CH_3)_2$ | 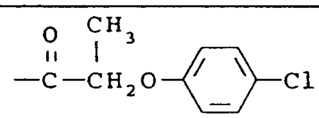 |
| $OCH_3$ | $OCH_3$ | $C(CH_3)_3$ | 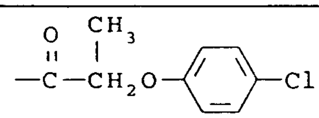 |
| $OCH_3$ | $OCH_3$ | $C_2H_5$ | 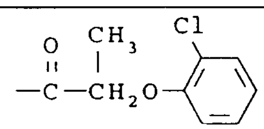 |

Table 1 (continued)

| R1 | R2 | R3 | R4 |
|---|---|---|---|
| $OCH_3$ | $OCH_3$ | $CH_2CH_2CH_3$ | 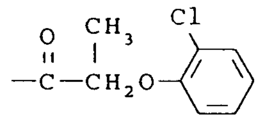 |
| $OCH_3$ | $OCH_3$ | $CH(CH_3)_2$ | 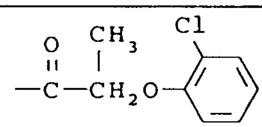 |
| $OCH_3$ | $OCH_3$ | $CH_2CH_2CH_2CH_3$ | 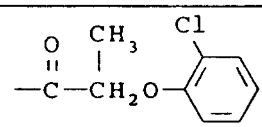 |
| $OCH_3$ | $OCH_3$ | $CH(CH_3)C_2H_5$ | 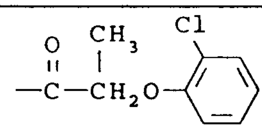 |
| $OCH_3$ | $OCH_3$ | $CH_2CH(CH_3)_2$ | 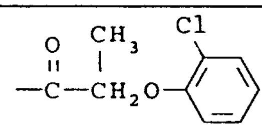 |
| $OCH_3$ | $OCH_3$ | $C(CH_3)_3$ | 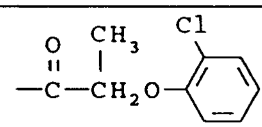 |
| $OCH_3$ | $OCH_3$ | $C_2H_5$ | 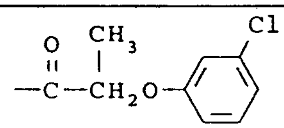 |
| $OCH_3$ | $OCH_3$ | $CH_2CH_2CH_3$ | 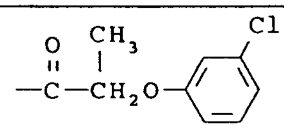 |
| $OCH_3$ | $OCH_3$ | $CH(CH_3)_2$ | 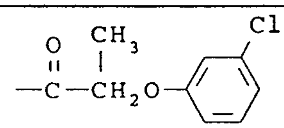 |
| $OCH_3$ | $OCH_3$ | $CH_2CH_2CH_2CH_3$ | 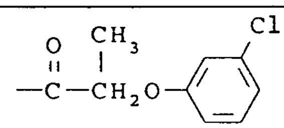 |

Table 1 (continued)

| R1 | R2 | R3 | R4 |
|---|---|---|---|
| $OCH_3$ | $OCH_3$ | $CH(CH_3)C_2H_5$ | 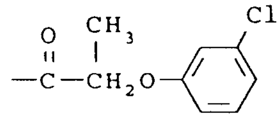 |
| $OCH_3$ | $OCH_3$ | $CH_2CH(CH_3)_2$ | 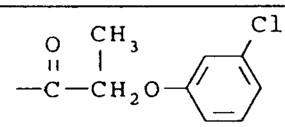 |
| $OCH_3$ | $OCH_3$ | $C(CH_3)_3$ | 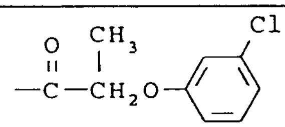 |
| $OCH_3$ | $OCH_3$ | $C_2H_5$ | 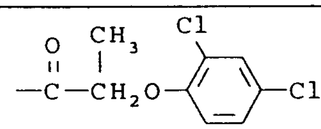 |
| $OCH_3$ | $OCH_3$ | $CH_2CH_2CH_3$ | 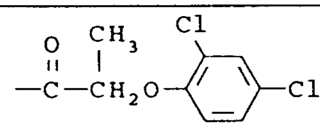 |
| $OCH_3$ | $OCH_3$ | $CH(CH_3)_2$ | 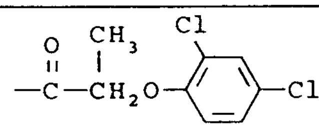 |
| $OCH_3$ | $OCH_3$ | $CH_2CH_2CH_2CH_3$ | 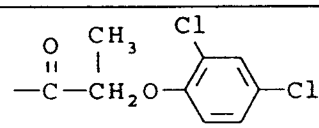 |
| $OCH_3$ | $OCH_3$ | $CH(CH_3)C_2H_5$ | 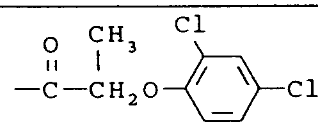 |
| $OCH_3$ | $OCH_3$ | $CH_2CH(CH_3)_2$ | 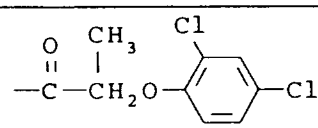 |

Table 1 (continued)

| R1 | R2 | R3 | R4 |
|---|---|---|---|
| $OCH_3$ | $OCH_3$ | $C(CH_3)_3$ | 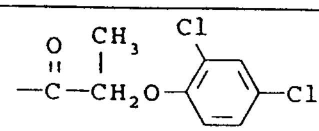 |
| $OCH_3$ | $OCH_3$ | $C_2H_5$ | 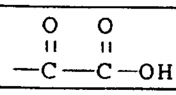 |
| $OCH_3$ | $OCH_3$ | $CH_2CH_2CH_3$ | 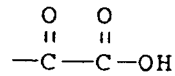 |
| $OCH_3$ | $OCH_3$ | $CH(CH_3)_2$ | 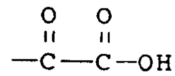 |
| $OCH_3$ | $OCH_3$ | $CH_2CH_2CH_2CH_3$ | 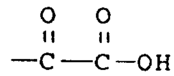 |
| $OCH_3$ | $OCH_3$ | $CH(CH_3)C_2H_5$ | 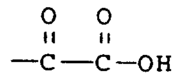 |
| $OCH_3$ | $OCH_3$ | $CH_2CH(CH_3)_2$ | 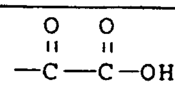 |
| $OCH_3$ | $OCH_3$ | $C(CH_3)_3$ | 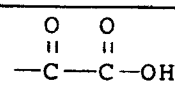 |
| $OCH_3$ | $OCH_3$ | $C_2H_5$ | 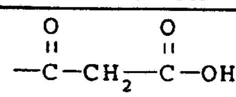 |
| $OCH_3$ | $OCH_3$ | $CH_2CH_2CH_3$ | 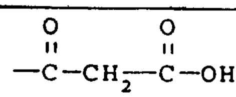 |
| $OCH_3$ | $OCH_3$ | $CH(CH_3)_2$ | 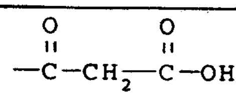 |
| $OCH_3$ | $OCH_3$ | $CH_2CH_2CH_2CH_3$ | 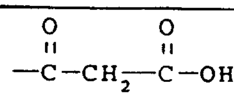 |
| $OCH_3$ | $OCH_3$ | $CH(CH_3)C_2H_5$ | 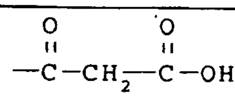 |
| $OCH_3$ | $OCH_3$ | $CH_2CH(CH_3)_2$ | 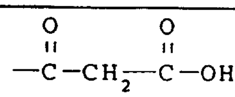 |
| $OCH_3$ | $OCH_3$ | $C(CH_3)_3$ | 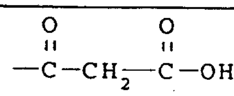 |

Table 1 (continued)

| R1 | R2 | R3 | R4 |
|---|---|---|---|
| $OCH_3$ | $OCH_3$ | $C_2H_5$ | 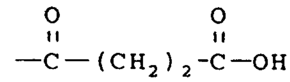 |
| $OCH_3$ | $OCH_3$ | $CH_2CH_2CH_3$ | 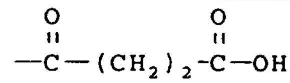 |
| $OCH_3$ | $OCH_3$ | $CH(CH_3)_2$ | 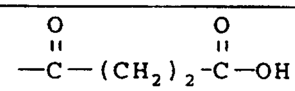 |
| $OCH_3$ | $OCH_3$ | $CH_2CH_2CH_2CH_3$ | 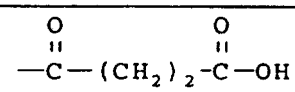 |
| $OCH_3$ | $OCH_3$ | $CH(CH_3)C_2H_5$ | 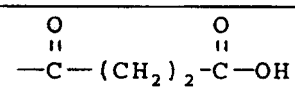 |
| $OCH_3$ | $OCH_3$ | $CH_2CH(CH_3)_2$ | 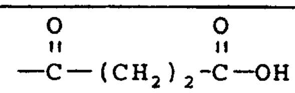 |
| $OCH_3$ | $OCH_3$ | $C(CH_3)_3$ | 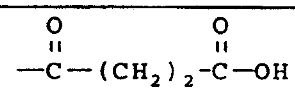 |
| $OCH_3$ | $OCH_3$ | $C_2H_5$ | 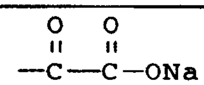 |
| $OCH_3$ | $OCH_3$ | $CH_2CH_2CH_3$ | 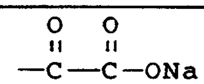 |
| $OCH_3$ | $OCH_3$ | $CH(CH_3)_2$ | 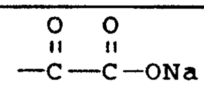 |
| $OCH_3$ | $OCH_3$ | $CH_2CH_2CH_2CH_3$ | 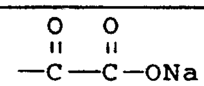 |
| $OCH_3$ | $OCH_3$ | $CH(CH_3)C_2H_5$ | 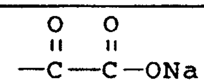 |
| $OCH_3$ | $OCH_3$ | $CH_2CH(CH_3)_2$ | 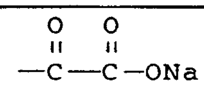 |
| $OCH_3$ | $OCH_3$ | $C(CH_3)_3$ | 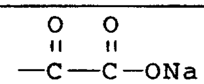 |
| $OCH_3$ | $OCH_3$ | $C_2H_5$ | 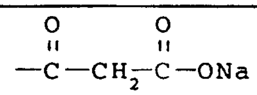 |

Table 1 (continued)

| R1 | R2 | R3 | R4 |
|---|---|---|---|
| $OCH_3$ | $OCH_3$ | $CH_2CH_2CH_3$ | 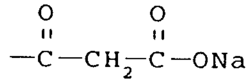 |
| $OCH_3$ | $OCH_3$ | $CH(CH_3)_2$ | 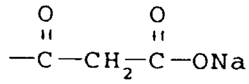 |
| $OCH_3$ | $OCH_3$ | $CH_2CH_2CH_2CH_3$ | 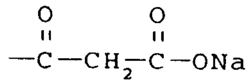 |
| $OCH_3$ | $OCH_3$ | $CH(CH_3)C_2H_5$ | 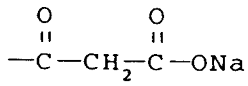 |
| $OCH_3$ | $OCH_3$ | $CH_2CH(CH_3)_2$ | 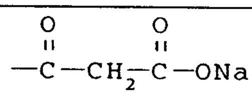 |
| $OCH_3$ | $OCH_3$ | $C(CH_3)_3$ | 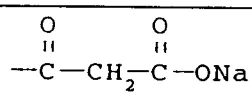 |
| $OCH_3$ | $OCH_3$ | $C_2H_5$ | 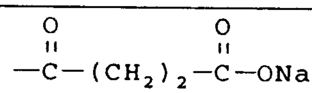 |
| $OCH_3$ | $OCH_3$ | $CH_2CH_2CH_3$ | 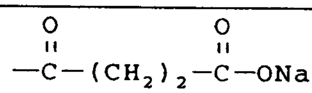 |
| $OCH_3$ | $OCH_3$ | $CH(CH_3)_2$ | 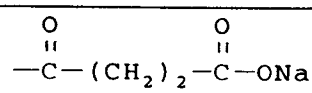 |
| $OCH_3$ | $OCH_3$ | $CH_2CH_2CH_2CH_3$ | 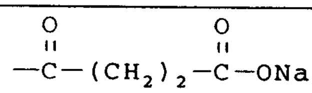 |
| $OCH_3$ | $OCH_3$ | $CH(CH_3)C_2H_5$ | 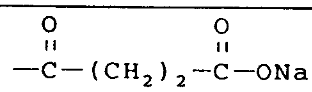 |
| $OCH_3$ | $OCH_3$ | $CH_2CH(CH_3)_2$ | 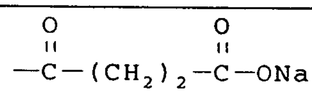 |
| $OCH_3$ | $OCH_3$ | $C(CH_3)_3$ | 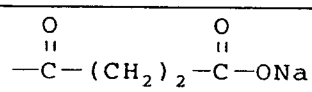 |
| $OCH_3$ | $OCH_3$ | $C_2H_5$ | 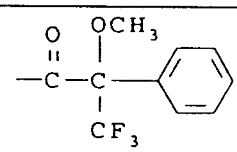 |

Table 1 (continued)

| R1 | R2 | R3 | R4 |
|---|---|---|---|
| $OCH_3$ | $OCH_3$ | $CH_2CH_2CH_3$ | 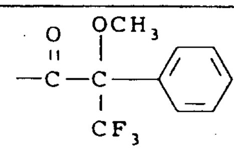 |
| $OCH_3$ | $OCH_3$ | $CH(CH_3)_2$ | 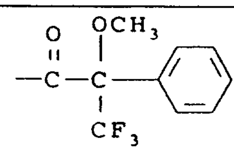 |
| $OCH_3$ | $OCH_3$ | $CH_2CH_2CH_2CH_3$ | 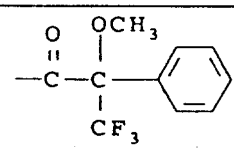 |
| $OCH_3$ | $OCH_3$ | $CH(CH_3)C_2H_5$ | 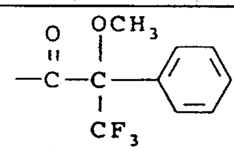 |
| $OCH_3$ | $OCH_3$ | $CH_2CH(CH_3)_2$ | 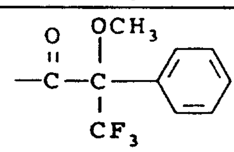 |
| $OCH_3$ | $OCH_3$ | $C(CH_3)_3$ | 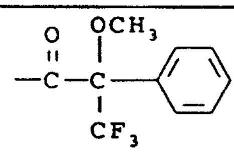 |
| $OCH_3$ | $OCH_3$ | $C_2H_5$ | 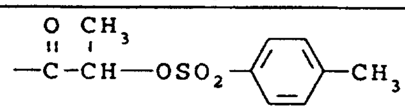 |
| $OCH_3$ | $OCH_3$ | $CH_2CH_2CH_3$ | 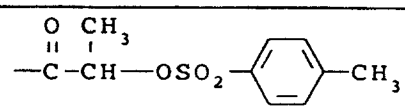 |
| $OCH_3$ | $OCH_3$ | $CH(CH_3)_2$ | 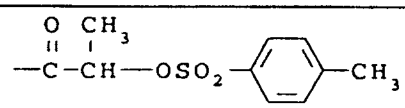 |
| $OCH_3$ | $OCH_3$ | $CH_2CH_2CH_2CH_3$ | 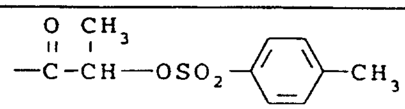 |
| $OCH_3$ | $OCH_3$ | $CH(CH_3)C_2H_5$ | 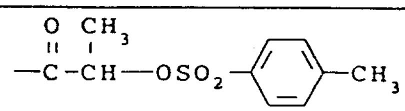 |

Table 1 (continued)

| R1 | R2 | R3 | R4 |
|---|---|---|---|
| $OCH_3$ | $OCH_3$ | $CH_2CH(CH_3)_2$ | 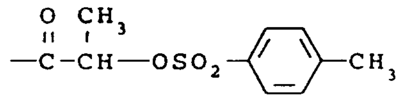 |
| $OCH_3$ | $OCH_3$ | $C(CH_3)_3$ | 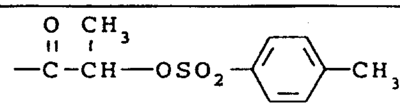 |
| $OCH_3$ | $OCH_3$ | $C_2H_5$ | 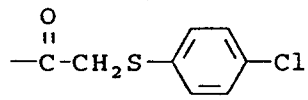 |
| $OCH_3$ | $OCH_3$ | $CH_2CH_2CH_3$ | 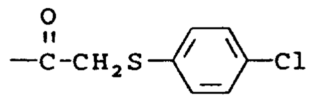 |
| $OCH_3$ | $OCH_3$ | $CH(CH_3)_2$ | 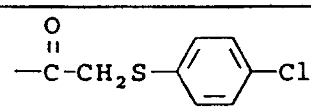 |
| $OCH_3$ | $OCH_3$ | $CH_2CH_2CH_2CH_3$ | 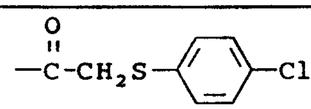 |
| $OCH_3$ | $OCH_3$ | $CH(CH_3)C_2H_5$ | 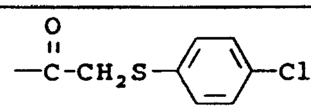 |
| $OCH_3$ | $OCH_3$ | $CH_2CH(CH_3)_2$ | 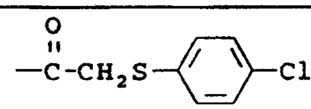 |
| $OCH_3$ | $OCH_3$ | $C(CH_3)_3$ | 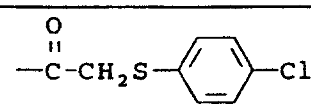 |
| $OCH_3$ | $OCH_3$ | $C_2H_5$ | 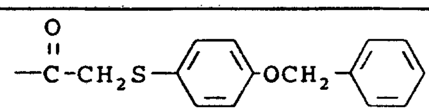 |
| $OCH_3$ | $OCH_3$ | $CH_2CH_2CH_3$ | 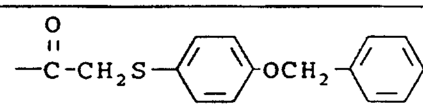 |
| $OCH_3$ | $OCH_3$ | $CH(CH_3)_2$ | 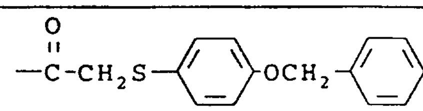 |

Table 1 (continued)

| R1 | R2 | R3 | R4 |
|---|---|---|---|
| $OCH_3$ | $OCH_3$ | $CH_2CH_2CH_2CH_3$ | 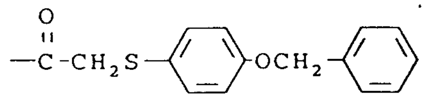 |
| $OCH_3$ | $OCH_3$ | $CH(CH_3)C_2H_5$ | 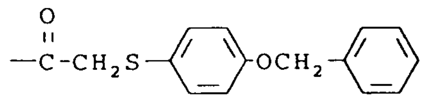 |
| $OCH_3$ | $OCH_3$ | $CH_2CH(CH_3)_2$ | 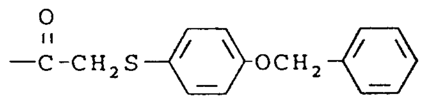 |
| $OCH_3$ | $OCH_3$ | $C(CH_3)_3$ | 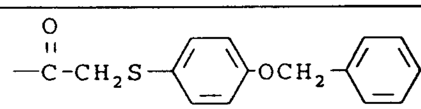 |
| $OCH_3$ | $OCH_3$ | $C_2H_5$ | 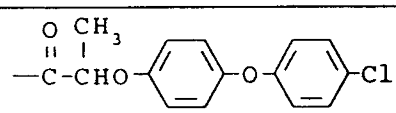 |
| $OCH_3$ | $OCH_3$ | $CH_2CH_2CH_3$ | 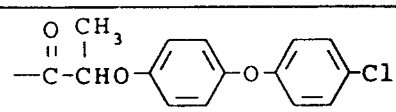 |
| $OCH_3$ | $OCH_3$ | $CH(CH_3)_2$ | 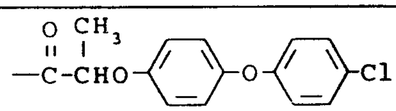 |
| $OCH_3$ | $OCH_3$ | $CH_2CH_2CH_2CH_3$ | 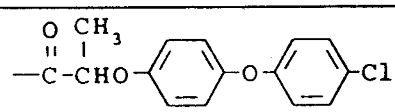 |
| $OCH_3$ | $OCH_3$ | $CH(CH_3)C_2H_5$ | 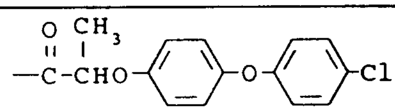 |
| $OCH_3$ | $OCH_3$ | $CH_2CH(CH_3)_2$ | 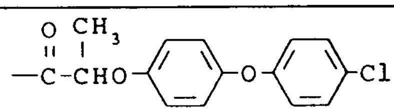 |
| $OCH_3$ | $OCH_3$ | $C(CH_3)_3$ | 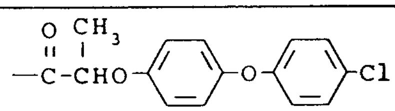 |
| $OCH_3$ | $OCH_3$ | $C_2H_5$ | 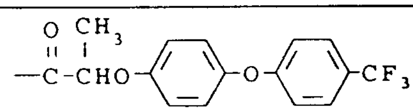 |

Table 1 (continued)

| R1 | R2 | R3 | R4 |
|---|---|---|---|
| $OCH_3$ | $OCH_3$ | $CH_2CH_2CH_3$ | 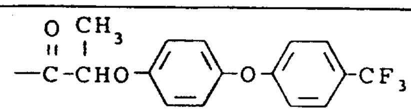 |
| $OCH_3$ | $OCH_3$ | $CH(CH_3)_2$ | 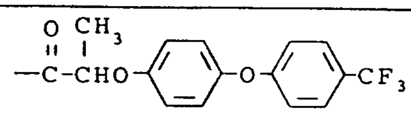 |
| $OCH_3$ | $OCH_3$ | $CH_2CH_2CH_2CH_3$ | 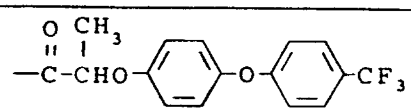 |
| $OCH_3$ | $OCH_3$ | $CH(CH_3)C_2H_5$ | 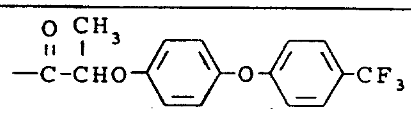 |
| $OCH_3$ | $OCH_3$ | $CH_2CH(CH_3)_2$ | 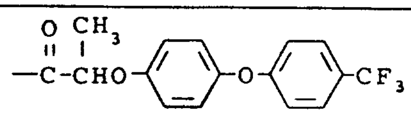 |
| $OCH_3$ | $OCH_3$ | $C(CH_3)_3$ | 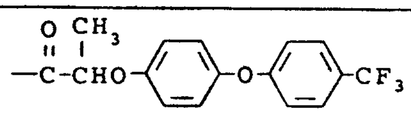 |
| $OCH_3$ | $OCH_3$ | $C_2H_5$ | 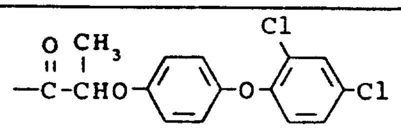 |
| $OCH_3$ | $OCH_3$ | $CH_2CH_2CH_3$ | 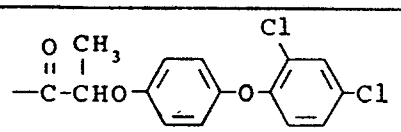 |
| $OCH_3$ | $OCH_3$ | $CH(CH_3)_2$ | 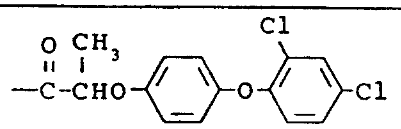 |
| $OCH_3$ | $OCH_3$ | $CH_2CH_2CH_2CH_3$ | 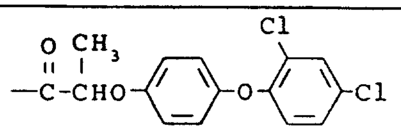 |
| $OCH_3$ | $OCH_3$ | $CH(CH_3)C_2H_5$ | 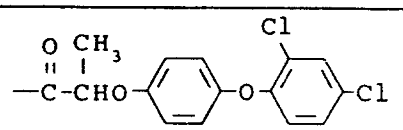 |

Table 1 (continued)

| R1 | R2 | R3 | R4 |
|---|---|---|---|
| $OCH_3$ | $OCH_3$ | $CH_2CH(CH_3)_2$ | 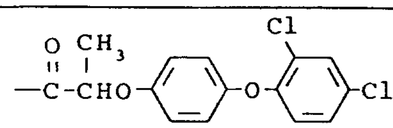 |
| $OCH_3$ | $OCH_3$ | $C(CH_3)_3$ | 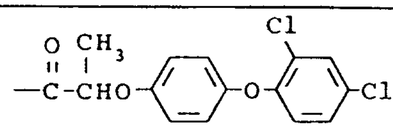 |
| $OCH_3$ | $OCH_3$ | $C_2H_5$ | 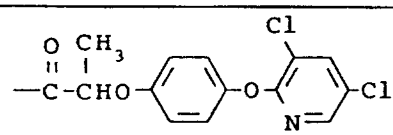 |
| $OCH_3$ | $OCH_3$ | $CH_2CH_2CH_3$ | 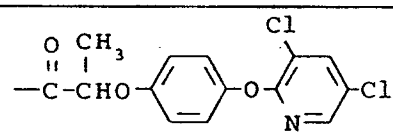 |
| $OCH_3$ | $OCH_3$ | $CH(CH_3)_2$ | 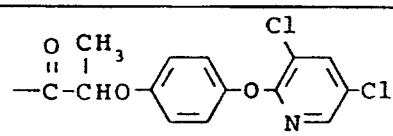 |
| $OCH_3$ | $OCH_3$ | $CH_2CH_2CH_2CH_3$ | 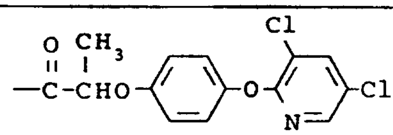 |
| $OCH_3$ | $OCH_3$ | $CH(CH_3)C_2H_5$ | 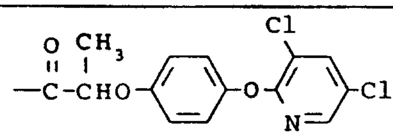 |
| $OCH_3$ | $OCH_3$ | $CH_2CH(CH_3)_2$ | 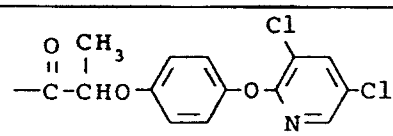 |
| $OCH_3$ | $OCH_3$ | $C(CH_3)_3$ | 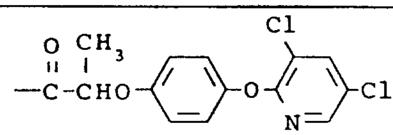 |
| $OCH_3$ | $OCH_3$ | $C_2H_5$ | 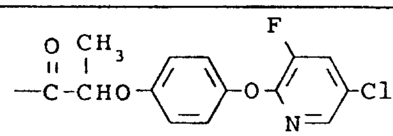 |

Table 1 (continued)

| R1 | R2 | R3 | R4 |
|---|---|---|---|
| $OCH_3$ | $OCH_3$ | $CH_2CH_2CH_3$ | 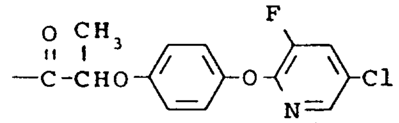 |
| $OCH_3$ | $OCH_3$ | $CH(CH_3)_2$ | 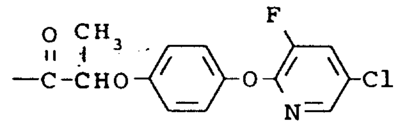 |
| $OCH_3$ | $OCH_3$ | $CH_2CH_2CH_2CH_3$ | 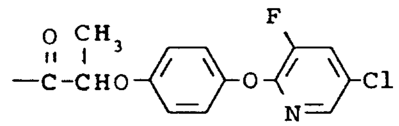 |
| $OCH_3$ | $OCH_3$ | $CH(CH_3)C_2H_5$ | 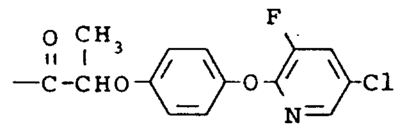 |
| $OCH_3$ | $OCH_3$ | $CH_2CH(CH_3)_2$ | 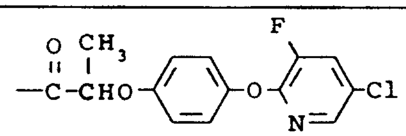 |
| $OCH_3$ | $OCH_3$ | $C(CH_3)_3$ | 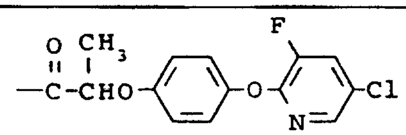 |
| $OCH_3$ | $OCH_3$ | $C_2H_5$ | 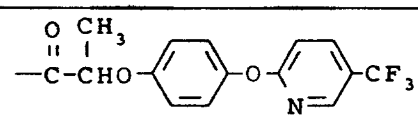 |
| $OCH_3$ | $OCH_3$ | $CH_2CH_2CH_3$ | 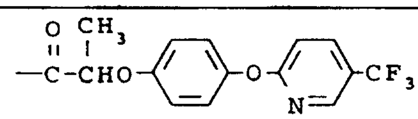 |
| $OCH_3$ | $OCH_3$ | $CH(CH_3)_2$ | 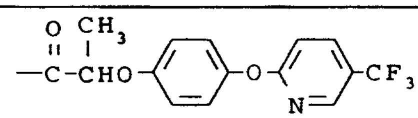 |
| $OCH_3$ | $OCH_3$ | $CH_2CH_2CH_2CH_3$ | 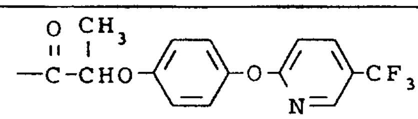 |
| $OCH_3$ | $OCH_3$ | $CH(CH_3)C_2H_5$ | 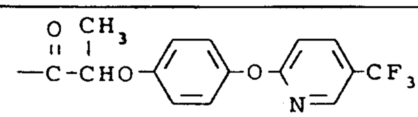 |

Table 1 (continued)

| R1 | R2 | R3 | R4 |
|---|---|---|---|
| $OCH_3$ | $OCH_3$ | $CH_2CH(CH_3)_2$ | 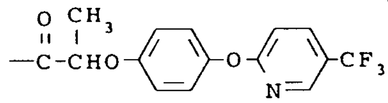 |
| $OCH_3$ | $OCH_3$ | $C(CH_3)_3$ | 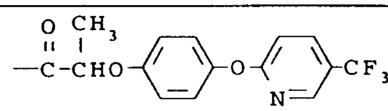 |
| $OCH_3$ | $OCH_3$ | $C_2H_5$ | 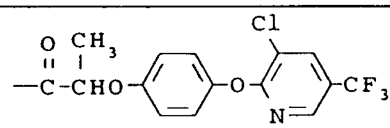 |
| $OCH_3$ | $OCH_3$ | $CH_2CH_2CH_3$ | 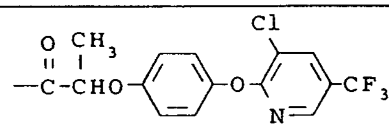 |
| $OCH_3$ | $OCH_3$ | $CH(CH_3)_2$ | 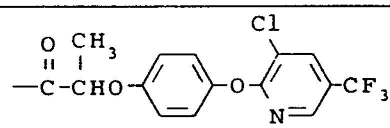 |
| $OCH_3$ | $OCH_3$ | $CH_2CH_2CH_2CH_3$ | 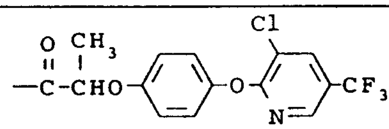 |
| $OCH_3$ | $OCH_3$ | $CH(CH_3)C_2H_5$ | 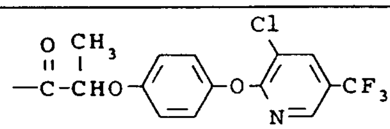 |
| $OCH_3$ | $OCH_3$ | $CH_2CH(CH_3)_2$ | 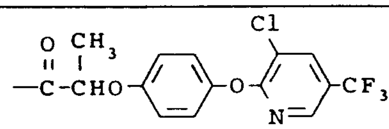 |
| $OCH_3$ | $OCH_3$ | $C(CH_3)_3$ | 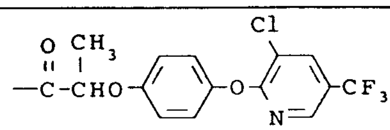 |
| $OCH_3$ | $OCH_3$ | $C_2H_5$ | 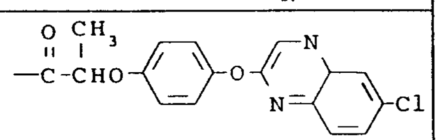 |
| $OCH_3$ | $OCH_3$ | $CH_2CH_2CH_3$ | 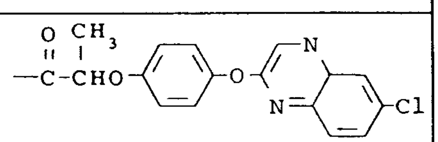 |

Table 1 (continued)

| R1 | R2 | R3 | R4 |
|---|---|---|---|
| $OCH_3$ | $OCH_3$ | $CH(CH_3)_2$ | 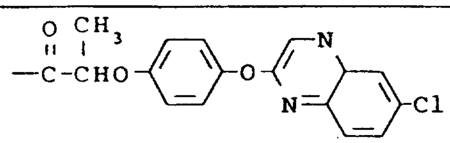 |
| $OCH_3$ | $OCH_3$ | $CH_2CH_2CH_2CH_3$ | 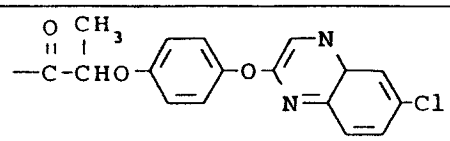 |
| $OCH_3$ | $OCH_3$ | $CH(CH_3)C_2H_5$ | 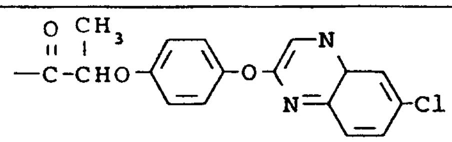 |
| $OCH_3$ | $OCH_3$ | $CH_2CH(CH_3)_2$ | 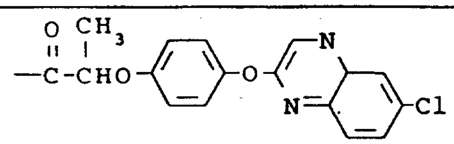 |
| $OCH_3$ | $OCH_3$ | $C(CH_3)_3$ | 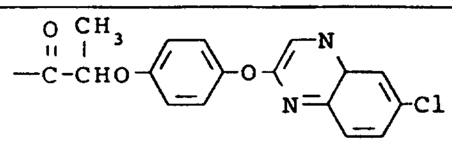 |
| $OCH_3$ | $OCH_3$ | $C_2H_5$ | 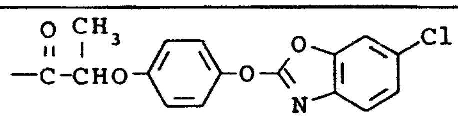 |
| $OCH_3$ | $OCH_3$ | $CH_2CH_2CH_3$ | 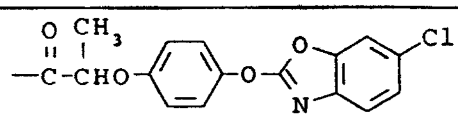 |
| $OCH_3$ | $OCH_3$ | $CH(CH_3)_2$ | 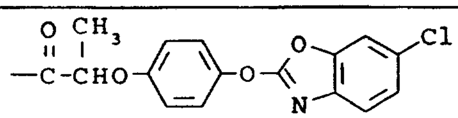 |
| $OCH_3$ | $OCH_3$ | $CH_2CH_2CH_2CH_3$ | 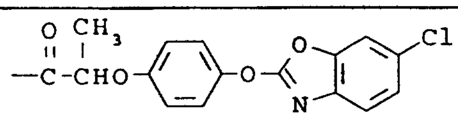 |
| $OCH_3$ | $OCH_3$ | $CH(CH_3)C_2H_5$ | 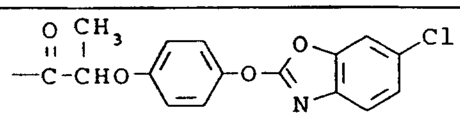 |
| $OCH_3$ | $OCH_3$ | $CH_2CH(CH_3)_2$ | 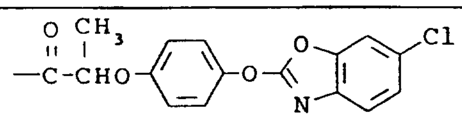 |

Table 1 (continued)

| R1 | R2 | R3 | R4 |
|---|---|---|---|
| $OCH_3$ | $OCH_3$ | $C(CH_3)_3$ | 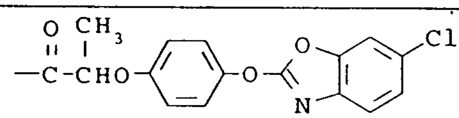 |
| $OCH_3$ | $OCH_3$ | $C_2H_5$ | 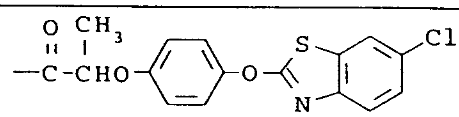 |
| $OCH_3$ | $OCH_3$ | $CH_2CH_2CH_3$ | 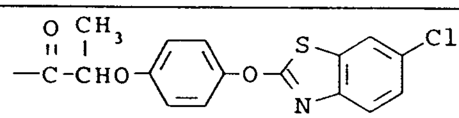 |
| $OCH_3$ | $OCH_3$ | $CH(CH_3)_2$ | 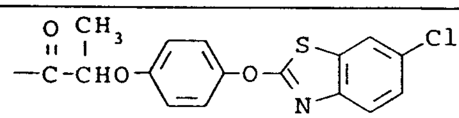 |
| $OCH_3$ | $OCH_3$ | $CH_2CH_2CH_2CH_3$ | 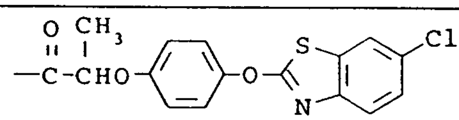 |
| $OCH_3$ | $OCH_3$ | $CH(CH_3)C_2H_5$ | 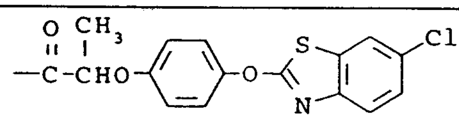 |
| $OCH_3$ | $OCH_3$ | $CH_2CH(CH_3)_2$ | 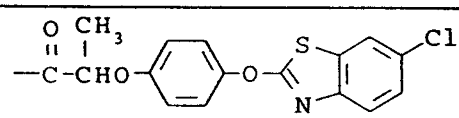 |
| $OCH_3$ | $OCH_3$ | $C(CH_3)_3$ | 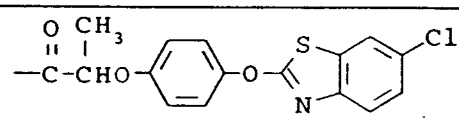 |
| $OCH_3$ | $OCH_3$ | $C_2H_5$ | 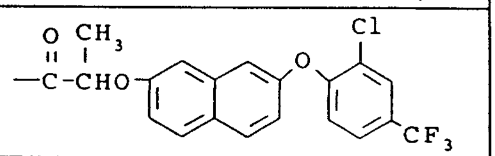 |
| $OCH_3$ | $OCH_3$ | $CH_2CH_2CH_3$ | 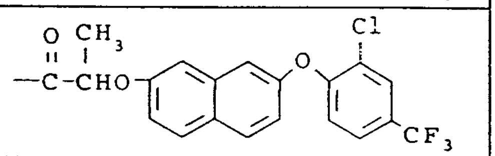 |
| $OCH_3$ | $OCH_3$ | $CH(CH_3)_2$ | 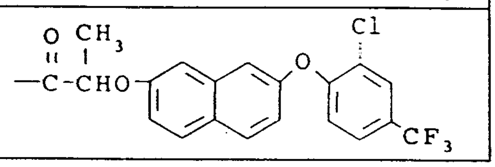 |

Table 1 (continued)

| R1 | R2 | R3 | R4 |
|---|---|---|---|
| $OCH_3$ | $OCH_3$ | $CH_2CH_2CH_2CH_3$ | 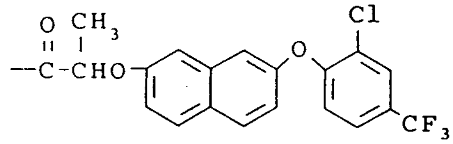 |
| $OCH_3$ | $OCH_3$ | $CH(CH_3)C_2H_5$ | 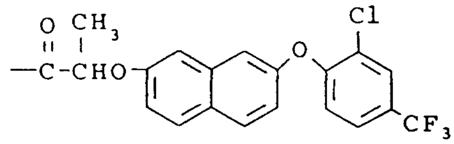 |
| $OCH_3$ | $OCH_3$ | $CH_2CH(CH_3)_2$ | 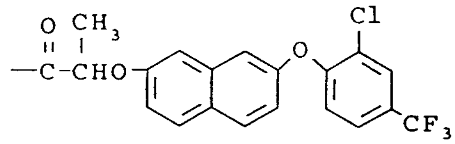 |
| $OCH_3$ | $OCH_3$ | $C(CH_3)_3$ | 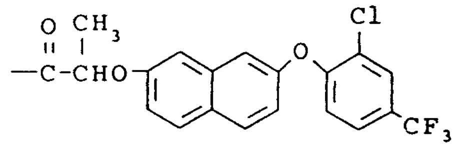 |
| $OCH_3$ | $OCH_3$ | $C_2H_5$ | 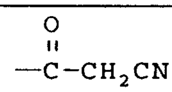 |
| $OCH_3$ | $OCH_3$ | $CH_2CH_2CH_3$ | 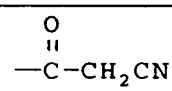 |
| $OCH_3$ | $OCH_3$ | $CH(CH_3)_2$ | 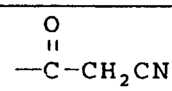 |
| $OCH_3$ | $OCH_3$ | $CH_2CH_2CH_2CH_3$ | 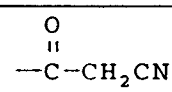 |
| $OCH_3$ | $OCH_3$ | $CH(CH_3)C_2H_5$ | 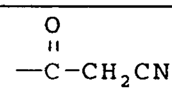 |
| $OCH_3$ | $OCH_3$ | $CH_2CH(CH_3)_2$ | 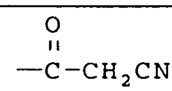 |
| $OCH_3$ | $OCH_3$ | $C(CH_3)_3$ | 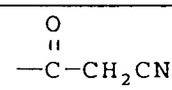 |
| $OCH_3$ | $OCH_3$ | $C_2H_5$ | 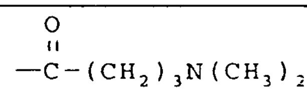 |

Table 1 (continued)

| R1 | R2 | R3 | R4 |
|---|---|---|---|
| $OCH_3$ | $OCH_3$ | $CH(CH_3)_2$ | 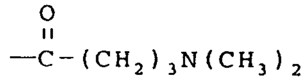 |
| $OCH_3$ | $OCH_3$ | $CH_2CH_2CH_2CH_3$ | 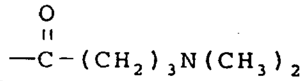 |
| $OCH_3$ | $OCH_3$ | $CH(CH_3)C_2H_5$ | 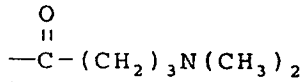 |
| $OCH_3$ | $OCH_3$ | $CH_2CH(CH_3)_2$ | 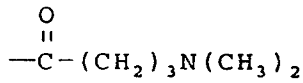 |
| $OCH_3$ | $OCH_3$ | $C(CH_3)_3$ | 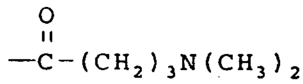 |
| $OCH_3$ | $OCH_3$ | $C_2H_5$ | 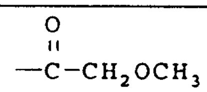 |
| $OCH_3$ | $OCH_3$ | $CH_2CH_2CH_3$ | 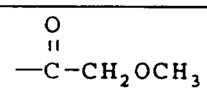 |
| $OCH_3$ | $OCH_3$ | $CH(CH_3)_2$ | 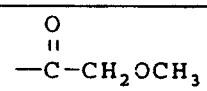 |
| $OCH_3$ | $OCH_3$ | $CH_2CH_2CH_2CH_3$ | 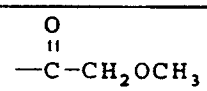 |
| $OCH_3$ | $OCH_3$ | $CH(CH_3)C_2H_5$ | 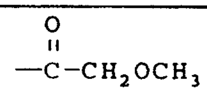 |
| $OCH_3$ | $OCH_3$ | $CH_2CH(CH_3)_2$ | 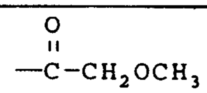 |
| $OCH_3$ | $OCH_3$ | $C(CH_3)_3$ | 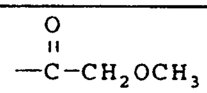 |
| $OCH_3$ | $OCH_3$ | $C_2H_5$ | 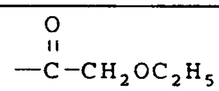 |
| $OCH_3$ | $OCH_3$ | $CH_2CH_2CH_3$ | 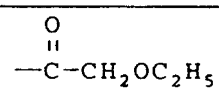 |

Table 1 (continued)

| R1 | R2 | R3 | R4 |
|---|---|---|---|
| $OCH_3$ | $OCH_3$ | $CH(CH_3)_2$ | $-\overset{O}{\overset{\|}{C}}-CH_2OC_2H_5$ |
| $OCH_3$ | $OCH_3$ | $CH_2CH_2CH_2CH_3$ | $-\overset{O}{\overset{\|}{C}}-CH_2OC_2H_5$ |
| $OCH_3$ | $OCH_3$ | $CH(CH_3)C_2H_5$ | $-\overset{O}{\overset{\|}{C}}-CH_2OC_2H_5$ |
| $OCF_3$ | $OCF_3$ | $CH(CH_3)_2$ | 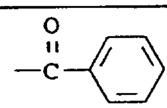 |
| $OCHF_2$ | $OCHF_2$ | $C(CH_3)_3$ | 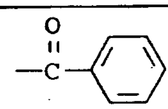 |
| $OCF_3$ | $OCF_3$ | $C(CH_3)_3$ | 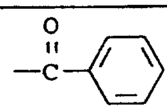 |
| $OCH_3$ | $OCH_3$ | $C(CH_3)_3$ | $-\overset{O}{\overset{\|}{C}}-CH=CH_2$ |
| $OCH_3$ | $OCH_3$ | $CH(CH_3)_2$ | $-\overset{O}{\overset{\|}{C}}-CH=CH_2$ |
| $OCH_3$ | $OCH_3$ | $C(CH_3)_3$ | $-\overset{O}{\overset{\|}{C}}-CH=CH_2$ |
| $OCH_3$ | $OCH_3$ | $C(CH_3)_3$ | $-\overset{O}{\overset{\|}{C}}-CHClCH_3$ |
| $OCH_3$ | $OCH_3$ | $C(CH_3)_3$ | $-\overset{O}{\overset{\|}{C}}-CH_2Cl$ |

Table 1 (continued)

| R1 | R2 | R3 | R4 |
|---|---|---|---|
| $OCF_3$ | $OCF_3$ | $CH_2CH_2CH_3$ | H |
| $OCH_3$ | $OCH_3$ | $CH_2CH_2CH_3$ | H |
| $OCH_3$ | $OCH_3$ | $CH(CH_2)_2$ | H |
| $OCHF_2$ | $OCHF_2$ | $C(CH_3)_3$ | H |
| $OCH_3$ | $OCH_3$ | $C(CH_3)_3$ | H |
| $OCF_3$ | $OCF_3$ | $CH_2CH_2CH_3$ | 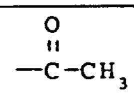 |
| $OCF_3$ | $OCF_3$ | $CH(CH_2)_2$ | 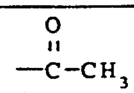 |
| $OCHF_2$ | $OCHF_2$ | $C(CH_3)_3$ | 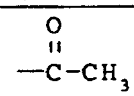 |
| $OCF_3$ | $OCF_3$ | $C(CH_3)_3$ | 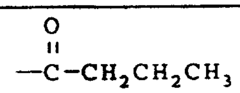 |
| $OCHF_2$ | $OCHF_2$ | $CH_2CH_2CH_3$ | 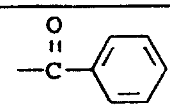 |
| $OCH_3$ | $OCH_3$ | $C(CH_3)_3$ | 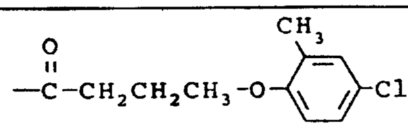 |

Table 1 (continued)

| R1 | R2 | R3 | R4 |
|---|---|---|---|
| $OCH_3$ | $OCH_3$ | $C(CH_3)_3$ | 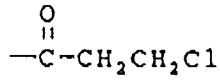 |
| $OCH_3$ | $OCH_3$ | $C(CH_3)_3$ | 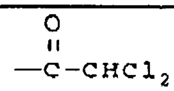 |
| $OCH_3$ | $OCH_3$ | $C(CH_3)_3$ | 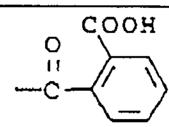 |
| $OCH_3$ | $OCH_3$ | $C(CH_3)_3$ | 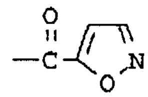 |
| $OCH_3$ | $OCH_3$ | $C(CH_3)_3$ | 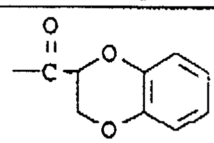 |
| $OCH_3$ | $OCH_3$ | $C(CH_3)_3$ | 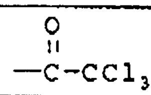 |
| $OCH_3$ | $OCH_3$ | $C(CH_3)_3$ | 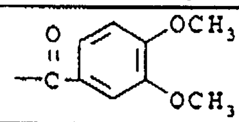 |
| $OCH_3$ | $OCH_3$ | $C(CH_3)_3$ | 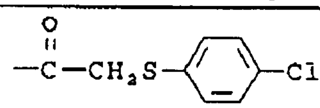 |
| $OCH_3$ | $OCH_3$ | $C(CH_3)_3$ | 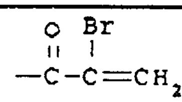 |
| $OCH_3$ | $OCH_3$ | $C(CH_3)_3$ | 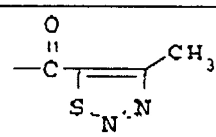 |
| $OCH_3$ | $OCH_3$ | $C(CH_3)_3$ | 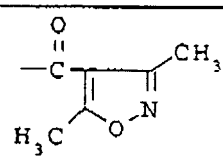 |
| $OCH_3$ | $OCH_3$ | $C(CH_3)_3$ | 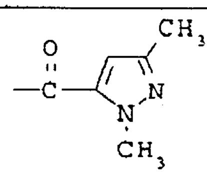 |

Table 1 (continued)

| R1 | R2 | R3 | R4 |
|---|---|---|---|
| $OCH_3$ | $OCH_3$ | $C(CH_3)_3$ | 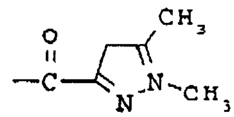 |
| $OCH_3$ | $OCH_3$ | $C(CH_3)_3$ | 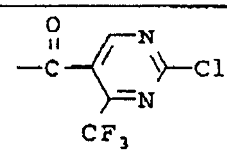 |
| $OCH_3$ | $OCH_3$ | $C(CH_3)_3$ | 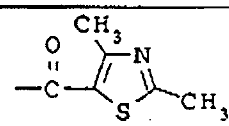 |
| $OCH_3$ | $OCH_3$ | $CH(CH_3)_2$ | 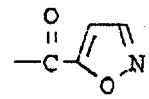 |
| $OCH_3$ | $OCH_3$ | $CH(CH_3)_2$ | 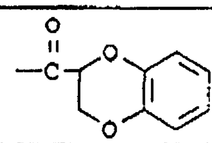 |
| $OCH_3$ | $OCH_3$ | $CH(CH_3)_2$ | 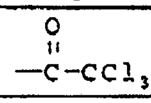 |
| $OCH_3$ | $OCH_3$ | $CH(CH_3)_2$ | 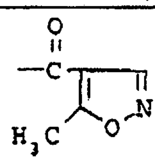 |
| $OCH_3$ | $OCH_3$ | $C(CH_3)_3$ | 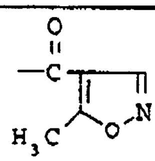 |
| $OCH_3$ | $OCH_3$ | $CH(CH_3)_2$ | 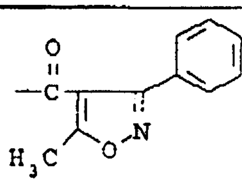 |
| $OCH_3$ | $OCH_3$ | $C(CH_3)_3$ | 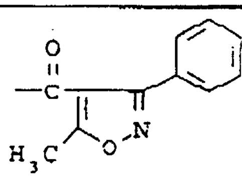 |

Table 1 (continued)

| R1 | R2 | R3 | R4 |
|---|---|---|---|
| $OCH_3$ | $OCH_3$ | $C(CH_3)_3$ | 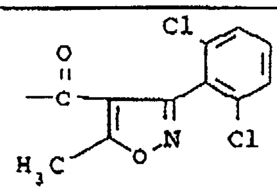 |
| $OCH_3$ | $OCH_3$ | $CH(CH_3)_2$ | 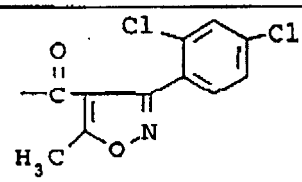 |
| $OCH_3$ | $OCH_3$ | $C(CH_3)_3$ | 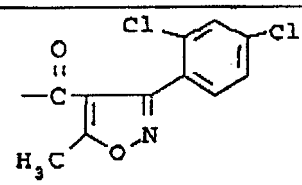 |
| $OCH_3$ | $OCH_3$ | $C(CH_3)_3$ | 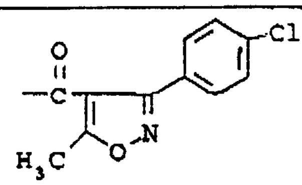 |
| $OCH_3$ | $OCH_3$ | $C(CH_3)_3$ | 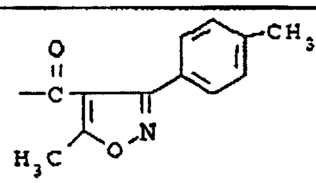 |
| $OCH_3$ | $OCH_3$ | $C(CH_3)_3$ | 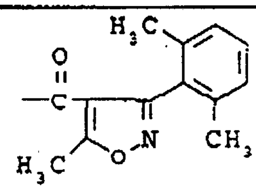 |
| $OCH_3$ | $OCH_3$ | $C(CH_3)_3$ | 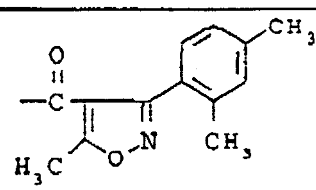 |
| $OCH_3$ | $OCH_3$ | $C(CH_3)_3$ | 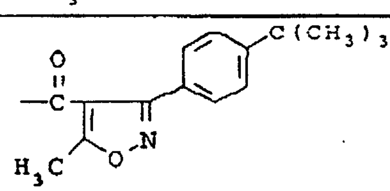 |

Table 1 (continued)

| R1 | R2 | R3 | R4 |
|---|---|---|---|
| $OCH_3$ | $OCH_3$ | $CH(CH_3)_2$ | 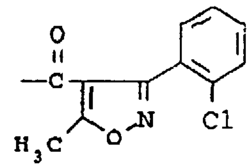 |
| $OCH_3$ | $OCH_3$ | $C(CH_3)_3$ | 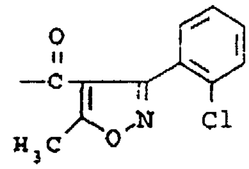 |
| $OCH_3$ | $OCH_3$ | $C(CH_3)_3$ | 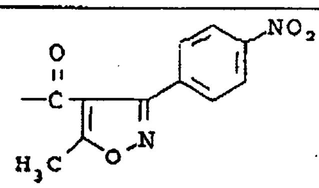 |
| $OCH_3$ | $OCH_3$ | $C(CH_3)_3$ | 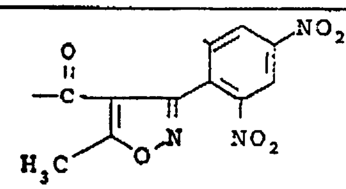 |
| $OCH_3$ | $OCH_3$ | $CH(CH_3)_2$ | 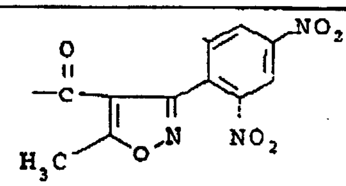 |
| $OCH_3$ | $OCH_3$ | $CH(CH_3)_2$ | 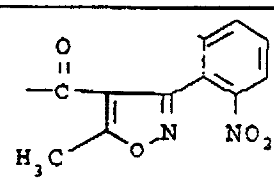 |
| $OCH_3$ | $OCH_3$ | $C(CH_3)_3$ | 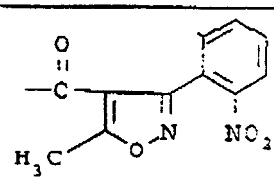 |
| $OCH_3$ | $OCH_3$ | $C(CH_3)_3$ | 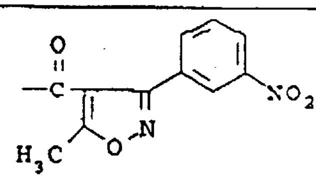 |

Table 1 (continued)

| R1 | R2 | R3 | R4 |
|---|---|---|---|
| $OCH_3$ | $OCH_3$ | $CH(CH_3)_2$ | 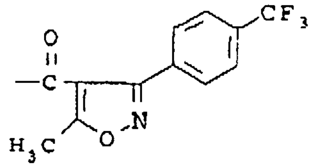 |
| $OCH_3$ | $OCH_3$ | $C(CH_3)_3$ | 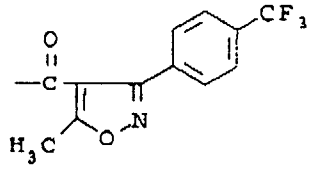 |
| $OCH_3$ | $OCH_3$ | $C(CH_3)_3$ | 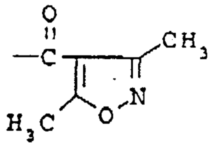 |
| $OCH_3$ | $OCH_3$ | $C(CH_3)_3$ | 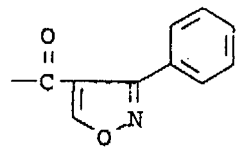 |
| $OCH_3$ | $OCH_3$ | $C(CH_3)_3$ | 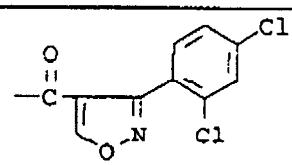 |
| $OCH_3$ | $OCH_3$ | $C(CH_3)_3$ | 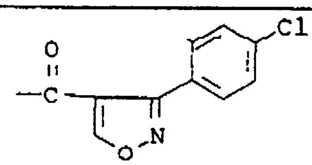 |
| $OCH_3$ | $OCH_3$ | $CH(CH_3)_2$ | 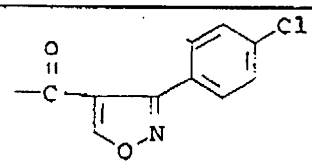 |
| $OCH_3$ | $OCH_3$ | $C(CH_3)_3$ | 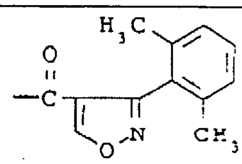 |
| $OCH_3$ | $OCH_3$ | $CH(CH_3)_2$ | 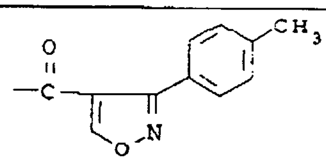 |

Table 1 (continued)

| R1 | R2 | R3 | R4 |
|---|---|---|---|
| $OCH_3$ | $OCH_3$ | $C(CH_3)_3$ | 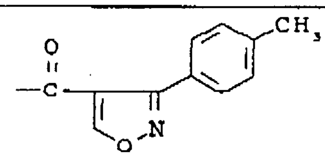 |
| $OCH_3$ | $OCH_3$ | $C(CH_3)_3$ | 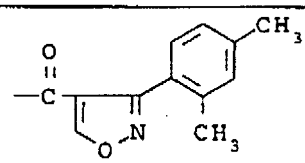 |
| $OCH_3$ | $OCH_3$ | $CH(CH_3)_2$ | 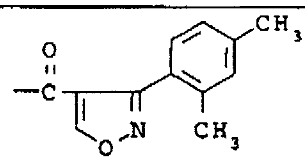 |
| $OCH_3$ | $OCH_3$ | $C(CH_3)_3$ | 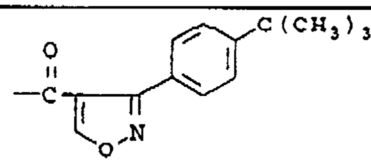 |
| $OCH_3$ | $OCH_3$ | $C(CH_3)_3$ | 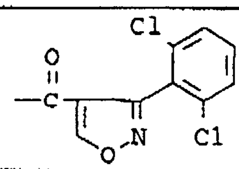 |
| $OCH_3$ | $OCH_3$ | $CH(CH_3)_2$ | 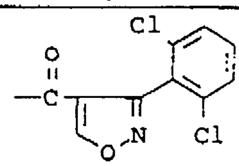 |
| $OCH_3$ | $OCH_3$ | $C(CH_3)_3$ | 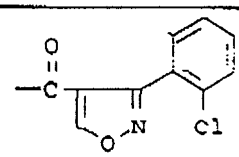 |
| $OCH_3$ | $OCH_3$ | $C(CH_3)_3$ | 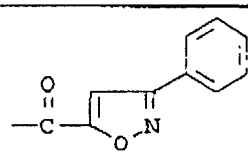 |
| $OCH_3$ | $OCH_3$ | $C(CH_3)_3$ | 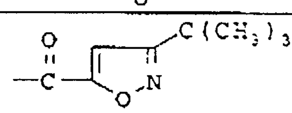 |
| $OCH_3$ | $OCH_3$ | $CH(CH_3)_2$ | 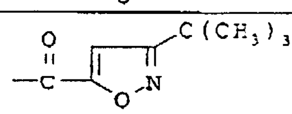 |

Table 1 (continued)

| R1 | R2 | R3 | R4 |
|---|---|---|---|
| $OCH_3$ | $OCH_3$ | $C(CH_3)_3$ | 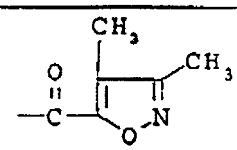 |
| $OCH_3$ | $OCH_3$ | $C(CH_3)_3$ | 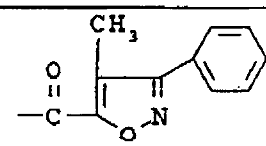 |
| $OCH_3$ | $OCH_3$ | $C(CH_3)_3$ | 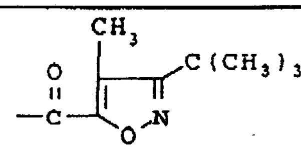 |
| $OCH_3$ | $OCH_3$ | $C(CH_3)_3$ | 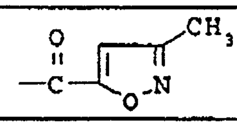 |
| $OCH_3$ | $OCH_3$ | $C(CH_3)_3$ | 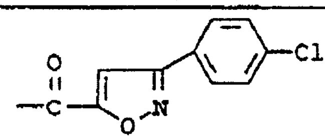 |
| $OCH_3$ | $OCH_3$ | $CH(CH_3)_2$ | 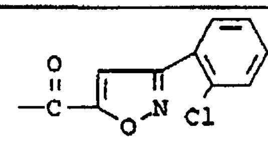 |
| $OCH_3$ | $OCH_3$ | $C(CH_3)_3$ | 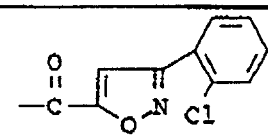 |
| $OCH_3$ | $OCH_3$ | $CH(CH_3)_2$ | 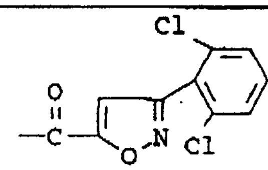 |
| $OCH_3$ | $OCH_3$ | $C(CH_3)_3$ | 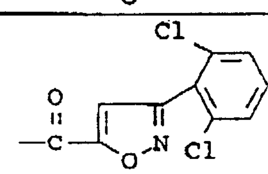 |
| $OCH_3$ | $OCH_3$ | $C(CH_3)_3$ | 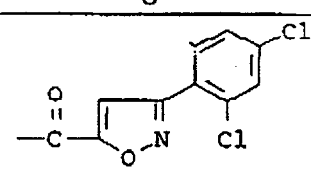 |

Table 1 (continued)

| R1 | R2 | R3 | R4 |
|---|---|---|---|
| $OCH_3$ | $OCH_3$ | $CH(CH_3)_2$ | 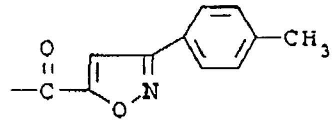 |
| $OCH_3$ | $OCH_3$ | $C(CH_3)_3$ | 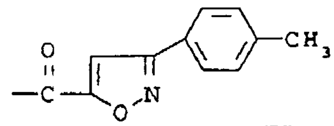 |
| $OCH_3$ | $OCH_3$ | $C(CH_3)_3$ | 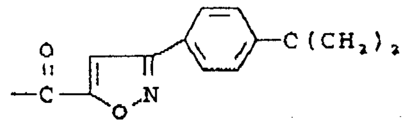 |
| $OCH_3$ | $OCH_3$ | $C(CH_3)_3$ | 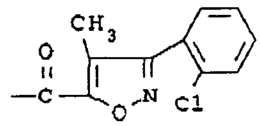 |
| $OCH_3$ | $OCH_3$ | $C(CH_3)_3$ | 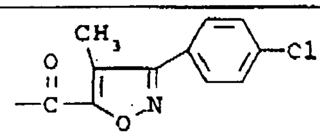 |
| $OCH_3$ | $OCH_3$ | $CH(CH_3)_2$ | 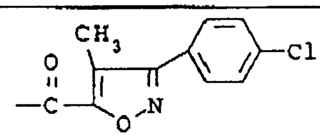 |
| $OCH_3$ | $OCH_3$ | $C(CH_3)_3$ | 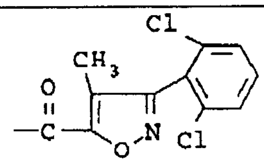 |
| $OCH_3$ | $OCH_3$ | $C(CH_3)_3$ | 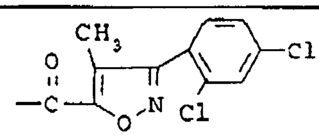 |
| $OCH_3$ | $OCH_3$ | $C(CH_3)_3$ | 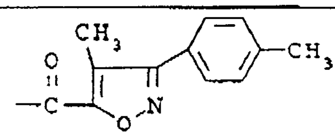 |
| $OCH_3$ | $OCH_3$ | $C(CH_3)_3$ | 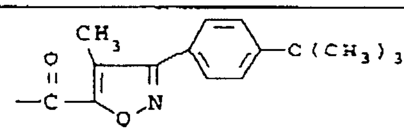 |
| $OCH_3$ | $OCH_3$ | $CH(CH_3)_2$ | 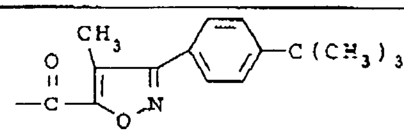 |

Table 1 (continued)

| R1 | R2 | R3 | R4 |
|---|---|---|---|
| $OCH_3$ | $OCH_3$ | $C(CH_3)_3$ | 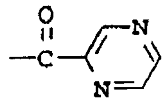 |
| $OCH_3$ | $OCH_3$ | $CH(CH_3)_2$ | 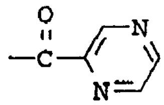 |
| $OCH_3$ | $OCH_3$ | $CH(CH_3)_2$ | 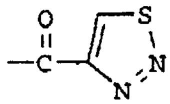 |
| $OCH_3$ | $OCH_3$ | $C(CH_3)_3$ | 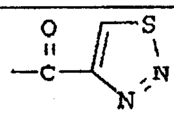 |
| $OCH_3$ | $OCH_3$ | $CH(CH_3)_2$ | 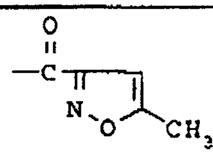 |
| $OCH_3$ | $OCH_3$ | $C(CH_3)_3$ | 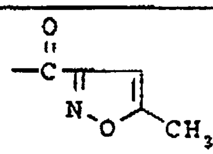 |
| $OCH_3$ | $OCH_3$ | $C(CH_3)_3$ | 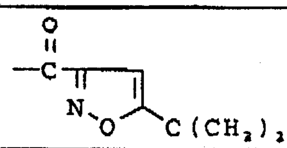 |
| $OCH_3$ | $OCH_3$ | $C(CH_3)_3$ | 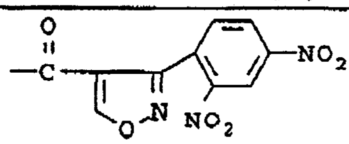 |
| $OCH_3$ | $OCH_3$ | $CH(CH_3)_2$ | 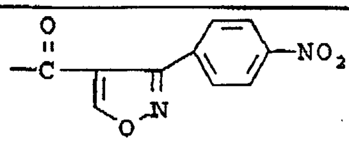 |
| $OCH_3$ | $OCH_3$ | $C(CH_3)_3$ | 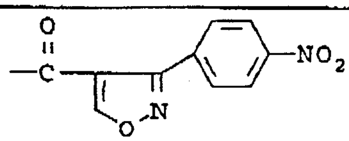 |
| $OCH_3$ | $OCH_3$ | $C(CH_3)_3$ | 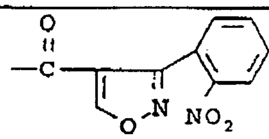 |

Table 1 (continued)

| R1 | R2 | R3 | R4 |
|---|---|---|---|
| $OCH_3$ | $OCH_3$ | $C(CH_3)_3$ | 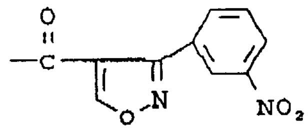 |
| $OCH_3$ | $OCH_3$ | $C(CH_3)_3$ | 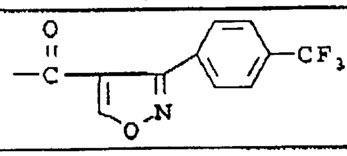 |
| $OCH_3$ | $OCH_3$ | $CH(CH_3)_2$ | 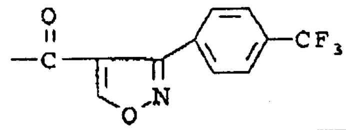 |

Table 2

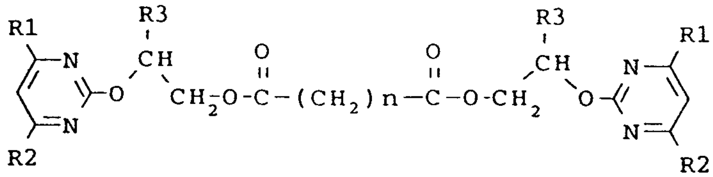

| R1 | R2 | R3 | n |
|---|---|---|---|
| $OCH_3$ | $OCH_3$ | $CH_3$ | 0 |
| $OCH_3$ | $OCH_3$ | $C_2H_5$ | 0 |
| $OCH_3$ | $OCH_3$ | $CH_2CH_2CH_3$ | 0 |
| $OCH_3$ | $OCH_3$ | $CH_2CH_2CH_3$ | 2 |
| $OCH_3$ | $OCH_3$ | $CH(CH_3)_2$ | 0 |
| $OCH_3$ | $OCH_3$ | $CH(CH_3)_2$ | 2 |
| $OCH_3$ | $OCH_3$ | $CH(CH_3)_2$ | 4 |
| $OCH_3$ | $OCH_3$ | $CH(CH_3)_2$ | 6 |
| $OCH_3$ | $OCH_3$ | $C(CH_3)_3$ | 0 |
| $OCH_3$ | $OCH_3$ | $C(CH_3)_3$ | 1 |
| $OCH_3$ | $OCH_3$ | $C(CH_3)_3$ | 2 |
| $OCH_3$ | $OCH_3$ | $C(CH_3)_3$ | 3 |
| $OCH_3$ | $OCH_3$ | $C(CH_3)_3$ | 4 |
| $OCH_3$ | $OCH_3$ | $C(CH_3)_3$ | 5 |
| $OCH_3$ | $OCH_3$ | $C(CH_3)_3$ | 6 |
| $OCH_3$ | $OCH_3$ | $CH_2CH_2CH_2CH_3$ | 0 |
| $OCH_3$ | $OCH_3$ | $CH_2CH_2CH_2CH_3$ | 1 |
| $OCH_3$ | $OCH_3$ | $CH_2CH_2CH_2CH_3$ | 2 |
| $OCH_3$ | $OCH_3$ | $CH_2CH_2CH_2CH_3$ | 4 |
| $OCH_3$ | $OCH_3$ | $CH_2CH(CH_3)_2$ | 0 |
| $OCH_3$ | $OCH_3$ | $CH_2CH(CH_3)_2$ | 2 |
| $OCH_3$ | $OCH_3$ | $CH(CH_3)CH_2CH_3$ | 0 |
| $OCH_3$ | $OCH_3$ | $CH(CH_3)CH_2CH_3$ | 2 |

Table 3

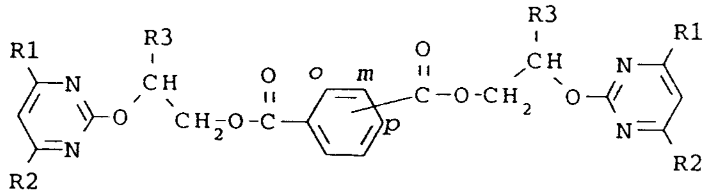

| R1 | R2 | R3 | Position of substituents |
|---|---|---|---|
| $OCH_3$ | $OCH_3$ | $CH_3$ | p |
| $OCH_3$ | $OCH_3$ | $C_2H_5$ | p |
| $OCH_3$ | $OCH_3$ | $CH_2CH_2CH_3$ | m |
| $OCH_3$ | $OCH_3$ | $CH_2CH_2CH_3$ | p |
| $OCH_3$ | $OCH_3$ | $CH(CH_3)_2$ | o |
| $OCH_3$ | $OCH_3$ | $CH(CH_3)_2$ | m |
| $OCH_3$ | $OCH_3$ | $CH(CH_3)_2$ | p |
| $OCH_3$ | $OCH_3$ | $CH_2CH_2CH_2CH_3$ | m |
| $OCH_3$ | $OCH_3$ | $CH_2CH_2CH_2CH_3$ | p |
| $OCH_3$ | $OCH_3$ | $CH_2CH(CH_3)_2$ | o |
| $OCH_3$ | $OCH_3$ | $CH_2CH(CH_3)_2$ | m |
| $OCH_3$ | $OCH_3$ | $CH_2CH(CH_3)_2$ | p |
| $OCH_3$ | $OCH_3$ | $CH(CH_3)CH_2CH_3$ | o |
| $OCH_3$ | $OCH_3$ | $CH(CH_3)CH_2CH_3$ | m |
| $OCH_3$ | $OCH_3$ | $CH(CH_3)CH_2CH_3$ | p |
| $OCH_3$ | $OCH_3$ | $C(CH_3)_3$ | o |
| $OCH_3$ | $OCH_3$ | $C(CH_3)_3$ | m |
| $OCH_3$ | $OCH_3$ | $C(CH_3)_3$ | p |

When in the process (a), if, for example, 2-(4,6-dimethoxy-2-pyrimidinyloxy)-3-methyl-butyraldehyde and sodium borohydride are used as starting materials, the course of the reaction can be represented by the following equation:

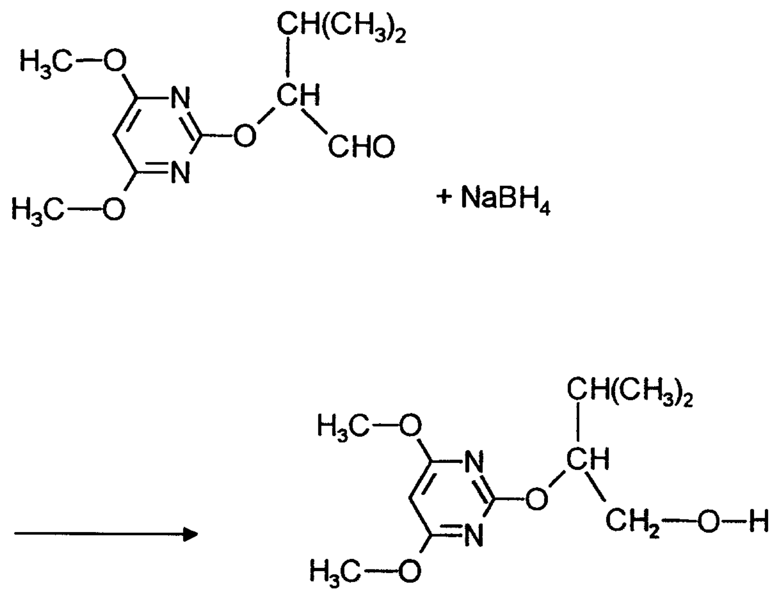


When in the process (b), if, for example, 2-(4,6-dimethoxy-2-pyrimidinyloxy)-3-methylbutanol and benzoylchloride are used as starting materials, the course of the reaction can be represented by the following equation:

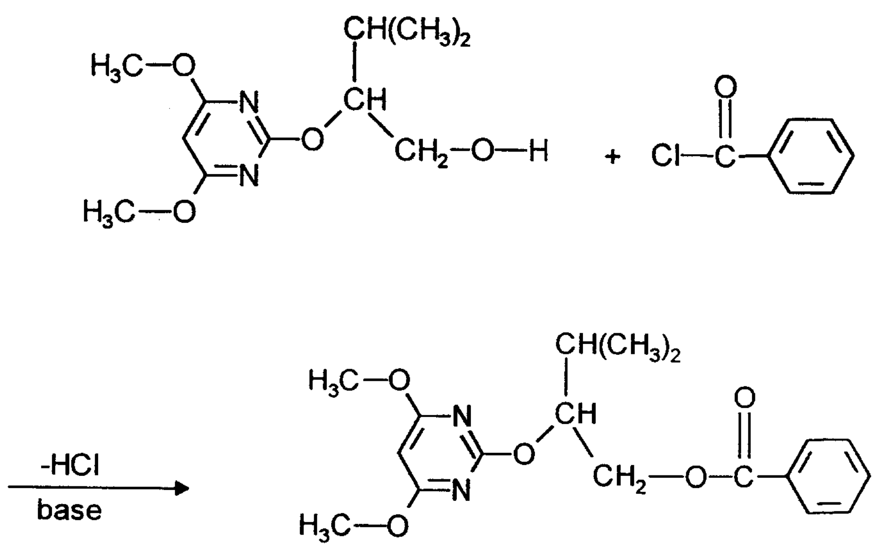


When in the process (c), if, for example, 2-(4,6-dimethoxy-2-pyrimidinyloxy)-3-methylbutanol and acetic anhydride are used as staring materials, the course of the reaction can be represented by the following equation:

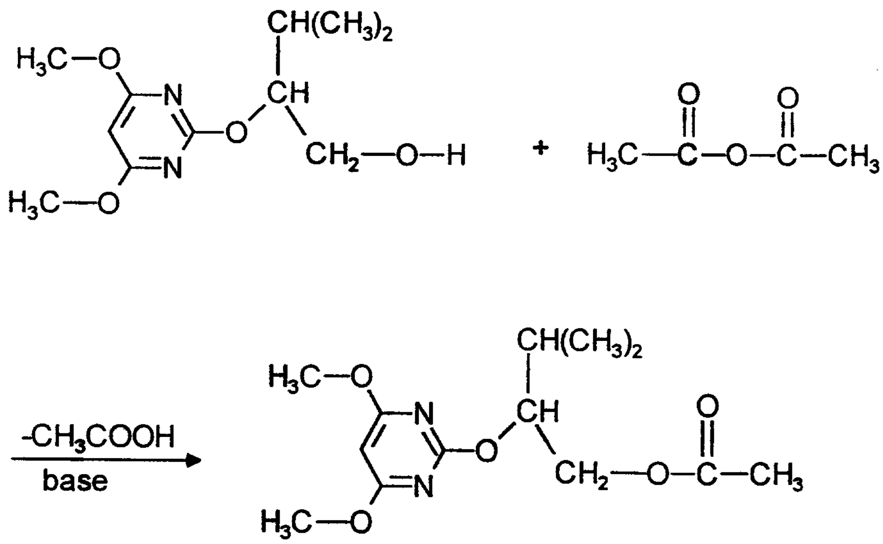


In the processes (a), the starting compounds of the formula (II) mean compounds based on the above definitions of $R_1$, $R_2$ $R_3$ and A in connection with the description of the compounds of the formula (I) according to the invention.

Preferred compounds of the formula (II) are those, wherein

$R_1$ represents methoxy, trifluoromethoxy or trifluoromethoxy,

$R_2$ represents methoxy, difluoromethoxy or trifluoromethoxy,

$R_3$ represents $C_{2-5}$ alkyl, and

A represents -CH=.

Particularly preferred compounds of the formula (II) are those, wherein

$R_1$ represents methoxy,

$R_2$ represents methoxy,

$R_3$ represents $C_{2-4}$ alkyl, and

A represents -CH=.

As very particularly preferred examples of the compounds represented by the formula (II) may be mentioned the compounds shown in the following Table:

Table 4

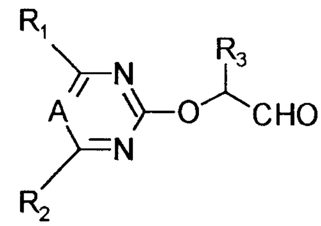

(II)

| A | $R_1$ | $R_2$ | $R_3$ |
|---|---|---|---|
| -CH= | $OCH_3$ | $OCH_3$ | $C_2H_5$ |
| -CH= | $OCH_3$ | $OCH_3$ | $CH_2CH_2CH_3$ |
| -CH= | $OCH_3$ | $OCH_3$ | $CH(CH_3)_2$ |
| -CH= | $OCH_3$ | $OCH_3$ | $CH_2CH_2CH_2CH_3$ |
| -CH= | $OCH_3$ | $OCH_3$ | $CH_2CH(CH_3)_2$ |
| -CH= | $OCH_3$ | $OCH_3$ | $CH(CH_3)CH_2CH_3$ |
| -CH= | $OCH_3$ | $OCH_3$ | $C(CH_3)_3$ |
| -CH= | $OCH_3$ | $OCH_3$ | $CH_2CH_2CH_2CH_2CH_3$ |
| -CH= | $OCH_3$ | $OCH_3$ | $CH(CH_3CH_2CH_2CH_3$ |
| -CH= | $OCH_3$ | $OCH_3$ | $CH_2CH_2CH(CH_3)_2$ |
| -CH= | $OCH_3$ | $OCH_3$ | $CH(C_2H_5)_2$ |
| -CH= | $OCH_3$ | $OCH_3$ | $C(CH_3)_2CH_2CH_3$ |
| -CH= | $OCH_3$ | $OCH_3$ | $CH_2C(CH_3)_3$ |
| -N= | $OCH_3$ | $OCH_3$ | $C_2H_5$ |
| -N= | $OCH_3$ | $OCH_3$ | $CH_2CH_2CH_3$ |
| -N= | $OCH_3$ | $OCH_3$ | $CH(CH_3)_2$ |
| -N= | $OCH_3$ | $OCH_3$ | $CH_2CH_2CH_2CH_3$ |
| -N= | $OCH_3$ | $OCH_3$ | $CH_2CH(CH_3)_2$ |
| -N= | $OCH_3$ | $OCH_3$ | $CH(CH_3)CH_2CH_3$ |
| -N= | $OCH_3$ | $OCH_3$ | $C(CH_3)_3$ |
| -N= | $OCH_3$ | $OCH_3$ | $CH_2CH_2CH_2CH_2CH_3$ |
| -N= | $OCH_3$ | $OCH_3$ | $CH(CH_3)CH_2CH_2CH_3$ |
| -N= | $OCH_3$ | $OCH_3$ | $CH_2CH_2CH(CH_3)_2$ |
| -N= | $OCH_3$ | $OCH_3$ | $CH(C_2H_5)_2$ |
| -N= | $OCH_3$ | $OCH_3$ | $C(CH_3)_2CH_2CH_3$ |
| -N= | $OCH_3$ | $OCH_3$ | $CH_2C(CH_3)_3$ |

The compounds of the formula (II) may be covered by the technical concept described in Japanese Laid-open Patent Application No. 194529/1992, but they are still novel as having not yet been concretely disclosed therein. And the compounds represented by the general formula (II) also exhibit an excellent herbicidal activity.

The compounds of the formula (II) can be obtained by the following process.

(d) A reaction of ozone-oxidation of a compound represented by the formula (III)

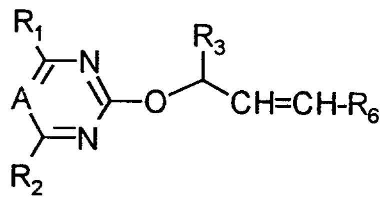

(III)

wherein

$R_1$, $R_2$, $R_3$ and A have the same meanings as mentioned above in connection with the description of the compounds of the formula (I) according to the invention and

$R_6$ represents hydrogen, optionally substituted $C_{1-4}$ alkyl, optionally substituted phenyl, an optionally substituted benzyl or an optionally substituted heterocyclic group.

Preferred compounds of formula (III) are those, wherein

$R_1$ represents methoxy, difluoromethoxy or trifluoromethoxy,

$R_2$ represents methoxy, difluoromethoxy or trifluoromethoxy,

$R_3$ represents $C_{2-5}$ alkyl,

A represents -CH= and

$R_6$ represents hydrogen an optionally substitutable $C_{1-3}$ alkyl (the substituent(s) may be selected from the group consisting of halogen, nitro, cyano, $C_{1-4}$ alkoxy and $C_{1-4}$ haloalkoxy), an optionally substitutable phenyl (the substituent(s) may be selected from the group consisting of halogen, nitro, cyano, $C_{1-4}$ alkyl, $C_{1-4}$ alkoxy, $C_{1-4}$ haloalkyl and $C_{1-4}$ haloalkoxy), optionally substitutable benzyl (the substituent(s) may be selected from the group consisting of halogen, nitro, cyano, $C_{1-4}$ alkyl, $C_{1-4}$ alkoxy, $C_{1-4}$ haloalkyl or $C_{1-4}$ haloalkoxy) or an optionally substitutable five- or six-membered heterocyclic group (the substituent(s) may be selected from the group consisting of halogen, nitro, cyano, $C_{1-4}$ alkyl, $C_{1-4}$ alkoxy, $C_{1-4}$ haloalkyl and $C_{1-4}$ haloalkoxy).

Particularly preferred compounds of formula (III) are those, wherein

$R_1$ represents methoxy,

$R_2$ represents methoxy,

$R_3$ represents $C_{2-4}$ alkyl,

A represents -CH= and

$R_6$ represents hydrogen, methyl, ethyl, propyl, isopropyl, an optionally substitutable phenyl (the substituent(s) may be selected from the group consisting of fluoro, chloro, bromo, nitro, cyano, methyl, ethyl, methoxy, ethoxy, trifluoromethyl and trifluoromethoxy),
optionally substitutable benzyl (the substituent(s) may be selected from the group consisting of fluoro, chloro, bromo, nitro, cyano, methyl, ethyl, methoxy, ethoxy, trifluoromethyl and trifluoromethoxy) or optionally substitutable five- or six-membered heterocyclic group (the substituent(s) may be selected from the group consisting of fluoro, chloro, bromo, nitro, cyano, methyl, ethyl, methoxy, ethoxy, trifluoromethyl and trifluoromethoxy).

In the process (d), the starting compounds of the formula (III) are also novel compounds and exhibit excellent herbicidal activities.

The compounds represented by the formula (III) can be obtained by the following process:

(e) in the case where A is -N=:

the compounds of the formula (VII),

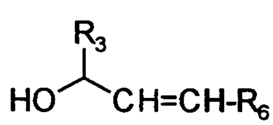

(VII)

wherein

$R_3$ and $R_6$ have the same meanings as mentioned above,

are reacted with compounds of the formula (VIII),

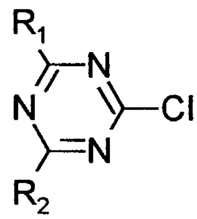

(VIII)

wherein

$R_1$ and $R_2$ have the same meanings as mentioned above,

in the presence of inert solvents and, if appropriate, in the presence of an acid binder,

or

(f) in the case where A is -CH=:

the above mentioned compounds of the formula (VII) are reacted with compounds of the formula (IX),

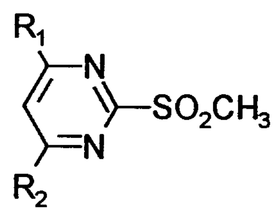

(IX)

wherein

$R_1$ and $R_2$ have the same meanings as mentioned above,

in the presence of inert solvents and, if appropriate, in the presence of an acid binder,

As the compounds represented by the general formula (III) may be mentioned the compounds shown in the following Tables 5 and 6:

Table 5

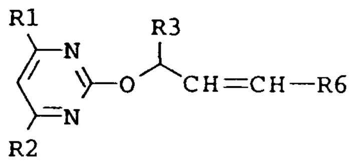

| R1 | R2 | R3 | R6 |
|---|---|---|---|
| $OCH_3$ | $OCH_3$ | $C_2H_5$ | H |
| $OCH_3$ | $OCH_3$ | $CH_2CH_2CH_3$ | H |
| $OCH_3$ | $OCH_3$ | $CH(CH_3)_2$ | H |
| $OCH_3$ | $OCH_3$ | $CH_2CH_2CH_2CH_3$ | H |
| $OCH_3$ | $OCH_3$ | $CH(CH_3)CH_2CH_3$ | H |
| $OCH_3$ | $OCH_3$ | $CH_2CH(CH_3)_2$ | H |
| $OCH_3$ | $OCH_3$ | $C(CH_3)_3$ | H |
| $OCH_3$ | $OCH_3$ | $(CH_2)_4CH_3$ | H |
| $OCH_3$ | $OCH_3$ | $CH(CH_3)CH_2CH_2CH_3$ | H |
| $OCH_3$ | $OCH_3$ | $CH(C_2H_5)_2$ | H |
| $OCH_3$ | $OCH_3$ | $C(CH_3)_2CH_2CH_3$ | H |
| $OCH_3$ | $OCH_3$ | $CH_2C(CH_3)_3$ | H |
| $OCH_3$ | $OCH_3$ | $CH_2CH_2CH(CH_3)_2$ | H |
| $OCH_3$ | $OCH_3$ | $C_2H_5$ | $CH_3$ |
| $OCH_3$ | $OCH_3$ | $C_2H_5$ | $C_2H_5$ |
| $OCH_3$ | $OCH_3$ | $C_2H_5$ | $CH_2CH_2CH_3$ |
| $OCH_3$ | $OCH_3$ | $C_2H_5$ | $CH(CH_3)_2$ |
| $OCH_3$ | $OCH_3$ | $C_2H_5$ | $CH_2CH_2CH_2CH_3$ |
| $OCH_3$ | $OCH_3$ | $C_2H_5$ | $CH(CH_3)CH_2CH_3$ |
| $OCH_3$ | $OCH_3$ | $C_2H_5$ | $CH_2CH(CH_3)_2$ |
| $OCH_3$ | $OCH_3$ | $C_2H_5$ | $C(CH_3)_3$ |

Table 5 (continued)

| R1 | R2 | R3 | R6 |
|---|---|---|---|
| $OCH_3$ | $OCH_3$ | $C_2H_5$ | 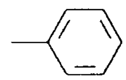 |
| $OCH_3$ | $OCH_3$ | $C_2H_5$ | 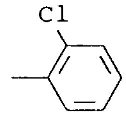 |
| $OCH_3$ | $OCH_3$ | $C_2H_5$ | 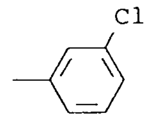 |
| $OCH_3$ | $OCH_3$ | $C_2H_5$ | 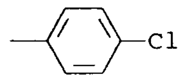 |
| $OCH_3$ | $OCH_3$ | $C_2H_5$ | 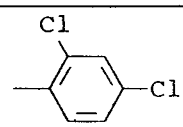 |
| $OCH_3$ | $OCH_3$ | $C_2H_5$ | 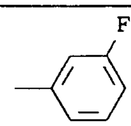 |
| $OCH_3$ | $OCH_3$ | $C_2H_5$ | 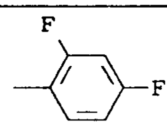 |
| $OCH_3$ | $OCH_3$ | $C_2H_5$ | 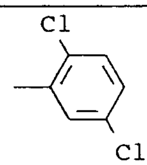 |
| $OCH_3$ | $OCH_3$ | $C_2H_5$ | 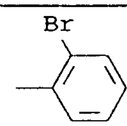 |
| $OCH_3$ | $OCH_3$ | $C_2H_5$ | 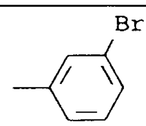 |
| $OCH_3$ | $OCH_3$ | $C_2H_5$ | 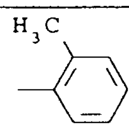 |
| $OCH_3$ | $OCH_3$ | $C_2H_5$ | 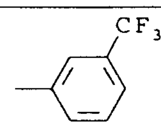 |

Table 5 (continued)

| R1 | R2 | R3 | R6 |
|---|---|---|---|
| $OCH_3$ | $OCH_3$ | $C_2H_5$ | 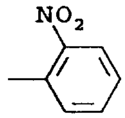 |
| $OCH_3$ | $OCH_3$ | $C_2H_5$ | 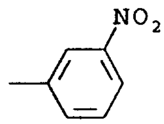 |
| $OCH_3$ | $OCH_3$ | $C_2H_5$ | 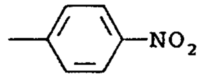 |
| $OCH_3$ | $OCH_3$ | $C_2H_5$ | 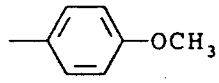 |
| $OCH_3$ | $OCH_3$ | $C_2H_5$ | 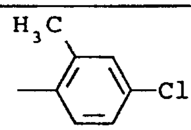 |
| $OCH_3$ | $OCH_3$ | $CH_2CH_2CH_3$ | $CH_3$ |
| $OCH_3$ | $OCH_3$ | $CH_2CH_2CH_3$ | $C_2H_5$ |
| $OCH_3$ | $OCH_3$ | $CH_2CH_2CH_3$ | $CH_2CH_2CH_3$ |
| $OCH_3$ | $OCH_3$ | $CH_2CH_2CH_3$ | $CH(CH_3)_2$ |
| $OCH_3$ | $OCH_3$ | $CH_2CH_2CH_3$ | $CH_2CH_2CH_2CH_3$ |
| $OCH_3$ | $OCH_3$ | $CH_2CH_2CH_3$ | $CH(CH_3)CH_2CH_3$ |
| $OCH_3$ | $OCH_3$ | $CH_2CH_2CH_3$ | $CH_2CH(CH_3)_2$ |
| $OCH_3$ | $OCH_3$ | $CH_2CH_2CH_3$ | $C(CH_3)_3$ |
| $OCH_3$ | $OCH_3$ | $CH_2CH_2CH_3$ | 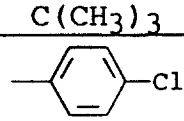 |
| $OCH_3$ | $OCH_3$ | $CH_2CH_2CH_3$ | 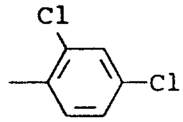 |
| $OCH_3$ | $OCH_3$ | $CH_2CH_2CH_3$ | 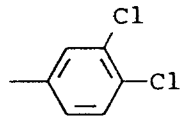 |
| $OCH_3$ | $OCH_3$ | $CH_2CH_2CH_3$ | 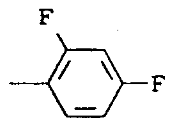 |

Table 5 (continued)

| | | | |
|---|---|---|---|
| $OCH_3$ | $OCH_3$ | $CH_2CH_2CH_3$ | 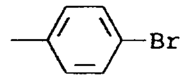 |
| $OCH_3$ | $OCH_3$ | $CH_2CH_2CH_3$ | 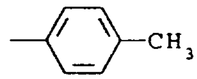 |
| $OCH_3$ | $OCH_3$ | $CH_2CH_2CH_3$ | 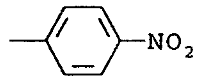 |
| $OCH_3$ | $OCH_3$ | $CH_2CH_2CH_3$ | 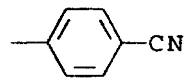 |
| $OCH_3$ | Cl | $CH(CH_3)_2$ | $CH_3$ |
| $OCH_3$ | Cl | $C(CH_3)_3$ | H |
| $OCF_3$ | $OCF_3$ | $CH(CH_3)_2$ | $C(CH_3)_3$ |
| $OCF_3$ | $OCF_3$ | $C(CH_3)_3$ | $CH_3$ |
| $OCHF_2$ | $OCHF_2$ | $CH(CH_3)_2$ | 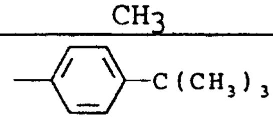 |
| $OCH_3$ | $OCH_3$ | $CH(CH_3)_2$ | $C_2H_5$ |
| $OCH_3$ | $OCH_3$ | $CH(CH_3)_2$ | $CH_2CH_2CH_3$ |
| $OCH_3$ | $OCH_3$ | $CH(CH_3)_2$ | $CH(CH_3)_2$ |
| $OCH_3$ | $OCH_3$ | $CH(CH_3)_2$ | $CH_2CH_2CH_2CH_3$ |
| $OCH_3$ | $OCH_3$ | $CH(CH_3)_2$ | $CH(CH_3)CH_2CH_3$ |
| $OCH_3$ | $OCH_3$ | $CH(CH_3)_2$ | $CH_2CH(CH_3)_2$ |
| $OCH_3$ | $OCH_3$ | $CH(CH_3)_2$ | $C(CH_3)_3$ |
| $OCH_3$ | $OCH_3$ | $CH(CH_3)_2$ | 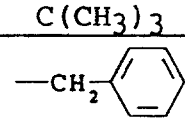 |
| $OCH_3$ | $OCH_3$ | $CH(CH_3)_2$ | 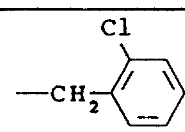 |
| $OCH_3$ | $OCH_3$ | $CH(CH_3)_2$ | 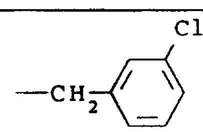 |
| $OCH_3$ | $OCH_3$ | $CH(CH_3)_2$ | 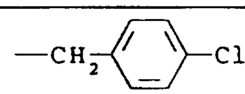 |

Table 5

| R1 | R2 | R3 | R6 |
|---|---|---|---|
| $OCH_3$ | $OCH_3$ | $CH(CH_3)_2$ | 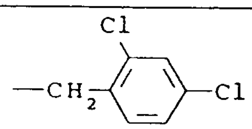 |
| $OCH_3$ | $OCH_3$ | $CH(CH_3)_2$ | 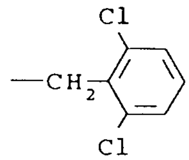 |
| $OCH_3$ | $OCH_3$ | $CH(CH_3)_2$ | 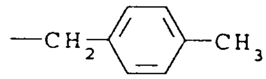 |
| $OCH_3$ | $OCH_3$ | $CH(CH_3)_2$ | 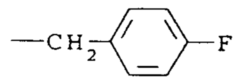 |
| $OCH_3$ | $OCH_3$ | $CH(CH_3)_2$ | 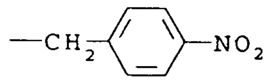 |
| $OCH_3$ | $OCH_3$ | $CH(CH_3)_2$ | 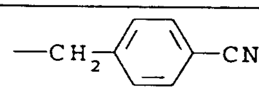 |
| $OCH_3$ | $OCH_3$ | $CH(CH_3)_2$ | 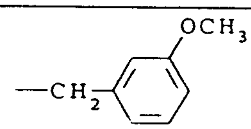 |
| $OCH_3$ | $OCH_3$ | $CH(CH_3)_2$ | 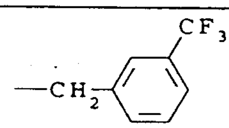 |
| $OCH_3$ | $OCH_3$ | $CH(CH_3)_2$ | 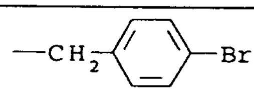 |
| $OCH_3$ | $OCH_3$ | $CH(CH_3)_2$ | 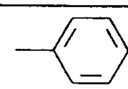 |
| $OCH_3$ | $OCH_3$ | $CH(CH_3)_2$ | 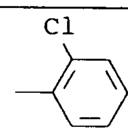 |
| $OCH_3$ | $OCH_3$ | $CH(CH_3)_2$ | 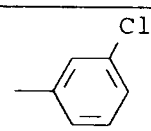 |

Table 5

| R1 | R2 | R3 | R6 |
|---|---|---|---|
| $OCH_3$ | $OCH_3$ | $CH(CH_3)_2$ | 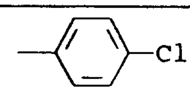 |
| $OCH_3$ | $OCH_3$ | $CH(CH_3)_2$ | 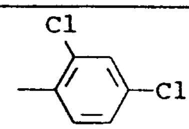 |
| $OCH_3$ | $OCH_3$ | $CH(CH_3)_2$ | 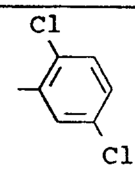 |
| $OCH_3$ | $OCH_3$ | $CH(CH_3)_2$ | 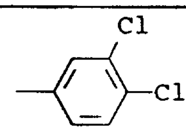 |
| $OCH_3$ | $OCH_3$ | $CH(CH_3)_2$ | 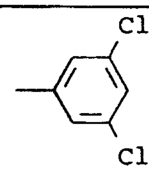 |
| $OCH_3$ | $OCH_3$ | $CH(CH_3)_2$ | 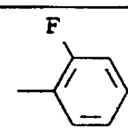 |
| $OCH_3$ | $OCH_3$ | $CH(CH_3)_2$ | 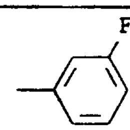 |
| $OCH_3$ | $OCH_3$ | $CH(CH_3)_2$ | 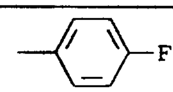 |
| $OCH_3$ | $OCH_3$ | $CH(CH_3)_2$ | 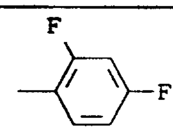 |
| $OCH_3$ | $OCH_3$ | $CH(CH_3)_2$ | 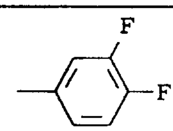 |
| $OCH_3$ | $OCH_3$ | $CH(CH_3)_2$ | 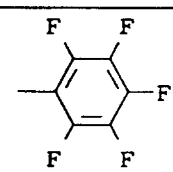 |

Table 5 (continued)

| R1 | R2 | R3 | R6 |
|---|---|---|---|
| $OCH_3$ | $OCH_3$ | $CH(CH_3)_2$ | 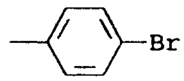 |
| $OCH_3$ | $OCH_3$ | $CH(CH_3)_2$ | 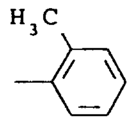 |
| $OCH_3$ | $OCH_3$ | $CH(CH_3)_2$ | 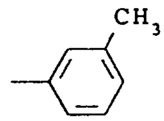 |
| $OCH_3$ | $OCH_3$ | $CH(CH_3)_2$ | 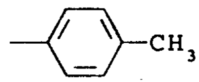 |
| $OCH_3$ | $OCH_3$ | $CH(CH_3)_2$ | 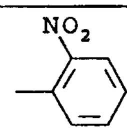 |
| $OCH_3$ | $OCH_3$ | $CH(CH_3)_2$ | 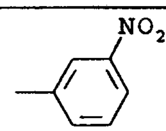 |
| $OCH_3$ | $OCH_3$ | $CH(CH_3)_2$ | 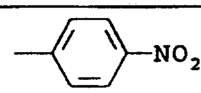 |
| $OCH_3$ | $OCH_3$ | $CH(CH_3)_2$ | 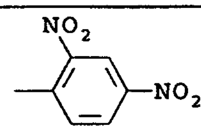 |
| $OCH_3$ | $OCH_3$ | $CH(CH_3)_2$ | 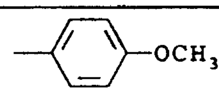 |
| $OCH_3$ | $OCH_3$ | $CH(CH_3)_2$ | 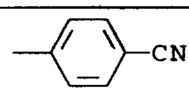 |
| $OCH_3$ | $OCH_3$ | $CH_2CH_2CH_2CH_3$ | $CH_2CH_2CH_3$ |
| $OCH_3$ | $OCH_3$ | $CH_2CH_2CH_2CH_3$ | $CH(CH_3)_2$ |
| $OCH_3$ | $OCH_3$ | $CH_2CH_2CH_2CH_3$ | $CH_2CH_2CH_2CH_3$ |
| $OCH_3$ | $OCH_3$ | $CH_2CH_2CH_2CH_3$ | $CH(CH_3)CH_2CH_3$ |
| $OCH_3$ | $OCH_3$ | $CH_2CH_2CH_2CH_3$ | $C(CH_3)_3$ |
| $OCH_3$ | $OCH_3$ | $CH_2CH_2CH_2CH_3$ | 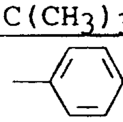 |

Table 5 (continued)

| R1 | R2 | R3 | R6 |
|---|---|---|---|
| $OCH_3$ | $OCH_3$ | $CH_2CH_2CH_2CH_3$ | 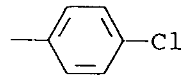 |
| $OCH_3$ | $OCH_3$ | $CH_2CH_2CH_2CH_3$ | 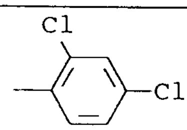 |
| $OCH_3$ | $OCH_3$ | $CH_2CH_2CH_2CH_3$ | 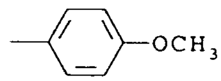 |
| $OCH_3$ | $OCH_3$ | $CH_2CH_2CH_2CH_3$ | 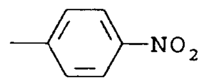 |
| $OCH_3$ | $OCH_3$ | $CH_2CH_2CH_2CH_3$ | 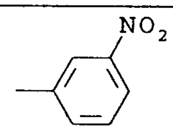 |
| $OCH_3$ | $OCH_3$ | $CH_2CH(CH_3)_2$ | $C_2H_5$ |
| $OCH_3$ | $OCH_3$ | $CH(CH_3)CH_2CH_3$ | $CH(CH_3)_2$ |
| $OCH_3$ | $OCH_3$ | $CH(CH_3)CH_2CH_3$ | $CH_2CH_2CH_2CH_3$ |
| $OCH_3$ | $OCH_3$ | $CH_2CH_2CH_2CH_3$ | $CH(CH_3)CH_2CH_3$ |
| $OCH_3$ | $OCH_3$ | $CH_2CH_2CH_2CH_3$ | $C(CH_3)_3$ |
| $OCH_3$ | $OCH_3$ | $CH_2CH(CH_3)_2$ | 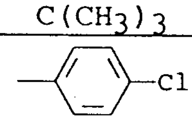 |
| $OCH_3$ | $OCH_3$ | $CH(CH_3)CH_2CH_3$ | 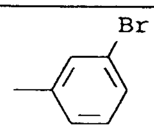 |
| $OCH_3$ | $OCH_3$ | $CH_2CH(CH_3)_2$ | 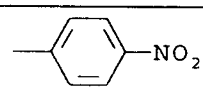 |
| $OCH_3$ | $OCH_3$ | $C(CH_3)_3$ | $CH_3$ |
| $OCH_3$ | $OCH_3$ | $C(CH_3)_3$ | $C_2H_5$ |
| $OCH_3$ | $OCH_3$ | $C(CH_3)_3$ | $CH_2CH_2CH_3$ |
| $OCH_3$ | $OCH_3$ | $C(CH_3)_3$ | $CH(CH_3)_2$ |
| $OCH_3$ | $OCH_3$ | $C(CH_3)_3$ | $CH_2CH_2CH_2CH_3$ |
| $OCH_3$ | $OCH_3$ | $C(CH_3)_3$ | $CH(CH_3)CH_2CH_3$ |

Table 5 (continued)

| R1 | R2 | R3 | R6 |
|---|---|---|---|
| $OCH_3$ | $OCH_3$ | $C(CH_3)_3$ | $CH_2CH(CH_3)_2$ |
| $OCH_3$ | $OCH_3$ | $C(CH_3)_3$ | $C(CH_3)_3$ |
| $OCH_3$ | $OCH_3$ | $C(CH_3)_3$ | 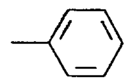 |
| $OCH_3$ | $OCH_3$ | $C(CH_3)_3$ | 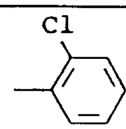 |
| $OCH_3$ | $OCH_3$ | $C(CH_3)_3$ | 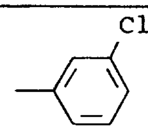 |
| $OCH_3$ | $OCH_3$ | $C(CH_3)_3$ | 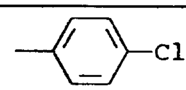 |
| $OCH_3$ | $OCH_3$ | $C(CH_3)_3$ | 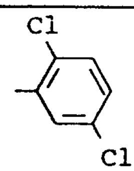 |
| $OCH_3$ | $OCH_3$ | $C(CH_3)_3$ | 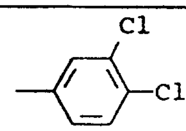 |
| $OCH_3$ | $OCH_3$ | $C(CH_3)_3$ | 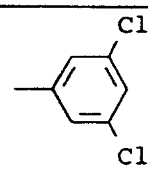 |
| $OCH_3$ | $OCH_3$ | $C(CH_3)_3$ | 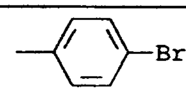 |
| $OCH_3$ | $OCH_3$ | $C(CH_3)_3$ | 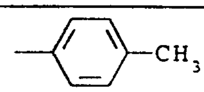 |
| $OCH_3$ | $OCH_3$ | $C(CH_3)_3$ | 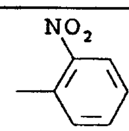 |
| $OCH_3$ | $OCH_3$ | $C(CH_3)_3$ | 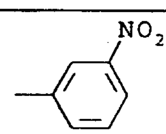 |

Table 5 (continued)

| R1 | R2 | R3 | R6 |
|---|---|---|---|
| $OCH_3$ | $OCH_3$ | $C(CH_3)_3$ | 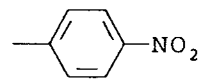 |
| $OCH_3$ | $OCH_3$ | $C(CH_3)_3$ | 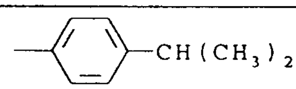 |
| $OCH_3$ | $OCH_3$ | $C(CH_3)_3$ | 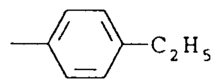 |
| $OCH_3$ | $OCH_3$ | $C(CH_3)_3$ | 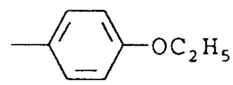 |
| $OCH_3$ | $OCH_3$ | $C(CH_3)_3$ | 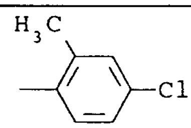 |
| $OCH_3$ | $OCH_3$ | $(CH_2)_4CH_3$ | $CH_3$ |
| $OCH_3$ | $OCH_3$ | $(CH_2)_4CH_3$ | $C_2H_5$ |
| $OCH_3$ | $OCH_3$ | $(CH_2)_4CH_3$ | $CH_2CH_2CH_3$ |
| $OCH_3$ | $OCH_3$ | $(CH_2)_4CH_3$ | $CH(CH_3)_2$ |
| $OCH_3$ | $OCH_3$ | $(CH_2)_4CH_3$ | $CH_2CH_2CH_2CH_3$ |
| $OCH_3$ | $OCH_3$ | $(CH_2)_4CH_3$ | $CH(CH_3)CH_2CH_3$ |
| $OCH_3$ | $OCH_3$ | $(CH_2)_4CH_3$ | $CH_2CH(CH_3)_2$ |
| $OCH_3$ | $OCH_3$ | $(CH_2)_4CH_3$ | $C(CH_3)_3$ |
| $OCH_3$ | $OCH_3$ | $(CH_2)_4CH_3$ | 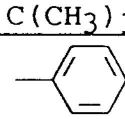 |
| $OCH_3$ | $OCH_3$ | $(CH_2)_4CH_3$ | 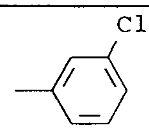 |
| $OCH_3$ | $OCH_3$ | $(CH_2)_4CH_3$ | 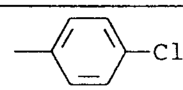 |
| $OCH_3$ | $OCH_3$ | $(CH_2)_4CH_3$ | 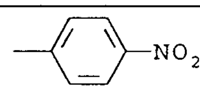 |
| $OCH_3$ | $OCH_3$ | $(CH_2)_4CH_3$ | 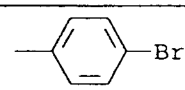 |
| $OCH_3$ | $OCH_3$ | $(CH_2)_4CH_3$ | 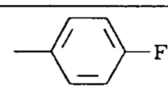 |

Table 5 (continued)

| R1 | R2 | R3 | R6 |
|---|---|---|---|
| $OCH_3$ | $OCH_3$ | $CH(C_2H_5)_2$ | $CH_3$ |
| $OCH_3$ | $OCH_3$ | $CH(C_2H_5)_2$ | $C(CH_3)_3$ |
| $OCH_3$ | $OCH_3$ | $CH(C_2H_5)_2$ | 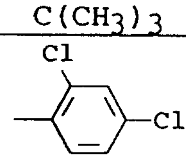 |
| $OCH_3$ | $OCH_3$ | $CH(C_2H_5)_2$ | 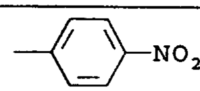 |
| $OCH_3$ | $OCH_3$ | $CH_2CH_2CH_2CH_3$ | $CH_2CH(CH_3)_2$ |
| $OCH_3$ | $OCH_3$ | $CH_2CH_2CH_2CH_3$ | $CH_3$ |
| $OCH_3$ | $OCH_3$ | $C(CH_3)_3$ | 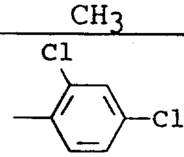 |
| $OCH_3$ | $OCH_3$ | $C(CH_3)_3$ | 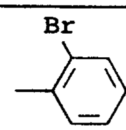 |
| $OCH_3$ | $OCH_3$ | $C(CH_3)_3$ | 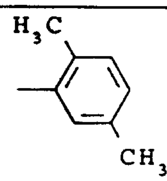 |
| $OCH_3$ | $OCH_3$ | $CH(CH_3)CH_2CH_2CH_3$ | $CH_3$ |
| $OCH_3$ | $OCH_3$ | $CH_2CH_2CH(CH_2)_2$ | $C(CH_3)_3$ |
| $OCH_3$ | $OCH_3$ | $CH(CH_3)_2$ | H |
| $OCH_3$ | $OCH_3$ | $C(CH_3)_3$ | 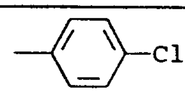 |
| $OCH_3$ | $OCH_3$ | $CH(CH_3)_2$ | $CH_3$ |
| $OCH_3$ | $OCH_3$ | $CH(CH_3)_2$ | 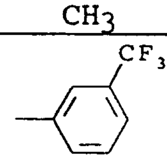 |

Table 5 (continued)

| $R_1$ | $R_2$ | $R_3$ | $R_6$ |
|---|---|---|---|
| $OCH_3$ | $OCH_3$ | $CH(CH_3)CH_2CH_3$ | 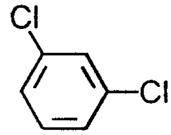 |
| $OCH_3$ | $OCH_3$ | $C(CH_3)_3$ | 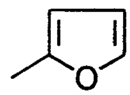 |

Table 6

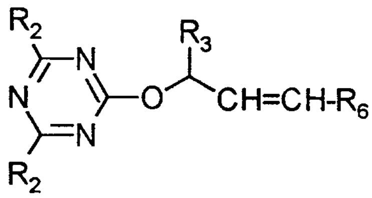

| $R_1$ | $R_2$ | $R_3$ | $R_6$ |
|---|---|---|---|
| $OCH_3$ | $OCH_3$ | $C_2H_5$ | H |
| $OCH_3$ | $OCH_3$ | $CH_2CH_2CH_3$ | H |
| $OCH_3$ | $OCH_3$ | $CH(CH_3)_2$ | H |
| $OCH_3$ | $OCH_3$ | $CH_2CH_2CH_2CH_3$ | H |
| $OCH_3$ | $OCH_3$ | $CH(CH_3)CH_2CH_3$ | H |
| $OCH_3$ | $OCH_3$ | $CH_2CH(CH_3)_2$ | H |
| $OCH_3$ | $OCH_3$ | $C(CH_3)_3$ | H |
| $OCH_3$ | $OCH_3$ | $(CH_2)_4CH_3$ | H |
| $OCH_3$ | $OCH_3$ | 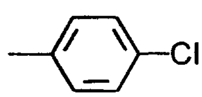 | H |

In the processes (e) and (f), the starting compounds of the formula (VII) mean compounds based on the above definitions of $R_3$ and $R_6$ and preferably substituents are based on the above preferred definitions of $R_3$ and $R_6$.

The starting compounds of the formula (VI) are well known in organic chemistry and may be exemplified by the following compounds:

1-(4-chlorophenyl)-4,4-dimethylpentene-3-ol,
1-phenyl-4-methylpentene-3-ol,
1-(4-bromophenyl)-4,4-dimethylpentene-3-ol,
1-(4-cyanophenyl)-4,4-dimethylpentene-3-ol,

1-(4-nitrophenyl)-4,4-dimethylpentene-3-ol, and
1-(2,4-dichlorophenyl)-4,4-dimethylpentene-3-ol.

In the process (e), the starting compounds of the formula (VIII) mean compounds based on the above definitions of $R_1$ and $R_2$ and preferred substituents are based on the above preferred definitions of $R_1$ and $R_2$.

The starting compounds of the formula (VIII) are well known in organic chemistry and may be exemplified by the following compounds:
2-chloro-4,6-dimethoxy-1,3,5-triazine, and
2-chloro-4-methoxy-6-methyl-1,3,5-triazine.

In the process (f), the starting compounds of the formula (IX) mean compounds based on the above definitions of $R_1$ and $R_2$, and preferred substituents are based on the above preferred definitions of $R_1$ and $R_2$.

The starting compounds of the formula (IX) are well known in organic chemistry and may be exemplified by the following compounds:
2-methanesulfonyl-4,6-dimethoxy-pyrimidine, and
2-methanesulfonyl-4-methoxy-6-methyl-pyrimidine.

In the process (a), the reducing agents employed therein can be exemplified by the following compounds:
Sodium borohydride and lithium aluminum hydride.

In the process (b) and (c) mentioned above, the starting compounds represented by the formula (IV) mean compounds based on the above definitions of $R_1$, $R_2$ and $R_3$ and preferred substituents are based on the above preferred definitions of $R_1$, $R_2$ and $R_3$.

The compounds represented by the formula (IV) are the compounds according to the invention which can be obtained according to the process (a).

In the process (b), the acid halide represented by the formula (V), mean compounds based on the above definitions of $R_5$, and $R_7$ and preferred substituents of $R_5$ are based on the above preferred definitions of $R_5$, and $R_7$ preferably represents bromide.

The acid halide represented by the formula (V) may be exemplified by one of the following compounds:
benzoyl chloride or bromide,
cinnamyl chloride or bromide,
propionyl chloride or bromide,
nicotinoyl chloride or bromide,
p-chlorobenzoyl chloride or bromide and
chloroacetyl chloride or bromide.

In the process (c), acid anhydrides, the starting compounds of the formula (VI) mean compounds based on the above definitions of $R_5$ and preferred substituents are based on the above preferred definitions of $R_5$.

The starting compounds of the formula (VI) are well known in organic chemistry and may be exemplified by the following compounds:
acetic anhydride,
propionic anhydride, and
acetic formic anhydride.

In carrying out the process (a) mentioned above, use may be made, as suitable diluent of any inert solvent.

Examples of such diluents are aliphatic, cycloaliphatic and aromatic, optionally chlorinated, hydrocarbons such as pentane, hexane, cyclohexane, petroleum ether, ligroin, benzene, toluene, xylene, dichloromethane, chloroform, tetrachloromethane, 1,2-dichloroethane, chlorobenzene, dichlorobenzene and the like; ethers such as diethyl ether, methyl ethyl ether, diisopropyl ether, dibutyl ether, dioxane, dimethoxyethane (DME), tetrahydrofurane (THF) and the like; ketones such as acetone, methylethyl ketone (MEK), methyl-iso-propyl ketone, methyl-iso-butyl ketone (MIBK) and the like; nitriles such as acetonitrile, propionitrile and the like; esters such as ethyl acetate, amyl acetate and the like, acid amides such as dimethyl formamide (DMF), dimethyl acetamide (DMA), N-methylpyrrolidone, 1,3-dimethyl-2-imidazolidinone, hexamethylphosphoric triamide (HMPA) and the like; sulfones and sulfoxides such as dimethyl sulfoxide (DMSO), sulfolane and the like.

In the above mentioned process (a), the reaction temperature can be varied within a substantially wide range. In general, the reaction is carried out at temperatures between -78 °C and 120 °C, preferably at temperatures between -20 °C and 60 °C.

Further, the reaction is carried out under normal pressure, although it is also possible to carry out the process under elevated or reduced pressure.

When the above mentioned process (a) according to the present invention is carried out, use is made, for example, about 0.5 to 0.6 mols of a reducing agent in a diluent such as methanol, per 1 mol of the compounds represented by the general formula (II) to obtain the desired compounds.

In carrying out the process (b) as mentioned above, use may be made, as suitable diluent, of any inert solvent.

Examples of such diluents are aliphatic, cycloaliphatic and aromatic, optionally chlorinated, hydrocarbons such as pentane, hexane, cyclohexane, petroleum ether, ligroin, benzene, toluene, xylene, dichloromethane, chloroform, carbon tetrachloride, 1,2-dichloroethane, chlorobenzene, dichlorobenzene and the like; ethers such as diethyl ether, methyl ethyl ether, diisopropyl ether, dibutyl ether, dioxane, dimethoxyethane (DME), tetrahydrofurane (THF) and the like; nitriles such as acetonitrile, propionitrile and the like; esters such as ethyl acetate, amyl acetate and the like, acid amides such as dimethyl formamide (DMF), dimethyl acetamide (DMA), N-methyl-pyrrolidone, 1,3-dimethyl-2-imidazolidinone, hexamethylphosphoric triamide (HMPA) and the like; sulfones and sulfoxides such as dimethyl sulfoxide (DMSO), sulfolane and the like and bases such as pyridine.

The process (b) according to the invention is carried out preferably in the presence of an acid binder.

As example of such an acid binder may be mentioned:

inorganic bases including hydroxide, carbonate, bicarbonate such as, for example, sodium hydrogen carbonate, potassium hydrogen carbonate, sodium carbonate, potassium carbonate, and the like, inorganic alkali metal amide including lithium amide, sodium amide, potassium amide and the like, organic bases including tertiary amines, dialkylamino anilines and pyridines such as, for example, triethyl-amine, tributyl-amine, 1,1,4,4-tetra-methylenediamine (TMEDA), N,N-dimethylaniline, N,N-diethylaniline, pyridine, 4-dimethylaminopyridine (DMAP), 1,4-diazabicyclo[2,2,2]octane (DABCO), 1,8-diazabicyclo[5,4,0]-undec-7-ene (DBU) and the like; and organic lithiums including methyllithium, n-butyllithium, sec-butyllithium, tert-butyllithium, phenyllithium, dimethyl copper lithium, lithium diisopropylamide, lithium cyclohexylisopropylamide, lithium dicyclohexylamide, n-butyl-lithium• DABCO, n-butyllithium • TMEDA and the like.

In the above mentioned process (b), the reaction temperature can be varied within a substantially wide rage. In general, the reaction is carried out at temperatures between -40 °C ad 100 °C, preferably at temperatures between 0 °C and 60 °C.

Further, the reaction is carried out under normal pressure although it is also possible to carry out the process under elevated pressure.

When the above mentioned process (b) according to the present invention is carried out, use is made, for example, about 1 to 1.2 mols of the acid halide represented by the formula (V) in pyridine, per 1 mol of the compounds represented by the general formula (IV) to obtain the desired compounds.

In carrying out the process (c) mentioned above, use may be made, as suitable diluent, of any inert solvent.

Examples of such diluents are water; aliphatic, cycloaliphatic and aromatic, optionally chlorinated hydrocarbons such as pentane, hexane, cyclohexane, petroleum ether, ligroin, benzene, toluene, xylene, dichloromethane, chloroform, carbon tetrachloride, 1,2-dichloroethane, chlorobenzene, dichlorobenzene and the like; ethers such as diethyl ether, methyl ethyl ether, diisopropyl ether, dibutyl ether, dioxane, dimethoxyethane (DME), tetrahydrofurane (THF), diethylene glycol dimethylether (DGM), and the like; ketones; acetene, methylethyl ketone (MEK), methyl-isopropyl ketone, methyl-iso-butyl ketone, and the like; nitriles such as acetonitrile, propionitrile, acrylonitrile and the like; estaers such as ethyl acetate, amyl acetate and the like; acid amides such as dimethyl formamide (DMF), dimethyl acetamide (DMA), N-methylpyrrolidone, 1,3-dimethyl-2-imidazolidinone, hexamethylphosphoric triamide (HMPA) and the like; sulfones and sulfoxides such as dimethyl sulfoxide (DMSO), sulfolane and the like; and bases such as pyridine.

The process (c) according to the invention is carried out preferably in the presence of an acid binder. As example of such acid binder may be mentioned: inorganic bases including hydroxide, carbonate, bicarbonate, alcolate of alkali metals such as, for example, sodium hydrogen carbonate, potassium hydrogen carbonate, sodium carbonate, potassium carbonate, organic bases including tertiary amines, dialkylaminoanilines and pyridines such as, for example, triethylamine, tributyl-amine, 1,1,4,4-tetra-methylenediamine (TMEDA), N,N-di-methylaniline, N,N-diethylaniline, pyridine, 4-dimethyl-amino pyridine (DMAP), 1,4-diazabicyclo-[2,2,2]octane (DABCO), 1,8-diazabicyclo[5,4,0]-undec-7-ene (DBU), organic lithium compounds such as methyl lithium, n-butyl lithium, sec-butyl lithium, tert-butyl lithium, phenyl lithium, dimethyl copper lithium, lithium diisopropyl amide, lithium cyclohexylisopropyl amide, lithium dicyclohexyl amide, n-butyl lithium • DABCO, n-butyl lithium • DBU, n-butyl lithium • TMEDA and the like.

In the above mentioned process (c), the reaction temperature can be varied within a substantially wide range. In general, the reaction is carried out at temperatures between -10 °C and 120 °C, preferably at temperatures between 0 °C and 50 °C.

Further, the reaction is carried out under normal pressure, although it is also possible to carry out the process under elevated or reduced pressure.

When the above mentioned process (c) according to the present invention is carried out, use is made, for example, 1 to 1.2 mol of the compounds of the formulae (VI) in a diluent such as pyridine, per mol of the compounds of the formula (IV) to obtain the desired compounds.

The active compounds according to the invention can be used as defoliants, desiccants, agents for destroying broad-leaved plants and, especially, as weed killers.

By weeds, in the broadest sense, there are to be understood all plants which grow in locations where they are undesired. Whether the substances according to the invention act as total or selective herbicides depends essentially on the amounts used.

The active compounds according to the invention can be used, for example, in connection with the following plants:
Dicotyledon weeds of the genera: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver and Centaurea.

Dicotyledon cultures of the genera: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis and Cucurbita.

Monocotyledon weeds of the genera: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus and Apera.

Monocotyledon cultures of the cultures of the genera: Oryza, Zea, Triticum, Hordeum, Avena, Secale, Sorghum, Panicum, Saccharum, Ananas, Asparagus and Allium.

However, the use of the active compounds according to the invention is in no way restricted to these genera, but also extends in the same manner to other plants.

The compounds are suitable, depending on the concentration, for the total combating of weeds, for example on industrial terrain and rail tracks and on paths and squares with or without tree plantings. Equally, the compounds can be employed for combating weeds in perennial cultures, for example afforestations, decorative tree plantings, orchards, vineyards, citrus groves, nut orchards, banana plantations, coffee plantations, tea plantations, rubber plantations, oil palm plantations, cocoa plantations, soft fruit plantings and hop fields, and for the selective combating of weeds in annual cultures.

The active compounds can be converted into the customary formulations, such as solutions, emulsions, wettable powders, suspensions, powders, foams, pastes, granules, tablets, aerosols, natural and synthetic materials impregnated with active compound, very fine capsules in polymeric substances, coating compositions for use on seed, and formulations used with burning equipment, such as fumigating cartridges, fumigating cans and fumigating coils, as well as ULV cold mist and warm mist formulations.

These formulations may be produced in known manner, for example by mixing the active compounds with extenders, that is to say liquid or liquefied gaseous or solid diluents or carriers, optionally with the use of surface-active agents, that is to say emulsifying agents and/or dispersing agents and/or foam-forming agents. In the case of the use of water as an extender, organic solvents can, for example, also be used as auxiliary solvents.

As liquid solvents diluents or carriers, there are suitable in the main, aromatic hydrocarbons, such as xylene, toluene or alkyl naphthalenes, chlorinated aromatic or chlorinated aliphatic hydrocarbons, such as chlorobenzenes, chloroethylenes or methylene chloride, aliphatic hydrocarbons, such as cyclohexane or paraffins, for example mineral oil fractions, alcohols, such as butanol or glycol as well as their ethers and esters, ketones, such as acetone, methyl ethyl ketone, methyl isobutyl ketone or cyclohexanone, or strongly polar solvents, such as dimethylformamide and dimethylsulphoxide, as well as water.

By liquefied gaseous diluents or carriers are meant liquids which would be gaseous at normal temperatures and under normal pressure, for example aerosol propellants, such as halogenated hydrocarbons as well as butane, propane, nitrogen and carbon dioxide.

As solid carriers there may be used ground natural minerals, such as kaolins clays, talc, chalk, quartz, attapulgite, montmorillonite or diatomaceous earth, and ground synthetic minerals, such as highly-dispersed silicic acid, alumina and silicates. As solid carriers for granules there may be used crushed and fractionated natural rocks such as calcite, marble, pumice, sepiolite and dolomite, as well as synthetic granules of

inorganic and organic meals, and granules of organic material such as sawdust, coconut shells, maize cobs and tobacco stalks.

As emulsifying and/or foam-forming agents there may be used non-ionic and anionic emulsifiers, such as polyoxyethylene-acid esters, polyoxyethylene-alcoholethers, for example alkylaryl polyglycol ethers, alkyl sulphonates, alkyl sulphates, aryl sulphonates as well as albumin hydrolysis products.

Dispersing agents include, for example, lignin sulphite waste liquors and methylcellulose.

Adhesives such as carboxymethylcellulose and natural and synthetic polymers in the form of powders, granules or latices, such as gum arabic, polyvinyl alcohol and polyvinyl acetate, can be used in the formulation.

It is possible to use colorants such as inorganic pigments, for example iron oxide, titanium oxide and Prussian Blue and organic dyestuffs, such as alizarin dyestuffs, azo dyestuffs or metal phthalocyanine dyestuffs, and trace nutrients, such as salts of iron, manganese boron, copper, cobalt, molybdenum and zinc.

The formulations in general contain from 0.1 to 95 per cent by weight of active compound, preferably from 0.5 to 90 per cent by weight.

The active compounds according to the invention, as such or in the form of their formulations, can also be used, for combating weeds, as mixtures with known herbicides, finished formulations or tank mixes being possible.

Mixtures with other known active compounds, such as herbicides, fungicides, insecticides, acaricides, nematicides, bird repellents, plant nutrients and agents which improve soil structure, are also possible.

The active compounds can be used as such, in the form of their formulations or in the use forms prepared therefrom by further dilution, such as ready-to-use solutions, suspensions, emulsions, powders, pastes and granules.

They are used in the customary manner, for example by watering, spraying, atomizing or scattering.

The active compounds according to the invention can be applied either before or after emergence of the plants.

They can also be incorporated into the soil before sowing.

They are used, in particular, after emergence of the plants.

The amount of active compound used can vary within a substantial range. It depends essentially on the nature of the desired effect. In general, the amounts used are between 0.001 and 10 kg of active compound per hectare of soil surface, preferably between 0.01 and 5 kg per ha.

The preparation and use of the active compound according to the invention can be seen from the following examples.

Preparation Examples:

Example 1

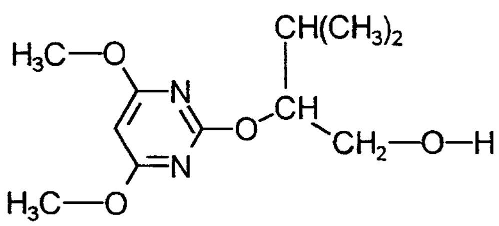

To a methanol solution (50 ml) of 0,006 Mol 2-(4,6-dimethoxy-2-pyimidinyloxy)-3-methylbutanol (1.5 g) was added sodium borohydride (0.2 g), followed by a tenminute stirring at room temperature. A small amount of water was then added to the reaction liquid, followed by distillation at a reduced pressure to remove the methanol therefrom and then addition of water to the thus obtained oily product that was in turn extracted with ethyl acetate. The resulting organic layer was water-washed and dried with anhydrous sodium sulfate. The organic layer was removed under reduced pressure distillation, and the oily residue was purified through silica gel column chromatography to give 2-(4,6-dimethoxy-2-pyrimidinyloxy)-3-methylbutanol (1.2 g). $n_D^{20}$ = 1.5026

Example 2

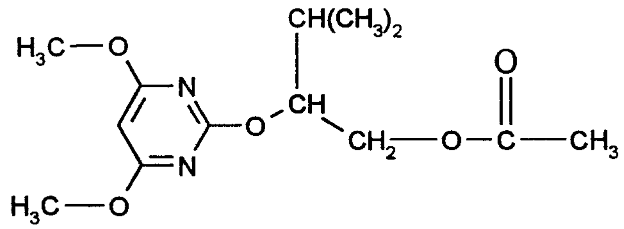

0,0049 Mol of 2-(4,6-dimethoxy-2-pyrimidinyloxy)-3methyl-butanol (1.2 g) was dissolved in pyridine (4 ml). To the resulting solution was dropwise added acetic anhydride (4 ml) at room temperature. After a three-hour stirring, the reaction liquid was poured into iced water and extracted with ethyl acetate, followed by washing with hydrochloric acid and water, in this order.

After drying with anhydrous sodium sulfate, the solvent was distilled off under reduced pressure and the oily substance thus obtained was purified by a silica gel column chromatography to give 2-(4,6-dimethoxy-2-pyrimidinyloxy)-3-methyl-1-butanol acetyl acetate (1.1 g). $n_D^{20}$ = 1.4883.

Example 3

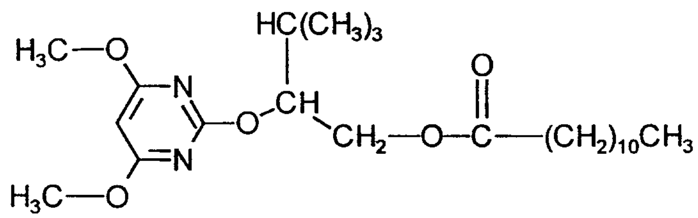

0,004 Mol of 2-(4,6-dimethoxy-2-pyrimidinyloxy)-3-methyl-1-butanol (1.1. g) and 0,0045 Mol triethylamide (0.45 g) was dissolved in methylene chloride (40 ml), followed by dropwise addition of 0,004 Mol lauryl chloride (0.95 g) dissolved in methylene chloride (10 ml) at room temperature. After a three-hour stirring, the reaction liquid was washed with water, and dried with anhydrous sodium sulfate, followed by a reduced pressure distillation to remove the solvent therefrom. The thus obtained oily substance was purified through a silica gel column chromatography to obtain 2-(4,6-dimethoxy-2-pyrimidinyloxy)-3,3-dimethylbutyl laurate (0.8 g). $n_D^{20}$ = 1.4749

Together with the compounds obtained in the above preparation Examples 1 to 3, compounds obtained by the same method as above preparation Examples 1 to 3 are shown in following Table 7.

Table 7

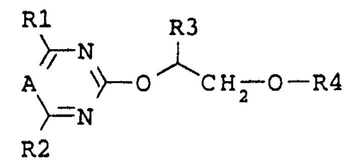

| Compound No. | A | R1 | R2 | R3 | R4 | Refractive index |
|---|---|---|---|---|---|---|
| 1 | -CH= | $OCH_3$ | $OCH_3$ | $C(CH_3)_3$ | H | $n_D^{20}$ 1.5030 |
| 2 | -CH= | $OCH_3$ | $OCH_3$ | $C(CH_3)_3$ | 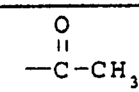 | $n_D^{20}$ 1.4824 |
| 3 | -CH= | $OCH_3$ | $OCH_3$ | $CH_2CH_2CH_3$ | H | $n_D^{20}$ 1.4971 |
| 4 | -CH= | $OCH_3$ | $OCH_3$ | $CH_2CH_2CH_3$ | 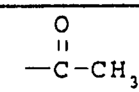 | $n_D^{20}$ 1.4861 |
| 5 | -CH= | $OCH_3$ | $OCH_3$ | $CH_2CH_2CH_3$ | 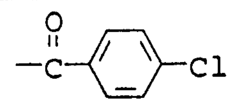 | $n_D^{20}$ 1.5385 |
| 6 | -CH= | $OCH_3$ | $OCH_3$ | $CH(CH_3)_2$ | H | $n_D^{20}$ 1.5026 |
| 7 | -CH= | $OCH_3$ | $OCH_3$ | $CH(CH_3)_2$ | 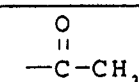 | $n_D^{20}$ 1.4883 |

Table 7 (contiuned)

| Compound No. | A | R1 | R2 | R3 | R4 | Refractive index |
|---|---|---|---|---|---|---|
| 8 | -CH= | $OCH_3$ | $OCH_3$ | $CH_2CH(CH_3)_2$ | H | $n_D^{20}$ 1.4955 |
| 9 | -CH= | $OCH_3$ | $OCH_3$ | $C(CH_3)_3$ | 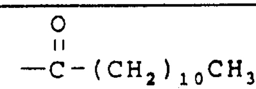 | $n_D^{20}$ 1.1419 |
| 10 | -CH= | $OCH_3$ | $OCH_3$ | $C(CH_3)_3$ | 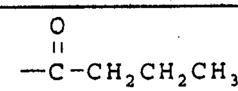 | $n_D^{20}$ 1.4768 |
| 11 | -CH= | $OCH_3$ | $OCH_3$ | $CH(CH_3)_2$ | 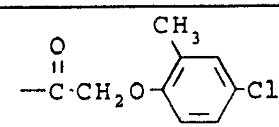 | $n_D^{20}$ 1.15164 |
| 12 | -CH= | $OCH_3$ | $OCH_3$ | $CH(CH_3)_2$ | 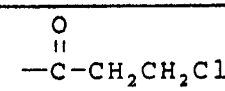 | $n_D^{20}$ 1.4962 |
| 13 | -CH= | $OCH_3$ | $OCH_3$ | $CH(CH_3)_2$ | 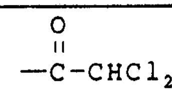 | $n_D^{20}$ 1.5181 |
| 14 | -CH= | $OCH_3$ | $OCH_3$ | $CH(CH_3)_2$ | 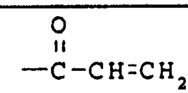 | $n_D^{20}$ 1.4992 |
| 15 | -CH= | $OCH_3$ | $OCH_3$ | $CH(CH_3)_2$ | 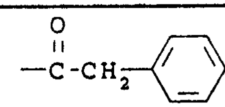 | $n_D^{20}$ 1.5272 |

Table 7 (contiuned)

| Compound No. | A | R1 | R2 | R3 | R4 | Refractive index |
|---|---|---|---|---|---|---|
| 16 | -CH= | $OCH_3$ | $OCH_3$ | $CH(CH_3)_2$ | 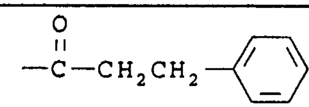 | $n_D^{20}$ 1.5246 |
| 17 | -CH= | $OCH_3$ | $OCH_3$ | $CH(CH_3)_2$ | 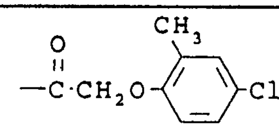 | $n_D^{20}$ 1.5230 |
| 18 | -CH= | $OCH_3$ | $OCH_3$ | $CH(CH_3)_2$ | 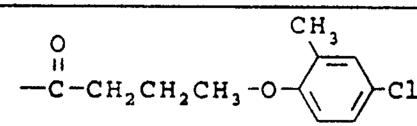 | $n_D^{20}$ 1.5152 |
| 19 | -CH= | $OCH_3$ | $OCH_3$ | $CH(CH_3)_2$ | 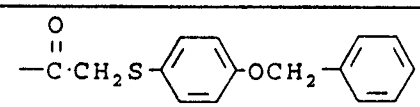 | $n_D^{20}$ 1.5552 |
| 20 | -CH= | $OCH_3$ | $OCH_3$ | $CH(CH_3)_2$ | 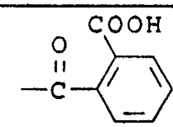 | $n_D^{20}$ 1.5306 |
| 21 | -CH= | $OCH_3$ | $OCH_3$ | $CH(CH_3)_2$ | 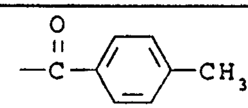 | $n_D^{20}$ 1.5451 |
| 22 | -CH= | $OCH_3$ | $OCH_3$ | $CH(CH_3)_2$ | 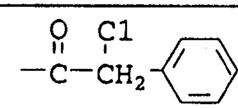 | $n_D^{20}$ 1.5316 |

Table 7 (contiuned)

| Compound No. | A | R1 | R2 | R3 | R4 | Refractive index |
|---|---|---|---|---|---|---|
| 23 | -CH= | $OCH_3$ | $OCH_3$ | $CH(CH_3)_2$ | 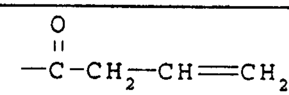 | $n_D^{20}$ 1.4856 |
| 24 | -CH= | $OCH_3$ | $OCH_3$ | $CH(CH_3)_2$ | 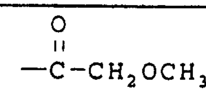 | $n_D^{20}$ 1.4874 |
| 25 | -CH= | $OCH_3$ | $OCH_3$ | $CH(CH_3)_2$ | 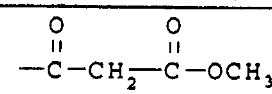 | $n_D^{20}$ 1.4884 |
| 26 | -CH= | $OCH_3$ | $OCH_3$ | $CH(CH_3)_2$ | 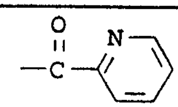 | $n_D^{20}$ 1.4969 |
| 27 | -CH= | $OCH_3$ | $OCH_3$ | $C(CH_3)_3$ | 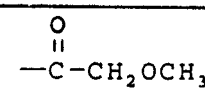 | $n_D^{20}$ 1.4859 |
| 28 | -CH= | $OCH_3$ | $OCH_3$ | $C(CH_3)_3$ | 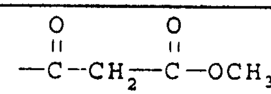 | $n_D^{20}$ 1.4845 |
| 29 | -CH= | $OCH_3$ | $OCH_3$ | $C(CH_3)_3$ | 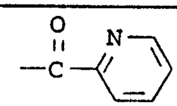 | $n_D^{20}$ 1.5229 |
| 30 | -CH= | $OCH_3$ | $OCH_3$ | $C(CH_3)_3$ | 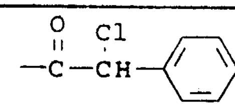 | $n_D^{20}$ 1.5252 |

Table 7 (contiuned)

| Compound No. | A | R1 | R2 | R3 | R4 | Physical constant |
|---|---|---|---|---|---|---|
| 31 | -CH= | $OCH_3$ | $OCH_3$ | $C(CH_3)_3$ | 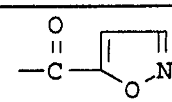 | mp 100.5 - 102 °C |
| 32 | -CH= | $OCH_3$ | $OCH_3$ | $C(CH_3)_3$ | 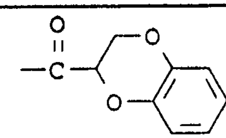 | $n_D^{20}$ 1.5220 |
| 33 | -CH= | $OCH_3$ | $OCH_3$ | $C(CH_3)_3$ | 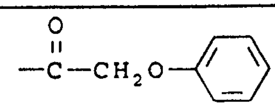 | $n_D^{20}$ 1.4845 |
| 34 | -CH= | $OCH_3$ | $OCH_3$ | $C(CH_3)_3$ | 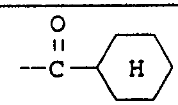 | $n_D^{20}$ 1.4900 |
| 35 | -CH= | $OCH_3$ | $OCH_3$ | $C(CH_3)_3$ | 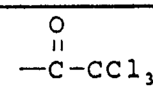 | $n_D^{20}$ 1.5013 |
| 36 | -CH= | $OCH_3$ | $OCH_3$ | $C(CH_3)_3$ | 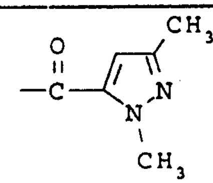 | $n_D^{20}$ 1.5084 |

Exammple 4: Synthesis of intermediate compounds

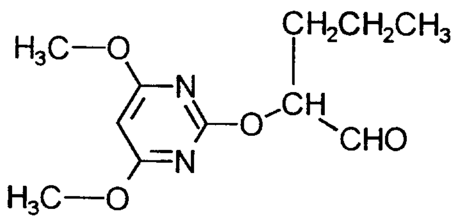

0,04 Mol of 2-(4,6-dimethoxy-2-pyrimidinyloxy)-1-hexane (10 g) was dissolved in methanol (300 ml), followed by ozone gas blown thereinto at a temperature between -20 to -10 °C for forty-eight hours. After the temperature having returned to room temperature (5 ml) water and acetic acid were added to the reaction liquid, followed by addition of zinc powder (1.5 g) dividedly in three portions (500 g x 3). After the above-mentioned addition, the reaction liquid was stirred for one hour and the methanol was distilled off under reduced pressure. Then, water was further added to the reaction product, followed by extraction with ethyl acetate. After drying with anhydrous sodium sulfate, the extracted substance was distilled off under reduced pressure, to obtain an oily product that was purified through a silica gel column chromatography to obtain 2-(4,6-dimethoxy-2-pyrimidinyloxy)pentanal. $n_D^{20}$ = 1.4978

Together with the compounds obtained in the above preparation Examples 4, compounds obtained by the same method as above preparation Example 4 are shown in following Table 8.

Table 8

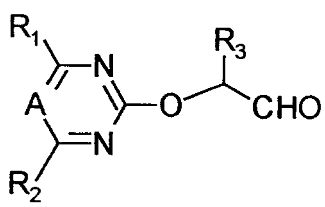

| A | $R_1$ | $R_2$ | $R_3$ | Physical Constant |
|---|---|---|---|---|
| -CH= | $OCH_3$ | $OCH_3$ | $C(CH_3)_3$ | mp. 66.5-70.9°C |
| -CH= | $OCH_3$ | $OCH_3$ | $CH_2CH_2CH_3$ | $n_D^{20}$ = 1.4978 |
| -CH= | $OCH_3$ | $OCH_3$ | $CH(CH_3)_2$ | $n_D^{20}$ = 1.4979 |
| -CH= | $OCH_3$ | $OCH_3$ | $CH_2CH(CH_3)_2$ | mp. 53.5-56.5°C |

Example 5: Synthesis of intermediate compound:

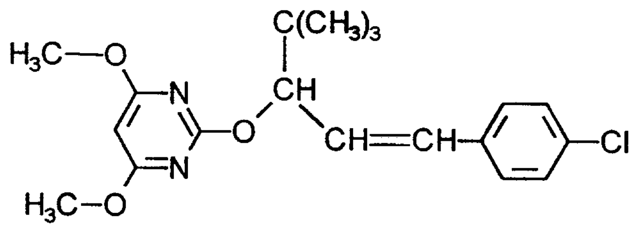

0,075 Mol 1-(4-chlorophenyl)-4,4-dimethylpentene-3-ol (17 g) was dissolved in dimethylformamide, followed by a slow addition of a 60% sodium hydride (3.5 g) at room temperature and then a one-hour stirring. To the reaction liquid was added dropwise a dimethylformamide solution of 2-methylsulfonyl-4,6-dimethoxy-pyrimidine (13 .4 g), followed by a five-hour stirring. Water was then added to the reaction liquid that was extracted with ethyl acetate and then dried with anhydrous sodium sulfate, to obtain an oily product after the removal of the solvent therefrom under reduced pressure. The thus obtained product was recrystallized from hexane/ethyl acetate to obtain 1-(4-chlorophenyl)-3-(4,6-dimethoxy-2-pyrimidinyloxy)-4,4-dimethylpentene (15 g). mp. 104.5-106 ° C

In the following are shown the compounds that were prepared the same way as the above-mentioned Synthesis Example 5, together with those that were actually prepared by said Example 5.

Table 9

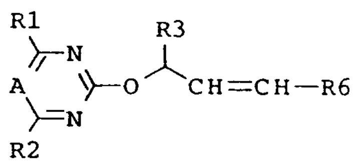

| A | R1 | R2 | R3 | R6 | physical constant |
|---|---|---|---|---|---|
| -CH= | $OCH_3$ | $OCH_3$ | $C(CH_3)_3$ | 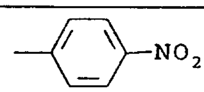 | mp 104.5 - 106 °C |
| -CH= | $OCH_3$ | $OCH_3$ | $C(CH_3)_3$ | 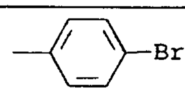 | mp 113 - 116.5 °C |
| -CH= | $OCH_3$ | $OCH_3$ | $C(CH_3)_3$ | 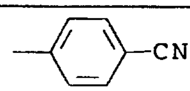 | mp 98 - 103.5 °C |
| -CH= | $OCH_3$ | $OCH_3$ | $C(CH_3)_3$ | 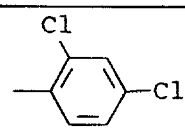 | mp 115.5 - 118.5 °C |
| -CH= | $OCH_3$ | $OCH_3$ | $CH_2CH_2CH_3$ | H | $n_D^{20}$ 1.4901 |
| -CH= | $OCH_3$ | $OCH_3$ | $C(CH_3)_3$ | 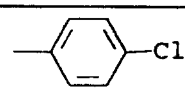 | mp 104.5 - 106 °C |
| -CH= | $OCH_3$ | $OCH_3$ | $C(CH_3)_3$ | 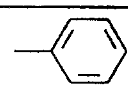 | mp 83 - 84.5 °C |
| -CH= | $OCH_3$ | $OCH_3$ | $CH(CH_3)_2$ | H | $n_D^{20}$ 1.4929 |
| -CH= | $OCH_3$ | $OCH_3$ | $C(CH_3)_3$ | 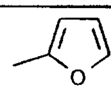 | $n_D^{20}$ 1.5005 |
| -CH= | $OCH_3$ | $OCH_3$ | $CH_2CH(CH_3)_2$ | H | $n_D^{20}$ 1.4882 |
| -CH= | $OCH_3$ | $OCH_3$ | $C(CH_3)_3$ | 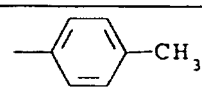 | mp 106.5 - 107.5 °C |
| -CH= | $OCH_3$ | $OCH_3$ | $C(CH_3)_3$ | $CH_3$ | $n_D^{20}$ 1.4882 |
| -CH= | $OCH_3$ | $OCH_3$ | $C(CH_3)_3$ | $CH_2CH_2CH_3$ | $n_D^{20}$ 1.4828 |

Biological test:

Test Example 1

Post-emergence foliage treatment on upland weeds

| Formulation of Active Compounds | |
|---|---|
| Carrier:<br>Emulsifier: | 5 parts by weight of acetone<br>1 part by weight of benzyloxy polyglycol ether |

To produce a suitable formulation of each of the active compounds, 1 part by weight of the active compound was mixed with a stated amount of carrier and with the stated amount of emulsifier, and the resulting emulsifiable concentrate was diluted with water to the desired concentration.

Test Procedures

In a greenhouse, a number of test pots each having an area of 120 $cm^2$ were charged with soil taken out from a cultivated field. Seeds of barnyard grass (Echinochloa crus-gall) and wild blite (Amaranthus Blitum), respectively, were sown onto the soil surface in the respective test pots with each of the sown soil surfaces being covered with a soil layer to allow them emerging.

After 10 days, predetermined dosages of the active compound formulations prepared as mentioned above were uniformly sprayed onto the foliage portions of the test weeds in the respective test pots.

Three weeks after the application of the active compound formulations, the degrees of herbicidal effect on the weeds were determined. The resulting herbicidal effect were rated according to the following assessment:

| | |
|---|---|
| Completely killed | 100% |
| Status equivalent to non-treated pots | 0% |

As the results of the present test, the compounds identified by the foregoing compounds Nos. 1, 2, 6 and 7 exhibited a herbicidal effect of 90% or higher on barnyard grass and wild blite, respectively, at a dosage of 2 kg/ha.

Test Example 2

Pre-emergence soil treatment test on upland weeds

Test Procedures

In a greenhouse, a number of test pots each having an area of 120 $cm^2$ were charged with soil taken out from a cultivated field. Onto the soil surface in the respective test pots were sown the seeds of barnyard grass (Echinochloa crus-gali) and wild blite (Amaranthus Blitum) followed by soil covering thereon.

Thereafter, predetermined dosages of the active compound formulations mentioned above were uniformly sprayed onto the soil surface of the respective test pots.

Four weeks after the application of the active compound formulations, the degrees of herbicidal effect on the weeds were determined.

As the results of the test, the compounds identified by the foregoing compound Nos. 1 and 2 (at a dosage of 0.5 kg/ha) and by the foregoing compound Nos. 3, 4, 6, 7 and 8 (at a dosage of 1.0 kg/ha) exhibited a herbicidal effect of 100% on barnyard grass and wild blite, respectively.

## Claims

**1.** Pyrimidinyloxy/triazinyloxy alkanol derivatives of the formula (I)

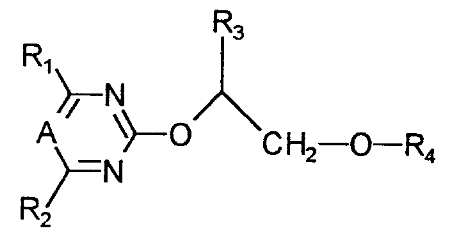 (I)

wherein

- $R_1$ represents $C_{1-4}$ alkyl, $C_{1-4}$ alkoxy, halogen, halogeno-$C_{1-4}$ alkyl or halogeno-$C_{1-4}$ alkoxy,
- $R_2$ represents $C_{1-4}$ alkoxy, halogen, halogeno-$C_{1-4}$ alkyl or halogeno-$C_{1-4}$ alkoxy,
- $R_3$ represents $c_{2-5}$ alkyl,
- A represents -CH= or -N=,
- $R_4$ represents hydrogen or -CO-$R_5$ and
- $R_5$ represents hydrogen, carboxyl, $C_{1-4}$ alkoxycarbonyl or in each case optionally unsubstituted or substituted $C_{1-20}$ saturated hydrocarbon, $C_{2-20}$ unsaturated hydrocarbon, phenyl or $C_{3-8}$ cycloalkyl,
- $R_5$ further represents in each case an optionally five- or six-membered heterocyclic group, a condensed heterocyclic group or one of the group represented by the following formulae:

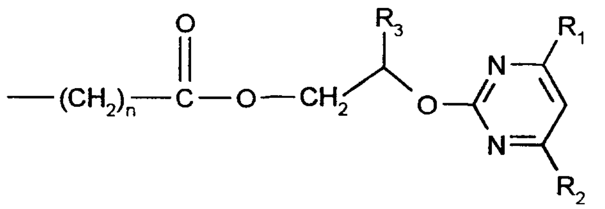 or

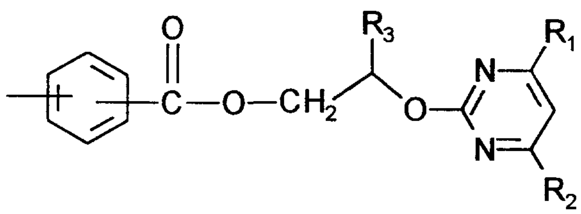

wherein

- $R_1$, $R_2$ and $R_3$ have the meanings as mentioned above and
- n represents all integer from 1 to 6.

**2.** The compounds of formula (I), according to claim 1, characterized in that

- $R_1$ represents methoxy, difluoromethoxy or trifluoromethoxy,
- $R_2$ represents methoxy, difluoromethoxy or trifluoromethoxy,
- $R_3$ represents $C_{2-5}$ alkyl,
- A represents -CH=,
- $R_4$ represents hydrogen or -CO-$R_5$,
- $R_5$ represents hydrogen,
- $R_5$ further represents an optionally unsubstituted or substituted **$C_{1-12}$ alkyl** (suitable substituent-(s) are cyano, halogen, carboxyl or its salt, $C_{1-4}$ alkoxy, $C_{3-7}$ cycloalkyl [which may be itself substituted by halogen or $C_{1-4}$ alkyl], $C_{1-4}$ alkylthio [which may be itself substituted by halogen or $C_{1-4}$], $C_{1-4}$ alkoxy-carbonyl, $C_{1-4}$ alkyl-carbonyl, a optionally substituted phenyl-sulfonyloxy, $C_{1-4}$ alkyl-sulfonyloxy, amino, $C_{1-4}$ alkyl-amino, di-$C_{1-4}$ alkyl-sulfonyloxy, amino, $C_{1-4}$ alkylamino, di-$C_{1-4}$ alkyl amino, an optionally substituted phenyl (which may be itself substituted by nitro, cyano, halogen, $C_{1-4}$ alkyl, $C_{1-4}$ alkoxy, halogeno-$C_{1-4}$ alkyl or halogeno-$C_{1-4}$ alkoxy], an optionally substituted phenoxy [which may be itself substituted by nitro, cyano, halogen, $C_{1-4}$ alkyl, $C_{1-4}$ alkoxy, halogeno-$C_{1-4}$ alkyl, halogeno-$C_{1-4}$ alkoxy, a

substituted phenoxy, a substituted pyrimidinyloxy, and the below-mentioned group Q],
an optionally substituted phenylthio [the substituent(s) may be selected from the group consisting of nitro, cyano, halogen, $C_{1-4}$ alkyl, $C_{1-4}$ alkoxy, halogeno-$C_{1-4}$ alkyl, halogeno-$C_{1-4}$ alkoxy, a substitutable phenoxy, a substitutable pyrimidinyloxy, and the below-mentioned group Q],
an optionally substituted naphthyl [the substituent(s) may be selected from the group consisting of nitro, cyano, halogen, $C_{1-4}$ alkyl, $C_{1-4}$ alkoxy, halogeno-$C_{1-4}$ alkyl, halogeno-$C_{1-4}$ alkoxy, and a phenoxy substitutable with halogen or halogenoalkyl],
an optionally substituted naphtoxy [the substituent(s) may be selected from the group consisting of nitro, cyano, halogen, $C_{1-4}$ alkyl, $C_{1-4}$ alkoxy, halogeno-$C_{1-4}$alkyl, halogeno-$C_{1-4}$ alkoxy, and a phenoxy which may be itself substituted by halogen or halogenoalkyl],

$R_5$ further represents an optionally substituted **$C_{2-12}$ alkenyl** (the substituent(s) may be selected from halogen, and phenyl which may be itself substituted by halogen or $C_{1-4}$ alkyl),

$R_5$ further represents **$C_{2-12}$ alkynyl**,

$R_5$ further represents a optionally substituted **phenyl** (the substituent(s) may be selected from the group consisting of cyano, nitro, halogen, $C_{1-4}$ alkyl, $C_{1-4}$ alkoxy, halogeno-$C_{1-4}$ alkyl or halogeno-$C_{1-4}$ alkoxy),

$R_5$ further represents **$C_{3-8}$ cycloalkyl** being, optionally substituted by $C_{1-4}$ alkyl,

$R_5$ further represents an optionally substituted **five- or six-membered heterocyclic group** (the hetero atom(s) of said heterocyclic group may be selected from the group consisting of nitrogen, oxygen or sulfur, and the substituents may be selected from the group consisting of cyano, nitro, halogen, $C_{1-4}$ alkyl, $C_{1-4}$ alkoxy, halogeno-$C_{1-4}$ alkyl, halogeno-$C_{1-4}$ alkoxy and an optionally substituted phenyl [which may be itself substituted by one of the group consisting of nitro, cyano, halogen, $C_{1-4}$ alkyl, $C_{1-4}$ alkoxy, halogeno-$C_{1-4}$ alkyl or halogeno-$C_{1-4}$ alkoxy] ),

$R_5$ further represents a optionally substituted **condensed heterocyclic group** (the hetero atom(s) of said heterocyclic group may be selected from the group consisting of nitrogen, oxygen or sulfur, and the substituents may be selected from the group consisting of cyano, nitro, halogen, $C_{1-4}$ alkyl, $C_{1-4}$ alkoxy, halogeno-$C_{1-4}$ alkyl or halogeno-$C_{1-4}$ alkoxy),

$R_5$ further represents **$C_{1-4}$ alkoxy-carbonyl, carboxyl or its salts** or one of the following group:

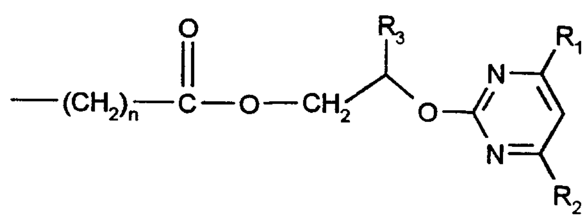

or

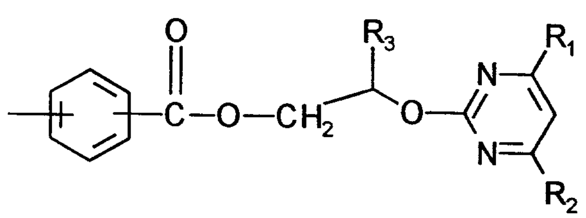

wherein $R_1$, $R_2$ and $R_3$ have the same meanings as mentioned above, and n represents 0, 1, 2, 4, 5 or 6,
and

Q represents an optionally halogen-substituted group selected from:

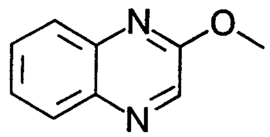 , 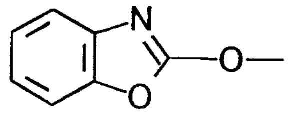 or

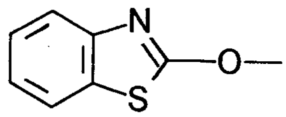

3. The compounds of formula (I) according to claim 1, characterized in that

$R_1$ represents methoxy,

$R_2$ represents methoxy,

$R_3$ represents $C_{2-4}$ alkyl,

A represents -CH=,

$R_4$ represents hydrogen or -CO-$R_5$,

$R_5$ represents hydrogen,

$R_5$ further represents an optionally substituted **$C_{1-12}$ alkyl** (suitable substituents are cyano, fluoro, chloro, carboxyl or its sodium salt, methoxy, ethoxy, butoxy, a methyl-substituted $C_{3-7}$ cycloalkyl, methylthio, ethylthio, butylthio, methoxy-carbonyl, methyl-carbonyl, a methyl-substituted phenyl-sulfonyloxy, methyl-sulfonyloxy, ethyl-sulfonyloxy, amino, methylamino, ethylamino, dimethylamino, an optionally substituted phenyl [the substituent(s) may be selected from the group consisting of nitro, cyano, fluoro, chloro, bromo, methyl or trifluoromethyl], an optionally substituted phenoxy [the substituent(s) may be selected from the group consisting of nitro, cyano, fluoro, chloro, bromo, methyl, trifluoromethyl, a substituted phenoxy (which may be itself substituted by fluoro, chloro, or trifluoromethyl), a substituted pyrimidinyloxy (which may be itself substituted by fluoro, chloro or trifluoromethyl), and the below-mentioned group Q], an optionally substitutable phenylthio [the substituent(s) may be selected from the group consisting of nitro, cyano, fluoro, chloro, bromo, methyl, trifluoromethyl, halogeno-$C_{1-4}$ alkyl, halogeno-$C_{1-4}$ alkoxy, a substituted phenyl (which may be itself substituted by fluoro, chloro, or trifluoromethyl), a substituted pyrimidinyloxy (which may be itself substituted by fluoro, chloro or trifluoromethyl), and the below-mentioned group Q], all optionally substituted naphtyl [the substituent(s) may be selected from the group consisting of nitro, cyano, fluoro, chloro, bromo, methyl, trifluoromethyl, halogeno-$C_{1-4}$ alkyl, halogeno-$C_{1-4}$ alkoxy, substituted phenyl (which may be itself substituted by fluoro, chloro or trifluoromethyl), optionally substituted pyrimidinyloxy (the substituent(s) may be selected from the group consisting of fluoro, chloro or trifluoromethyl), and a phenoxy that may be optionally substituted with chloro or trifluoromethoxy], an optionally substituted naphtoxy [the substituent(s) may be selected from the group consisting of nitro, cyano, fluoro, chloro, bromo, methyl, trifluoromethyl, halogeno-$C_{1-4}$ alkyl,halogeno-$C_{1-4}$ alkoxy, optionally substituted phenyl (the substituent(s) may be selected from the group consisting of fluoro, chloro or trifluoromethyl), optionally substituted pyrimidinyloxy (the substituent(s) may be selected from the group consisting of fluoro, chloro or trifluoromethyl), and a phenoxy that may be optionally substituted by chloro or trifluoromethoxy]),

$R_5$ further represents an optionally substituted **$C_{2-12}$ alkenyl** (the substituent(s) may be selected from chloro, bromo and a phenyl which may be optionally substituted by fluoro, chloro, bromo, methyl or ethyl),

$R_5$ further represents **$C_{2-12}$ alkynyl**,

$R_5$ further represents an optionally substituted **phenyl** (the substituent(s) may be selected from the group consisting of cyano, nitro, fluoro, chloro, bromo, methyl, ethyl, methoxy, ethoxy, trifluoromethyl or trifluoromethoxy),

$R_5$ further represents in each case optionally methyl-substituted **cyclopropyl**, **cyclopentyl or cyclohexyl**,

$R_5$ further represents in each case a optionally substituted, **thiadiazolyl**, **thiazolyl, imidazolyl, furyl, pyridyl, pyrimidinyl, pyrazinyl, pyridazinyl, isoxazolyl, pyrazolyl and thienyl** (the

substituent(s) may be selected from the group consisting of cyano, nitro, fluoro, chloro, methyl, ethyl, methoxy, ethoxy, trifluoromethyl, trifluoromethoxy and an optionally substituted phenyl (the substituent(s) may be selected from the group consisting of nitro, fluoro, chloro, bromo, methyl, ethyl, methoxy or trifluoromethyl]),

$R_5$ further represents an optionally substituted **quinolyl**, **benzoxazolyl**, **benzothiazolyl, benzimidazolyl** and **phtalazinyl** (the substituent(s) may be selected from the group consisting of cyano, nitro, fluoro, chloro, methyl, ethyl, methoxy, ethoxy, trifluoromethyl or trifluoromethoxy),

$R_5$ further represents **methoxycarbonyl**, **carboxyl or its sodium salt**, or one of the following group:

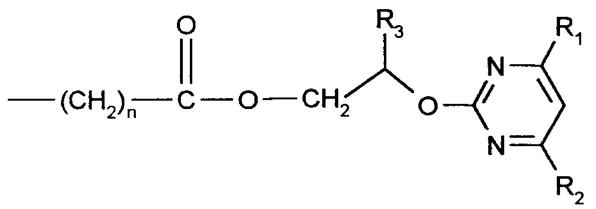

or

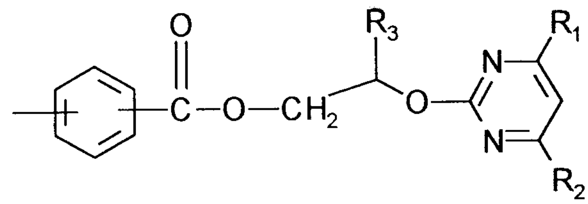

wherein $R_1$, $R_2$ and $R_3$ have the same meanings as mentioned above, and n represents 0, 1, 2, 4, 5 or 6,

and

Q represents an optionally halogen-substituted group selected from:

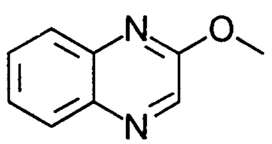

,

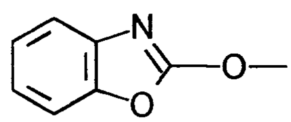

or

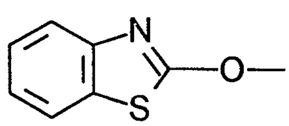

.

**4.** Process for the preparation of pyrimidinyloxy/triazinyloxy alkanol derivatives of the formula (I)

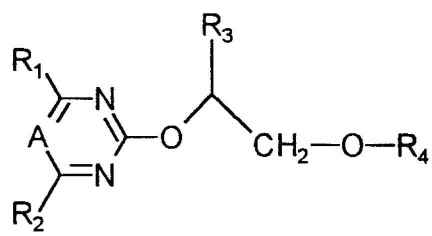

(I)

wherein

$R_1$ represents $C_{1-4}$ alkyl, $C_{1-4}$ alkoxy, halogen, halogeno-$C_{1-4}$ alkyl or halogeno-$C_{1-4}$ alkoxy,

$R_2$ represents $C_{1-4}$ alkoxy, halogen, halogeno-$C_{1-4}$ alkyl or halogeno-$C_{1-4}$ alkoxy,

$R_3$ represents $C_{2-5}$ alkyl,

A represents -CH= or -N=,

$R_4$ represents hydrogen or -CO-$R_5$ and

$R_5$ represents hydrogen, carboxyl, $C_{1-4}$ alkoxycarbonyl or in each case optionally unsubstituted or substituted $C_{1-20}$ saturated hydrocarbon, $C_{2-20}$ unsaturated hydrocarbon or phenyl or $C_{3-8}$ cycloalkyl,

$R_5$ further represents in each case an optionally five- or six-membered heterocyclic group, a condensed heterocyclic group or one of the group represented by the following formulae:

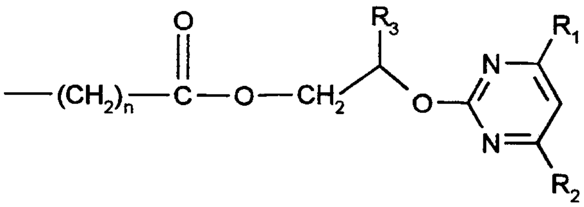

or

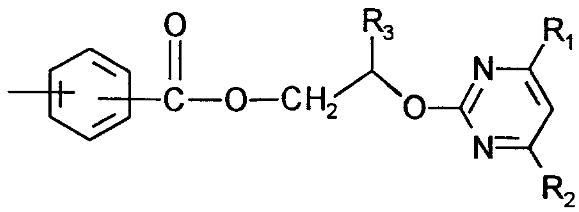

wherein

$R_1$, $R_2$ and $R_3$ have the meanings as mentioned above and

n represents an integer from 1 to 6,

characterized in that

(a) in the case where $R_4$ represents hydrogen atom:
compound of the formula (II)

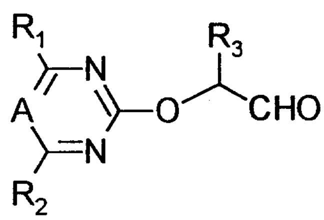

(II)

wherein

$R_1$, $R_2$, $R_3$ and A have the same meanings as mentioned above,

are reduced in the presence of inert solvents,

or

(b) in the case where the $R_4$ represents -CO-$R_5$:
compounds of the formula (IV)

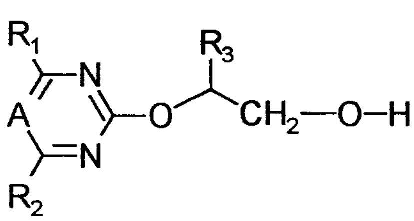

(IV)

wherein

$R_1$, $R_2$, $R_3$ and A have the same meanings as mentioned above,
are reacted with an acid halide of the formula (V),

$R_5$-CO-$R_7$ (V)

wherein
$R_5$ has the same meanings as mentioned above, and $R_7$ represents halogen,
in the presence of inert solvents and if appropriat, in the presence of an acid binder,
or
(c) in the case where $R_4$ represents -CO-$R_5$:
above mentioned compounds of the formula (III) are reacted with acid anhydrides of the following formula (VI):

$R_5$-CO-O-CO-$R_5$ (VI)

wherein
$R_5$ has the same meanings as mentioned above, and the two $R_5$ may be the same or different from each other,
in the presence of inert solvents and if appropriate, in the presence of an acid binder.

**5.** Herbicidal compositions, characterized in that they contain at least one pyrimidinyloxy/triazinyloxy alkanol derivative of the formula (I) according to claim 1.

**6.** Process for combating weeds, characterized in that pyrimidinyloxy/triazinyloxy alkanol derivatives of the formula (I) according to claim 1 are allowed to act on weeds and/or their habitat.

**7.** Use of pyrimidinyloxy/triazinyloxy alkanol derivatives of the formula (I) according to claim 1 for combating weeds.

**8.** Process for the preparation of herbicidal compositions, characterized in that pyrimidinyloxy/triazinyloxy alkanol derivatives of the formula (I) according to claim 1 are mixed with extenders and/or surface active agents.

**9.** Pyrimidinyloxy-or triazinyloxy alkylaldehydes of the formula (II)

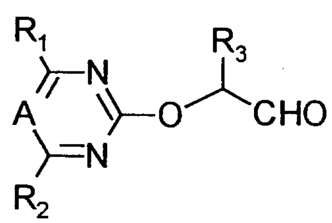 (II)

wherein
$R_1$ represents $C_{1-4}$ alkyl, $C_{1-4}$ alkoxy, halogen, halogeno-$C_{1-4}$ alkyl or halogeno-$C_{1-4}$ alkoxy,
$R_2$ represents $C_{1-4}$ alkoxy, halogen, halogeno-$C_{1-4}$ alkyl or halogeno-$C_{1-4}$ alkoxy,
$R_3$ represents $C_{2-5}$ alkyl, and
A represents -CH= or -N=.

**10.** Pyrimidinyloxy or triazinyloxy alkenes of the formula (III)

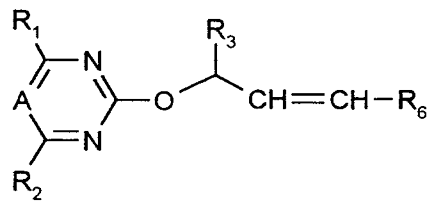

(III)

wherein

$R_1$ represents $C_{1-4}$ alkyl, $C_{1-4}$ alkoxy, halogen, halogeno-$C_{1-4}$ alkyl or halogeno-$C_{1-4}$ alkoxy,

$R_2$ represents $C_{1-4}$ alkoxy, halogen, halogeno-$C_{1-4}$ alkyl or halogeno-$C_{1-4}$ alkoxy,

$R_3$ represents $C_{2-5}$ alkyl,

A represents -CH= or -N= and

$R_6$ represents hydrogen, optionally substituted $C_{1-4}$ alkyl, optionally substituted phenyl, an optionally substituted benzyl or an optionally substituted heterocyclic group.

European Patent Office

# EUROPEAN SEARCH REPORT

Application Number
EP 94 10 1542

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| X | EP-A-0 400 741 (SHELL)<br>* claims *<br>--- | 9 | C07D239/60<br>C07D401/12<br>A01N43/54 |
| D,A | EP-A-0 409 368 (SCHERING)<br>* claims *<br>--- | 1,5 | |
| P,A | EP-A-0 565 951 (NIHON BAYER)<br>* the whole document *<br>----- | 1,5,9 | |
| | | | TECHNICAL FIELDS SEARCHED (Int.Cl.5) |
| | | | C07D |

The present search report has been drawn up for all claims

| Place of search | Date of completion of the search | Examiner |
|---|---|---|
| THE HAGUE | 2 May 1994 | Francois, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)